(19) Europäisches Patentamt
European Patent Office
Office européen des brevets

(11) EP 1 622 880 B3

(12) NEUE EUROPÄISCHE PATENTSCHRIFT
Nach dem Beschränkungsverfahren (B3-1)

(45) Hinweis auf die Patenterteilung:
**28.02.2007 Patentblatt 2007/09**

(45) Veröffentlichungstag und Bekanntmachung des Hinweises auf die Entscheidung über den Antrag auf Beschränkung:
**B3-1 23.05.2018 Patentblatt 2018/21**

(21) Anmeldenummer: **04728119.1**

(22) Anmeldetag: **17.04.2004**

(51) Int Cl.:
***C07D 239/84*** (2006.01) ***A61K 31/517*** (2006.01)
***A61P 31/22*** (2006.01)

(86) Internationale Anmeldenummer:
**PCT/EP2004/004103**

(87) Internationale Veröffentlichungsnummer:
**WO 2004/096778 (11.11.2004 Gazette 2004/46)**

# (54) SUBSTITUIERTE DIHYDROCHINAZOLINE MIT ANTIVIRALEN EIGENSCHAFTEN

SUBSTITUTED DIHYDROCHINAZOLINES HAVING ANTIVIRAL PROPERTIES

DIHYDROQUINAZOLINES SUBSTITUEES A PROPRIETES ANTIVIRALES

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**HR LT LV**

(30) Priorität: **02.05.2003 DE 10319612**

(43) Veröffentlichungstag der Anmeldung:
**08.02.2006 Patentblatt 2006/06**

(73) Patentinhaber: **AiCuris Anti-infective Cures GmbH**
**42117 Wuppertal (DE)**

(72) Erfinder:
- **WUNBERG, Tobias**
**42699 Solingen (DE)**
- **BAUMEISTER, Judith**
**42277 Wuppertal (DE)**
- **BETZ, Ulrich**
**64354 Reinheim (DE)**
- **JESKE, Mario**
**42699 Solingen (DE)**
- **LAMPE, Thomas**
**40223 Düsseldorf (DE)**
- **NIKOLIC, Susanne**
**40789 Monheim (DE)**
- **REEFSCHLÄGER, Jürgen**
**26125 Oldenburg (DE)**
- **SCHOHE-LOOP, Rudolf**
**42327 Wuppertal (DE)**
- **SÜSSMEIER, Frank**
**42277 Wuppertal (DE)**
- **ZIMMERMANN, Holger**
**42113 Wuppertal (DE)**
- **GROSSER, Rolf**
**51373 Leverkusen (DE)**
- **HENNINGER, Kerstin**
**42115 Wuppertal (DE)**
- **HEWLETT, Guy**
**42327 Wuppertal (DE)**
- **KELDENICH, Jörg**
**42113 Wuppertal (DE)**
- **LANG, Dieter**
**42553 Velbert (DE)**
- **NELL, Peter**
**42115 Wuppertal (DE)**

(74) Vertreter: **Kilger, Ute et al**
**Boehmert & Boehmert**
**Anwaltspartnerschaft mbB**
**Pettenkoferstrasse 22**
**80336 München (DE)**

(56) Entgegenhaltungen:
**EP-A- 1 201 765**

- **MARTINEZ, ANA ET AL: "Benzothiadiazine dioxide human cytomegalovirus inhibitors: Synthesis and antiviral evaluation of main heterocycle modified derivatives" ANTIVIRAL CHEMISTRY & CHEMOTHERAPY , 14(2), 107-114 CODEN: ACCHEH; ISSN: 0956-3202, 2003, XP009033652**
- **GRIBAUDO, G. ET AL: "The anticytomegaloviral activity of raltitrexed is abrogated in quiescent mouse fibroblasts that overexpress thymidylate synthase" VIRUS RESEARCH , 73(1), 57-65 CODEN: VIREDF; ISSN: 0168-1702, 2001, XP002288380**



Printed by Jouve, 75001 PARIS (FR)

## Beschreibung

Die Erfindung betrifft substituierte Dihydrochinazoline und Verfahren zu ihrer Herstellung sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

Die Synthese von Dihydrochinazolinen ist beschrieben in Saito T., et al. Tetrahedron Lett., 1996, 37, 209-212 und in Wang F., et al. Tetrahedron Lett., 1997, 38, 8651-8654.

Auf dem Markt sind zwar strukturell andersartige antiviral wirkende Mittel vorhanden, es kann aber regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Dihydrochinazoline antiviral wirksam sind.

Gegenstand der Erfindung sind Verbindungen der Formel

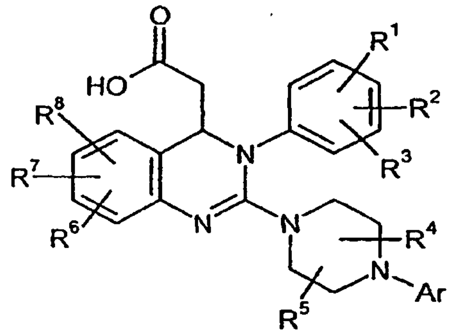

(I),

in welcher

Ar für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Formyl, Carboxyl, Alkylcarbonyl, Alkoxycarbonyl, Trifluormethyl, Halogen, Cyano, Hydroxy, Amino, Alkylamino, Aminocarbonyl und Nitro, worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl, oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls vorhandener dritter Substituent unabhängig davon aus der genannten Gruppe ausgewählt wird,

$R^1$ für Wasserstoff, Amino, Alkyl, Alkoxy, Alkylamino, Alkylthio, Cyano, Halogen, Nitro oder Trifluormethyl steht,

$R^2$ für Wasserstoff, Alkyl, Alkoxy, Alkylthio, Cyano, Halogen, Nitro oder Trifluormethyl steht,

$R^3$ für Amino, Alkyl, Alkoxy, Alkylamino, Alkylthio, Cyano, Halogen, Nitro, Trifluormethyl, Alkylsulfonyl oder Alkylaminosulfonyl steht

oder

einer der Reste $R^1$, $R^2$ und $R^3$ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,

$R^4$ für Wasserstoff oder Alkyl steht,

$R^5$ für Wasserstoff oder Alkyl steht

oder

die Reste $R^4$ und $R^5$ im Piperazin-Ring an genau gegenüberliegenden Kohlenstoffatomen gebunden sind und eine gegebenenfalls mit 1 bis 2 Methylgruppen substituierte Methylen-Brücke bilden,

$R^6$ für Alkyl, Alkoxy, Alkylthio, Fonnyl, Carboxyl, Aminocarbonyl, Alkylcarbonyl, Alkoxycarbonyl, Trifluormethyl, Halogen, Cyano, Hydroxy oder Nitro steht,

$R^7$ für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht

und

$R^8$ für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht,

und ihre Salze, ihre Solvate und die Solvate ihrer Salze.

Erfindungsgemäße Verbindungen sind die Verbindungen der Formel (I) und deren Salze, Solvate und Solvate der Salze, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze derChlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino Alkylcarbonyl, Alkylsulfonyl Alkylaminosulfonyl und Alkoxycarbonyl stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-N-methylamino. $C_1$-$C_3$-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylsulfonyl steht beispielhaft und vorzugsweise für Methylsulfonyl, Ethylsulfonyl, n-Propylsulfonyl, Isopropylsulfonyl, tert.-Butylsulfonyl, n-Pentylsulfonyl und n-Hexylsulfonyl.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, Isopropylaminosulfonyl, tert.-Butylaminosulfonyl, n-Pentylaminosulfonyl, n-Hexyl-aminosulfonyl, *N,N*-Dimethylaminosulfonyl, *N,N*-Diethylaminosulfonyl, *N*-Ethyl-*N*-methylaminosulfonyl, *N*-Methyl-*N*-n-propylaminosulfonyl, *N*-Isopropyl-*N*-n-propyl-aminosulfonyl, *N*-tert.-Butyl-*N*-methylaminosulfonyl, *N*-Ethyl-*N*-n-pentylamino-sulfonyl und *N*-n-Hexyl-*N*-methylaminosulfonyl. $C_1$-$C_3$-Alkylaminosulfonyl steht beispielsweise für einen Monoalkylaminosulfonylrest mit 1 bis 3 Kohlenstoffatomen oder für einen Dialkylaminosulfonylrest mit jeweils 1 bis 3 Kohlenstoffatomen pro Alkylsubstituent.

Alkylcarbonyl steht beispielhaft und vorzugsweise für Acetyl und Propanoyl.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Halogen steht für Fluor, Chlor, Brom und Jod, bevorzugt Fluor und Chlor.

Ein Symbol* an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfindung ein Enantiomerenüberschuss (enantiomeric excess) von mehr als 90 % verstanden wird (> 90 % ee).

Bevorzugt sind solche Verbindungen der Formel (I), in welchen

Ar für Phenyl steht, worin Phenyl substituiert sein kann mit 1 bis 3 Substituenten, wobei die Substituenten unabhängig voneinander ausgewählt werden aus der Gruppe, bestehend aus $C_1$-$C_6$-Alkyl. $C_1$-$C_6$-Alkoxy, Carboxyl, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino und Nitro,
oder zwei der Substituenten am Phenyl zusammen mit den Kohlenstoffatomen, an die sie gebunden sind, ein 1,3-Dioxolan bilden und ein gegebenenfalls vorhandener dritter Substituent unabhängig davon aus der genannten Gruppe ausgewählt wird,

$R^1$ für Wasserstoff, $C_1$-$C_3$-Alkyl. $C_1$-$C_3$-Alkoxy, $C_1$-$C_3$-Alkylthio, Fluor oder Chlor steht,

$R^2$ für Wasserstoff, $C_1$-$C_3$-Alkyl, $C_1$-$C_3$-Alkoxy, $C_1$-$C_3$-Alkylthio, Fluor oder Chlor steht,

$R^3$ für $C_1$-$C_4$-Alkyl, Cyano, Fluor, Chlor, Nitro, Trifluormethyl oder $C_1$-$C_3$-Alkylsulfonyl steht,

oder
einer der Reste $R^1$, $R^2$ und $R^3$ für Wasserstoff, $C_1$-$C_3$-Alkyl, $C_1$-$C_3$-Alkoxy, Cyano, Halogen, Nitro oder Trifluormethyl steht und die anderen beiden zusammen mit den Kohlenstoffatomen, an die sie gebunden sind, einen Cyclopentan-Ring oder einen Cyclohexan-Ring bilden,

$R^4$ für Wasserstoff oder Methyl steht,

$R^5$ für Wasserstoff steht,

$R^6$ für $C_1$-$C_3$-Alkyl, $C_1$-$C_3$-Alkoxy, Carboxyl, Aminocarbonyl, Trifluormethyl, Fluor, Chlor, Cyano, Hydroxy oder Nitro steht,

$R^7$ für Wasserstoff, $C_1$-$C_3$-Alkyl, $C_1$-$C_3$-Alkoxy, Fluor, Chlor, Cyano oder Hydroxy steht

und

$R^8$ für Wasserstoff, $C_1$-$C_3$-Alkyl, $C_1$-$C_3$-Alkoxy, Fluor, Chlor, Cyano oder Hydroxy steht.

Bevorzugt sind darunter besonders solche Verbindungen der Formel (I), in weichen

Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
$R^1$ für Wasserstoff, Methyl, Methoxy, Methylthio, Fluor oder Chlor steht,
$R^2$ für Wasserstoff steht,
$R^3$ für Methyl, iso-Propyl, tert.-Butyl, Cyano, Fluor, Chlor, Nitro oder Trifluormethyl steht,
$R^4$ für Wasserstoff steht,
$R^5$ für Wasserstoff steht,

$R^6$ für Aminocarbonyl, Fluor, Chlor, Cyano oder Hydroxy steht,
$R^7$ für Wasserstoff steht

und

$R^8$ für Wasserstoff, Fluor oder Chlor steht.

Bevorzugt sind darunter besonders auch solche Verbindungen der Formel (I), in welchen

Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
$R^1$ für Wasserstoff, Methyl, Methoxy, Methylthio, Fluor oder Chlor steht,
$R^2$ für Wasserstoff steht,
$R^3$ für Methyl, tert.-Butyl, Cyano, Fluor, Chlor, Nitro oder Trifluormethyl steht,
$R^4$ für Wasserstoff steht,
$R^5$ für Wasserstoff steht,
$R^6$ für Aminocarbonyl, Fluor, Chlor, Cyano oder Hydroxy steht,
$R^7$ für Wasserstoff steht

und

$R^8$ für Wasserstoff, Fluor oder Chlor steht.

Bevorzugt sind darunter ganz besonders solche Verbindungen der Formel (I), in welchen

Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor,
$R^1$ für Wasserstoff oder Methoxy steht,
$R^2$ für Wasserstoff steht,
$R^3$ für Methyl, tert.-Butyl, Chlor oder Trifluormethyl steht,
$R^4$ für Wasserstoff steht,
$R^5$ für Wasserstoff steht,
$R^6$ für Aminocarbonyl oder Fluor steht,
$R^7$ für Wasserstoff steht

und

$R^8$ für Wasserstoff oder Fluor steht.

Bevorzugt sind auch solche Verbindungen der Formel (I), in welchen $R^1$ für Wasserstoff, Methyl, Methoxy oder Fluor steht.

Bevorzugt sind darunter besonders solche Verbindungen der Formel (I), in welchen $R^1$ für Methoxy steht.

Bevorzugt sind auch solche Verbindungen der Formel (I), in welchen $R^1$ über die ortho-Position zur Verknüpfungsstelle des Phenylrings an den Phenylring gebunden ist. Unter der Verknüpfungsstelle des mit den Resten $R^1$, $R^2$ und $R^3$ substituierten Phenylrings wird im Rahmen der vorliegenden Erfindung das gemäß Formel (I) mit einem der beiden Dihydrochinazolin-Stickstoffatome verknüpfte Kohlenstoffatom des Phenylrings verstanden.

Besonders bevorzugt sind solche Verbindungen der Formel (I), in welchen $R^1$ für Methoxy steht und $R^1$ über die ortho-Position zur Verknüpfungsstelle des Phenylrings an den Phenylring gebunden ist.

Bevorzugt sind auch solche Verbindungen der Formel (I), in welchen $R^2$ für Wasserstoff steht.

Bevorzugt sind auch solche Verbindungen der Formel (I), in welchen $R^3$ für Trifluormethyl, Chlor, Methyl, iso-Propyl oder tert.-Butyl steht.

Bevorzugt sind darunter besonders solche Verbindungen der Formel (I), in welchen $R^3$ für Trifluormethyl, Chlor oder Methyl steht.

Bevorzugt sind darunter ganz besonders solche Verbindungen der Formel (I), in welchen $R^3$ für Trifluormethyl steht.

Bevorzugt sind auch solche Verbindungen der Formel (I), in welchen $R^1$ über die ortho-Position zur Verknüpfungsstelle des Phenylrings an den Phenylring gebunden ist und $R^3$ über die $R^1$ gegenüberliegende meta-Position zur Verknüpfungsstelle des Phenylrings an den Phenylring gebunden ist.

Besonders bevorzugt sind solche Verbindungen der Formel (I), in welchen $R^1$ über die ortho-Position zur Verknüpfungsstelle des Phenylrings an den Phenylring gebunden ist, $R^3$ für Trifluormethyl, Chlor oder Methyl steht und $R^3$ über die $R^1$ gegenüberliegende meta-Position zur Verknüpfungsstelle des Phenylrings an den Phenylring gebunden ist.

Besonders bevorzugt sind darunter solche Verbindungen der Formel (I), in welchen $R^1$ über die ortho-Position zur Verknüpfungsstelle des Phenylrings an den Phenylring gebunden ist, $R^3$ für Trifluormethyl steht und $R^3$ über die $R^1$ gegenüberliegende meta-Position zur Verknüpfungsstelle des Phenylrings an den Phenylring gebunden ist.

Bevorzugt sind auch solche Verbindungen der Formel (I), in welchen $R^4$ und $R^5$ für Wasserstoff stehen.

Bevorzugt sind auch solche Verbindungen der Formel (I), in welchen $R^6$ für Fluor steht.

Besonders bevorzugt sind solche Verbindungen der Formel (I), in welchen $R^6$ für Fluor steht und $R^6$, wie in Formel

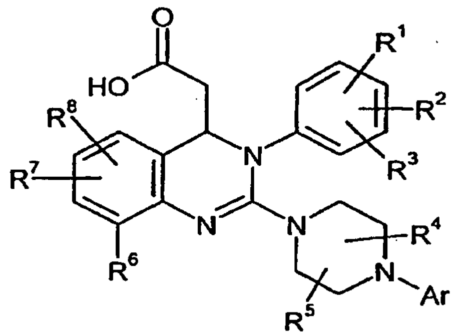

(Ia)

beschrieben, an den Aromaten des Dihydrochinazolines gebunden ist.

Bevorzugt sind auch solche Verbindungen der Formel (I), in welchen $R^7$ für Wasserstoff steht.

Bevorzugt sind darunter besonders solche Verbindungen der Formel (I), in welchen $R^8$ für Wasserstoff, Methyl oder Fluor steht.

Bevorzugt sind darunter ganz besonders solche Verbindungen der Formel (I), in welchen $R^8$ für Wasserstoff steht.

Bevorzugt sind auch solche Verbindungen der Formel (I), in welchen Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), wobei Verbindungen der Formel

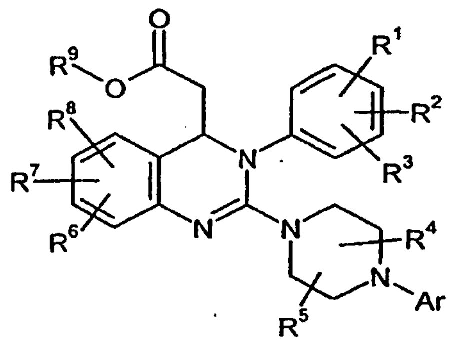

(II),

in welcher

| | |
|---|---|
| Ar, $R^1$, $R^2$, $R^3$, $R^4$, $R^5$, $R^6$, $R^7$ und $R^8$ | die oben angegebene Bedeutung haben und |
| $R^9$ | für Alkyl, bevorzugt für Methyl oder Ethyl oder für tert.-Butyl, steht, |

mit Basen oder Säuren umgesetzt werden.

Die Umsetzung erfolgt im Falle von Methyl und Ethyl im allgemeinen mit Basen in inerten Lösungsmitteln, bevorzugt in einem Temperaturbereich von Raumtemperatur bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, gegebenenfalls in wässriger Lösung, bevorzugt ist Natriumhydroxid in Wasser.

Inerte Lösungsmittel sind beispielsweise Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Gemischen von Lösungsmitteln, bevorzugt ist Dioxan oder Tetrahydrofuran.

Die Umsetzung erfolgt im Falle von tert.-Butyl im allgemeinen mit Säuren in inerten Lösungsmitteln, bevorzugt in einem Temperaturbereich von 0°C bis 40°C bei Normaldruck.

Als Säuren eignen sich hierbei Chlorwasserstoff in Dioxan, Bromwasserstoff in Essigsäure oder Trifluoressigsäure in Methylenchlorid.

Die Verbindungen der Formel (II) sind bekanntoder können hergestellt werden, indem Verbindungen der Formel

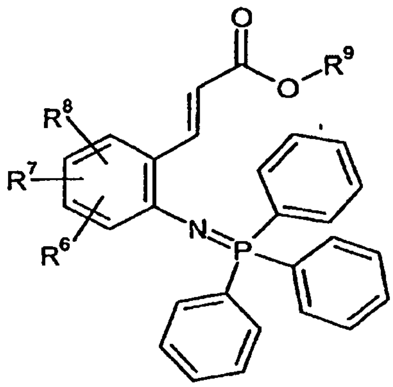

(III),

in welcher

$R^6$, $R^7$, $R^8$ und $R^9$ die oben angegebene Bedeutung haben,

in einer zweistufigen Reaktion zunächst mit Verbindungen der Formel

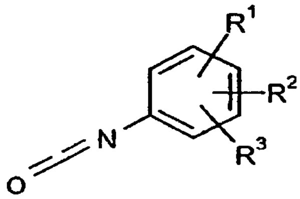

(IV),

in welcher

$R^1$, $R^2$ und $R^3$ die oben angegebene Bedeutung haben,

und anschließend mit Verbindungen der Formel

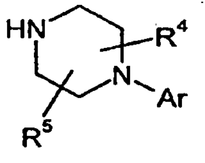

(V),

in welcher

Ar, $R^4$ und $R^5$ die oben angegebene Bedeutung haben,

umgesetzt werden.

Die Umsetzung erfolgt in beiden Stufen im allgemeinen in inerten Lösungsmitteln, bevorzugt in einem Temperaturbereich von Raumtemperatur bis 100°C bei Normaldruck. In der zweiten Stufe wird gegebenenfalls Kieselgel zu der Reaktionsmischung dazugegeben. Die Umsetzung erfolgt bevorzugt mit einer Aufarbeitung zwischen der ersten und der zweiten Stufe.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Etherwie Diethylether, Methyl-tert.-butylether, 1,2-Dimethoxyethan, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Essigsäureethylester, oder Gemischen von Lösungsmitteln, bevorzugt ist Methylenchlorid.

Die Verbindungen der Formel (IV) sind bekannt oder lassen sich nach bekannten Verfahren aus den entsprechenden Edukten synthetisieren.

Die Verbindungen der Formel (V) sind bekannt oder lassen sich nach bekannten Verfahren aus den entsprechenden Edukten synthetisieren, beispielsweise durch eine Buchwald-Hartwig-Reaktion nach folgendem Syntheseschema (Übersicht in: C.G. Frost, P. Mendonca, J. Chem. Soc., Perkin Trans I, 1998, 2615-2623):

Buchwald-Hartwig-Reaktion:

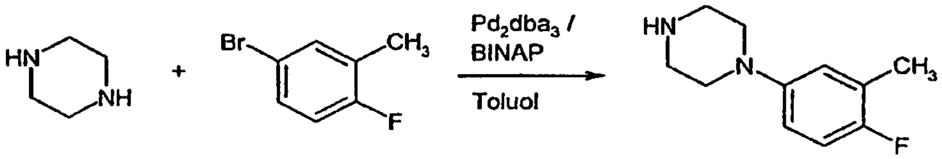


Die dafür benötigten Edukte sind bekannt oder lassen sich nach bekannten Verfahren aus den entsprechenden Edukten synthetisieren.

Die Verbindungen der Formel (III) sind bekannt oder können hergestellt werden, indem Verbindungen der Formel

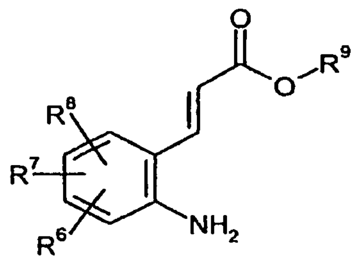

(VI),

in welcher

$R^6$, $R^7$, $R^8$ und $R^9$ die oben angegebene Bedeutung haben,

mit Triphenylphosphin und Tetrachlorkohlenstoff umgesetzt werden.

Die Umsetzung erfolgt im allgemeinen in inerten Lösungsmitteln, in Gegenwart einer Base, bevorzugt in einem Temperaturbereich von Raumtemperatur bis 50°C bei Normaldruck.

Inerte Lösungsmittel sind beispielsweise Ether wie Diethylether, Methyl-tert.-butylether, 1,2-Dimethoxyethan, Dioxan, Tetrahydrofuran, Glykoldimethyletheroder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril oder Pyridin, bevorzugt ist Acetonitril.

Basen sind beispielsweise Alkali- und Erdalkalicarbonate wie Cäsiumcarbonat, Natrium- oder Kaliumcarbonat oder Amine wie Triethylamin, Diisopropylethylamin, N-Methylmorpholin oder Pyridin, bevorzugt ist Triethylamin.

Die Verbindungen der Formel (VI) sind bekannt oder lassen sich nach bekannten Verfahren aus den entsprechenden Edukten synthetisieren, beispielsweise durch eine Heck-Reaktion oder eine Wittig-Horner-Reaktion nach fol-

genden Syntheseschemata:

Heck-Reaktion:

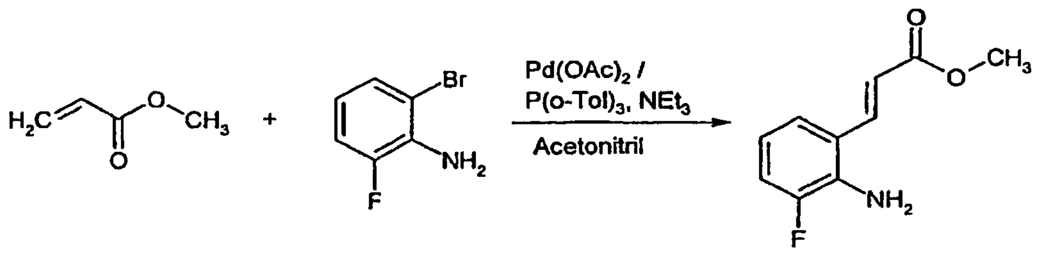


Wittig-Horner-Reaktion:

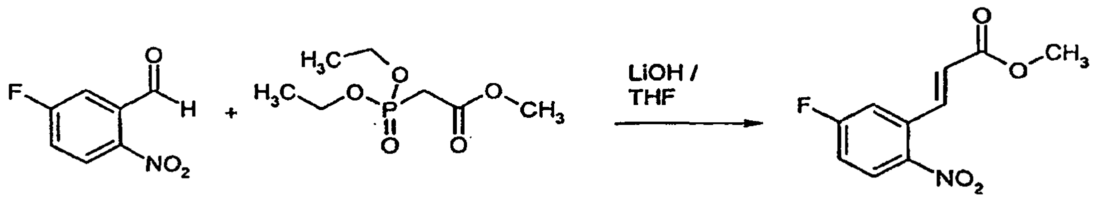


Die dafür benötigten Edukte sind bekannt oder lassen sich nach bekannten Verfahren aus den entsprechenden Edukten synthetisieren.

Die Herstellung der erfindungsgemäßen Verbindungen kann durch das folgende Syntheseschema verdeutlicht werden.

Syntheseschema:

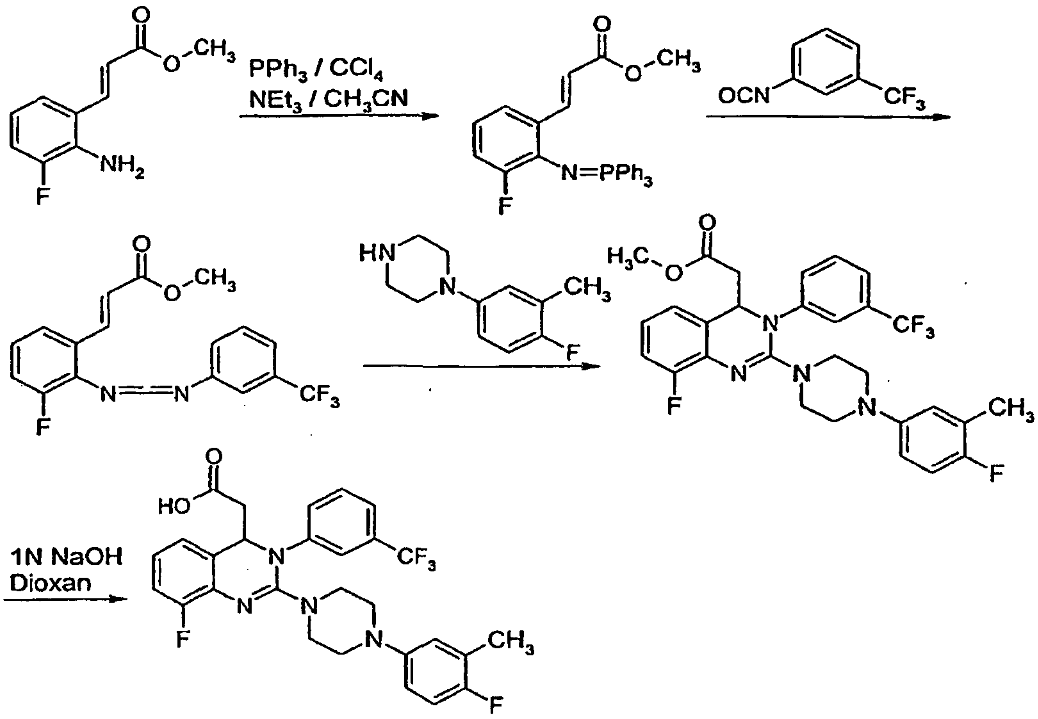


Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).

Als Indikationsgebiete können beispielsweise genannt werden:

1) Behandlung und Prophylaxe von HCMV-Infektionen bei AIDS-Patienten (Retinitis, Pneumonitis, gastrointestinale Infektionen).

2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

3) Behandlung und Prophylaxe von HCMV-Infektionen bei Neugeborenen und Kleinkindern.

4) Behandlung einer akuten HCMV-Infektion bei Schwangeren.

5) Behandlung der HCMV-Infektion bei immunsupprimierten Patienten bei Krebs und KrebsTherapie.

6) Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Weiterer Gegenstand der vorliegenden Erfindung ist die Verwendung der erfindungsgemäßen Verbindungen zur Herstellung eines Medikaments zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Herstellung eines Medikaments zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur

Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: antivirale Wirkstoffe wie Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, iritraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

## A. Beispiele

### Abkürzungen:

| | |
|---|---|
| ca. | circa |

| | |
|---|---|
| BINAP | 2,2'-Bis(diphenylphosphino)-1,1'-binaphthyl |
| $CDCl_3$ | Deuterochloroform |
| DC | Dünnschichtchromatographie |
| DCI | direkte chemische Ionisation (bei MS) |
| DCM | Dichlormethan |
| DIEA | *N,N*-Diisopropylethylamin |
| DMSO | Dimethylsulfoxid |
| DMF | *N,N*-Dimethylformamid |
| d. Th. | der Theorie |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| h | Stunde |
| HPLC | Hochdruck-, Hochleistungsflüssigchromatographie |
| konz. | konzentriert |
| LC-MS | Flüssigchromatographie-gekoppelte Massenspektroskopie |
| LDA | Lithium-Diisopropylamid |
| min | Minuten |
| MS | Massenspektroskopie |
| MTBE | Methyl-tert.-butylether |
| NMR | Kernresonanzspektroskopie |
| Pd-C | Palladium auf Kohle |
| proz. | prozentig |
| RP-HPLC | Reverse Phase HPLC |
| RT | Raumtemperatur |
| $R_t$ | Retentionszeit (bei HPLC) |
| THF | Tetrahydrofuran |

**Allgemeine Methoden LC-MS und HPLC :**

**Methode 1 (analytische HPLC):** Säule: Kromasil C18 60 mm x 2 mm; Temperatur: 30°C; Fluss: 0.75 ml/min; Eluent A: 0.005 M $HClO_4$, Eluent B: Acetonitril; Gradient: → 0.5 min 98%A, → 4.5 min 10%A, → 6.5 min 10%A.

**Methode 2 (präparative HPLC):** Säule: GromSil C18, 250 mm x 30 mm; Fluss: 50 ml/min; Laufzeit: 38 min; Detektion: 210 nm; Eluent A: Wasser, Eluent B: Acetonitril, Gradient: 10%B (3 min) -> 90%B (31 min) -> 90%B (34 min) -> 10%B (34.01 min).

**Methode 3 (LC-MS):** Säule: GromSil 120 ODS-4 HE, 50 mm x 2.0 mm, 3 µm; Eluent A: 1l Wasser + 1ml 50%ige Ameisensäure, Eluent B: 1l Acetonitril + 1ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.

**Methode 4 (präparative HPLC, Enantiomerentrennung, Carbonsäuren): Säule:** Packungsmaterial Chiraler Kieselgelselektor KBD 8361 (420 mm x 100 mm) basierend auf dem Selektor Poly(N-methacryloyl-L-leucin-1-menthylamid); Temperatur: 23°C; Eluent: Methyl-tert.-butylether; Fluss: 100 ml/min; Verbindung gelöst in Methyl-tert.-butylether/Essigsäureethylester (9:1).

**Methode 5 (präparative HPLC):** Säule: GromSil C18, 250 mm x 30 mm; Fluss: 50 ml/min; Laufzeit: 38 min; Detektion: 210 nm; Eluent A: Wasser mit 0.1% Ameisensäure, Eluent B: Acetonitril, Gradient: 10%B (3 min) -> 90%B (31 min) -> 90%B (34 min) -> 10%B (34.01 min).

**Methode 6 (analytische HPLC):** Instrument: HP 1100 mit DAD-Detektion; Säule: Kromasil RP-18, 60 mm x 2 mm, 3.5 µm; Eluent A: 5 ml $HClO_4$/l Wasser, Eluent B: Acetonitril; Gradient: 0 min 2%B, 0.5 min 2%B, 4.5 min 90%B, 9 min 90%B; Fluss: 0.75 ml/min; Temp.: 30°C, Detektion: UV 210 nm.

**Methode 7 (LC-MC):** Instrument: Micromass Platform LCZ mit HPLC Agilent Serie 1100; Säule: Grom-SIL120 ODS-4 HE, 50 mm x 2.0 mm, 3 µm; Eluent A: 1l Wasser + 1ml 50%ige Ameisensäure, Eluent B: 1l Acetonitril + 1ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 210 nm.

**Methode 8 (LC-MC):** Instrument: Micromass Platform LCZ, HP1100; Säule: Symmetry C18, 50 mm x 2.1 mm, 3.5 µm; Eluent A: Acetonitril + 0.1% Ameisensäure, Eluent B: Wasser + 0.1% Ameisensäure; Gradient: 0.0 min 10%A

$\rightarrow$ 4.0 min 90%A $\rightarrow$ 6.0 min 90%A; Ofen: 40°C; Fluss: 0.5 ml/min; UV-Detektion: 208-400 nm.

**Methode 9 (LC-MC):** Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure/ 1; Eluent B: Acetonitril + 500 µl 50%ige Ameisensäure/ 1; Gradient: 0.0 min 10%B $\rightarrow$ 3.0 min 95%B $\rightarrow$ 4.0 min 95%B; Ofen: 35°C; Fluss: 0.0 min 1.0 ml/min $\rightarrow$ 3.0 min 3.0 ml/min $\rightarrow$ 4.0 min 3.0 ml/min; UV-Detektion: 210 nm.

**Methode 10 (LC-MC): Gerätetyp MS:** Micromass ZQ; Gerätetyp HPLC: HP 1100 Series; UV DAD; Säule: Grom-Sit 120 ODS-4 HE 50 mm x 2 mm, 3.0 µm; Eluent A: Wasser + 500 µl 50%ige Ameisensäure/ 1, Eluent B: Acetonitril + 500 µl 50%ige Ameisensäure/ 1; Gradient: 0.0 min 0%B $\rightarrow$ 2.9 min 70%B $\rightarrow$ 3.1 min 90%B $\rightarrow$ 4.5 min 90%B; Ofen: 50°C; Fluss: 0.8 ml/min; UV-Detektion: 210 nm.

**Methode 11 (präparative HPLC, Enantiomerentrennung):** Säule: Packungsmaterial Chiraler Kieselgelselektor KBD 8361 (250 mm x 20 mm) basierend auf dem Selektor Poly(N-methacryloyl-L-leucin-1-menthylamid); Temperatur: 23°C; Eluent: Methyl-tert.-butylether + 5% Essigsäureethylester; Fluss: 25 ml/min.

**Methode 12 (präparative HPLC, Enantiomerentrennung):** Säule: Packungsmaterial Chiraler Kieselgelselektor KBD 5326 (250 mm x 20 mm) basierend auf dem Selektor Poly(N-methacryloyl-L-leucin-dicyclopropylmethylamid); Temperatur: 23°C; Eluent: Methyl-tert.-butylether + 5% Essigsäureethylester; Fluss: 25 ml/min.

**Methode 13 (präparative HPLC, Enantiomerentrennung):** Säule: Packungsmaterial Chiraler Kieselgelselektor KBD 8361 (250 mm x 20 mm) basierend auf dem Selektor Poly(N-methacryloyl-L-leucin-1-menthylamid); Temperatur: 23°C; Eluent: Methyl-tert.-butylether; Fluss: 25 ml/min.

**Methode 14 (präparative HPLC, Enantiomerentrennung, Ester):** Säule: Packungsmaterial Chiraler Kieselgelselektor KBD 8361 (420 mm x 100 mm) basierend auf dem Selektor Poly(N-methacryloyl-L-leucin-1-menthylamid); Temperatur: 23°C; Eluent: i-Hexan/Essigsäureethylester 85/15 v/v; Fluss: 100 ml/min; Verbindung gelöst in i-Hexan/ Essigsäureethylester (85:15).

**Methode 15 (präparative HPLC, Enantiomerentrennung, Ester):** Säule: Packungsmaterial Chiraler Kieselgelselektor KBD 8361 (420 mm x 100 mm) basierend auf dem Selektor Poly(N-methacryloyl-L-leucin-1-menthylamid); Temperatur: 23°C; Eluent: Methyl-tert.-butylether; Fluss: 100 ml/min; Verbindung gelöst in Methyl-tert.-butylether.

**Methode 16 (LC-MS):** Instrument: Micromass Platform LCZ mit HPLC Agilent Serie 1100; Säule: Grom-SIL120 ODS-4 HE, 50 mm x 2.0 mm, 3 µm ; Eluent A: 1 1 Wasser + 1 ml 50%ige Ameisensäure, Eluent B: 1 1 Acetonitril + 1 ml 50%ige Ameisensäure; Gradient: 0.0 min 100%A $\rightarrow$ 0.2 min 100%A $\rightarrow$ 2.9 min 30%A $\rightarrow$ 3.1 min 10%A $\rightarrow$ 4.5 min 10%A; Ofen: 55°C; Fluss: 0.8 ml/min; UV-Detektion: 208-400 nm.

**Methode 17 (LC-MS):** Gerätetyp MS: Micromass ZQ; Gerätetyp HPLC: Waters Alliance 2790; Säule: Grom-Sil 120 ODS-4 HE 50 mm x 2 mm, 3.0 µm; Eluent A: Wasser + 500µl 50%ige Ameisensäure/1: Eluent B: Acetonitril + 500µl 50%ige Ameisensäure/l; Gradient: 0.0 min 0%B$\rightarrow$ 0.2 min 0%B$\rightarrow$ 2.9 min 70%B$\rightarrow$ 3.1 min 90%B$\rightarrow$ 4.5 min 90%B; Ofen: 45°C; Fluss: 0.8 ml/min; UV-Detektion: 210 nm.

## Ausgangsverbindungen

### Allgemeine Arbeitsvorschrift [A]: Synthese von substituierten 2-Aminozimtsäurederivaten mittels Heck-Kupplung aus 2-halogensubstituierten Anilinen

In einem Einhalskolben werden 1.0 Äquivalente eines Arylhalogenids mit 1.6 Äquivalenten Acrylsäuremethylester bzw. Acrylsäure-tert.-butylester, 2.0 Äquivalenten Triethylamin, 0.03 Äquivalenten Palladium(II)acetat und 0.03 Äquivalenten Tri-o-tolylphosphin in Acetonitril vorgelegt (ca. IM-Lösung). Man lässt das Gemisch unter Rückfluss für 48 Stunden rühren. Nach beendeter Reaktion (Reaktionskontrolle mittels DC) wird das Lösemittel entfernt. Der Rückstand wird über Kieselgel mit Cyclohexan/Essigsäureethylester = 8:2 v/v chromatographisch gereinigt.

### Beispiel 1A

(2E)-3-[2-Amino-3-fluorphenyl]-propensäuremethylester

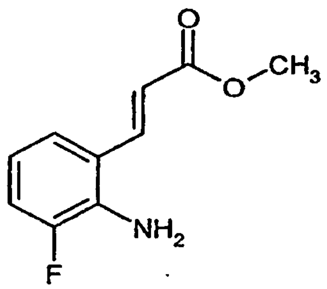

Ausgehend von 42.00 g (221.04 mmol) 2-Brom-6-fluoranilin werden nach der allgemeinen Arbeitsvorschrift [A] 29.66 g (68% d. Th.) Produkt erhalten.
HPLC (Methode 1): $R_t$= 4.14 min
MS (ESI-pos): m/z = 196 $(M+H)^+$

**Beispiel 2A**

2-Amino-3-[(1E)-3-methoxy-3-oxo-1-propenyl]benzoesäuremethylester

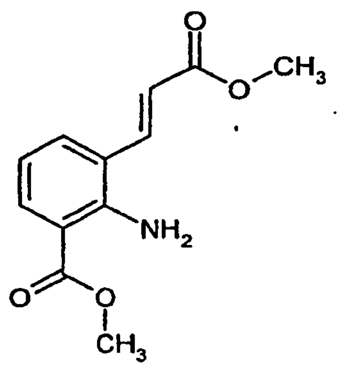

Ausgehend von 2.00 g (8.69 mmol) 2-Amino-3-brombenzoesäuremediylester werden nach der allgemeinen Arbeitsvorschrift [A] 1.29 g (60% d. Th.) Produkt erhalten.
HPLC (Methode 1): $R_t$=4.42 min
MS (ESI-pos): m/z = 236 $(M+H)^+$

**Beispiel 3A**

(2E)-3-(2-Amino-3,5-difluorphenyl)-2-propensäuremethylester

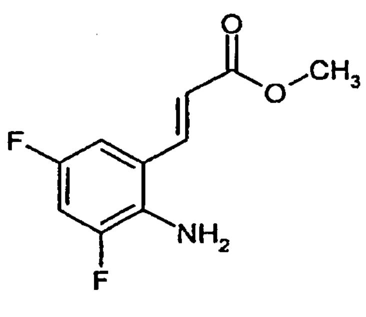

Ausgehend von 3.00 g (14.42 mmol) 2-Brom-4,6-difluoranilin werden nach der allgemeinen Arbeitsvorschrift [A] 1.41 g (45% d. Th.) Produkt erhalten.
HPLC (Methode 1): $R_t$ = 4.23 min
MS (ESI-pos): m/z = 214 $(M+H)^+$

**Beispiel 4A**

4-Amino-3-[(1*E*)-3-methoxy-3-oxo-1-propenyl]benzoesäuremethylester

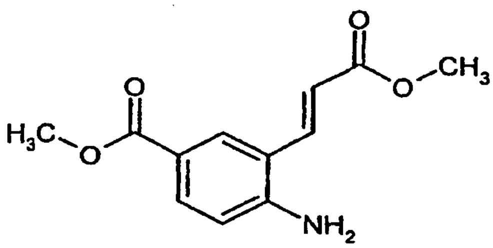

Ausgehend von 25.00 g (90.23 mmol) 4-Amino-3-iod-benzoesäuremethylester werden nach der allgemeinen Arbeitsvorschrift [A] 24.31 g (92% d. Th.) Produkt erhalten.
HPLC (Methode 1): $R_t$= 4.71 min
MS (ESI-pos): m/z = 278 $(M+H)^+$

**Beispiel 5A**

(2E)-3-[2-Amino-5-cyanophenyl]-2-propensäuremethylester

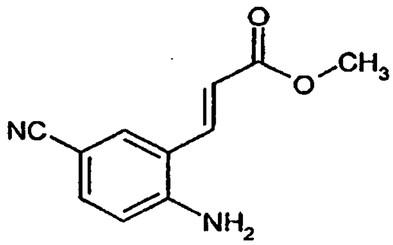

Ausgehend von 1.90 g (9.64 mmol) 3-Brom-4-aminobenzonitril werden nach der allgemeinen Arbeitsvorschrift [A] 1.28 g (50% d. Th.) Produkt erhalten.
HPLC (Methode 1): $R_t$= 2.85 min
MS (DCI-pos): m/z = 220 $(M+NH_4)^+$

**Allgemeine Arbeitsvorschrift [B]: Synthese von substituierten 2-Nitrozimtsäure-Derivaten mittels Wittig-Horner-Reaktion aus 2-halogensubstituierten Benzaldehyden**

In einem 100 ml Einhalskolben werden 27.5 mmol Methyldiethylphosphonacetat, 25.0 mmol des Benzaldehyds mit 27.5 mmol Lithiumhydroxid in Tetrahydrofuran suspendiert. Nach beendeter Reaktion (Reaktionskontrolle mittels DC) wird der Ansatz mit gleichem Volumen Wasser versetzt. Man extrahiert die wässrige Phase dreimal mit Essigsäureethylester. Die vereinigten organischen Phasen werden mit gesättigter Natriumchlorid-Lösung gewaschen, über Natriumsulfat getrocknet und das Lösemittel entfernt. Das Produkt wird im Hochvakuum bei RT ohne weitere Reinigung getrocknet. Gegebenenfalls wird bei starker Verunreinigung säulenchromatographisch über Kieselgel mit Cyclohexan/Essigsäureethylester gereinigt.

**Beispiel 6A**

(2E)-3-(3-Methoxy-2-nitrophenyl)-2-propensäuremethylester

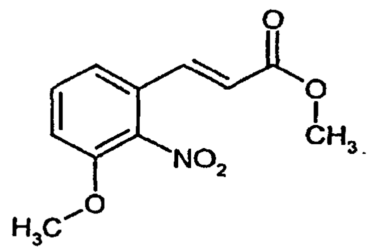

Ausgehend von 2.00 g (11.04 mmol) 3-Methoxy-2-nitrobenzaldehyd werden nach der allgemeinen Arbeitsvorschrift [B] 2.46 g (92% d. Th.) Produkt erhalten.
HPLC (Methode 1): $R_t$=4.37 min
MS (ESI-pos): m/z = 238 $(M+H)^+$

**Beispiel 7A**

(2E)-3-(5-Fluor-2-nitrophenyl)-2-propensäuremethylester

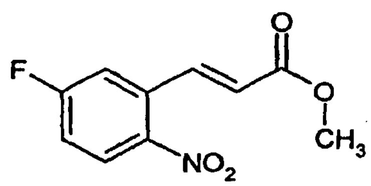

Ausgehend von 20.0g (118.3 mmol) 5-Fluor-2-nitrobenzaldehyd werden nach der allgemeinen Arbeitsvorschrift [B] 7.25 g (27% d. Th.) Produkt erhalten.
MS (DCI): m/z = 243 $(M+NH_4)^+$

**Allgemeine Arbeitsvorschrift [C]: Herstellung eines 2-Nitrobenzaldehyds aus einem Benzylhalogenid**

10.0 mmol des Benzylhalogenids werden mit 4.1 g Molekularsieb 4Å und 20.0 mmol *N*-Methylmorpholin-*N*-Oxid in 45 ml Acetonitril suspendiert. Man lässt bis zur Umsetzung (Reaktionskontrolle mittels DC) bei RT rühren. Nach beendeter Reaktion wird das Molekularsieb abfiltriert, das Lösungsmittel eingeengt und der Rückstand wieder in Essigsäureethylester aufgenommen. Diese Lösung wird zunächst mit 1N Salzsäure gewaschen und dann mit gesättigter Natriumchlorid-Lösung. Die abgetrennte organische Phase lässt man dann über Natriumsulfat trocknen und engt das Lösungsmittel wieder ein. Das Rohprodukt verfügt laut Analytik über eine genügend hohe Reinheit und kann direkt weiter umgesetzt werden.

**Beispiel 8A**

2-Fluor-6-nitrobenzaldehyd

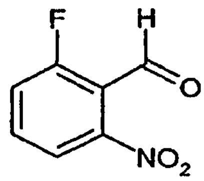

Ausgehend von 2.00 g (8.55 mmol) 3-Fluor-6-nitrobenzylbromid werden nach der allgemeinen Arbeitsvorschrift [C] 1.09 g (75% d. Th.) Produkt erhalten.
HPLC (Methode 1): $R_t$= 3.58 min

**Allgemeine Arbeitsvorschrift [D]: Reduktion der Nitrogruppe der 2-Nitrozimtsäurederivate**

In einem 250 ml Zweihalskolben werden unter Argon in 60 ml absolutem Ethanol 25 mmol der Nitroverbindung und 125 mmol Zinn-II-chloriddihydrat vorgelegt. Diese Suspension wird 30 Minuten unter Rückfluss gerührt, und es entsteht eine klare Lösung. Dann lässt man die Lösung auf Raumtemperatur abkühlen und gießt sie danach auf Eiswasser. Der pH-Wert wird entweder mit festem Natriumhydrogencarbonat oder mit einer gesättigten Natriumcarbonat-Lösung auf pH=7-8 eingestellt. Jetzt gibt man 60 ml Essigsäureethylester hinzu und filtriert die ausgefallenen Zinnsalze über Kieselgur (ca. 1 cm Schichtdicke) ab. Die organische Phase wird abgetrennt, und die wässrige Phase wird noch einmal mit Essigsäureethylester extrahiert. Man vereinigt die organischen Phasen und wäscht sie einmal mit gesättigter Natriumchlorid-Lösung, trocknet sie über Natriumsulfat und engt das Lösemittel ca. um die Hälfte ein. Nun fügt man Aktivkohle hinzu, entsprechend 1% des Gewichts der Nitroverbindung, und erhitzt für 30 Minuten unter Rückfluss (Verfärbung der Lösung). Die Aktivkohle wird abfiltriert und das Lösemittel eingeengt.
Als Rückstand verbleibt ein Öl, das bei Trocknung bei RT im Hochvakuum Kristalle ausbildet. Ohne weitere Aufreinigung erfolgt eine direkte Umsetzung zur nächsten Stufe.

**Beispiel 9A**

3-[2-Amino-6-fluorphenyl]-propensäuremethylester

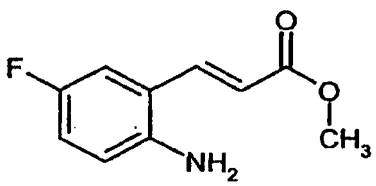

Ausgehend von 7.25 g (32.2 mmol) Nitroverbindung aus Beispiel 7A werden nach der allgemeinen Arbeitsvorschrift [D] 5.0 g (58% d. Th.) Produkt erhalten.
HPLC (Methode 1): $R_t$ = 3.33 min

**Allgemeine Arbeitsvorschrift [E1]: Synthese der Iminophosphorane mittels Appel-Reaktion der substituierten Aniline**

In einem 50 ml Einhalskolben werden 10.0 mmol des Amins des 2-Aminozimtsäureesters, 20.0 mmol Triphenylphosphin, 100.0 mmol Tetrachlorkohlenstoff und 100.0 mmol Triethylamin in 20 ml Acetonitril gelöst. Man lässt 2 Stunden bei Raumtemperatur rühren. Nach beendeter Reaktion (Reaktionskontrolle mittels DC oder analytischer HPLC) wird das Lösungsmittel im Vakuum entfernt und der Rückstand wird durch Säulenchromatographie an Kieselgel mit Cyclohexan/Essigsäureethylester = 7:3 gereinigt.

**Beispiel 10A**

(2E)-3-{3-Fluor-2-[(triphenylphosphoranyliden)amino]phenyl}-propensäuremethylester

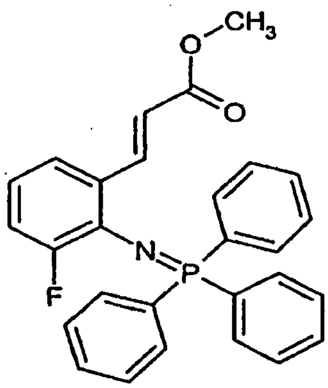

Ausgehend von 29.3 g (150.1 mmol) Aminverbindung aus Beispiel 1A werden nach der allgemeinen Arbeitsvorschrift [E] 55.0 g (80% d. Th.) Produkt erhalten.
HPLC (Methode 1): $R_t$ = 4.46 min
MS (ESI-pos): m/z = 456 $(M+H)^+$

**Beispiel 11A**

(2E)-3-{5-Fluor-2-[(triphenylphosphoranyliden)amino]phenyl}-propensäuremethylester

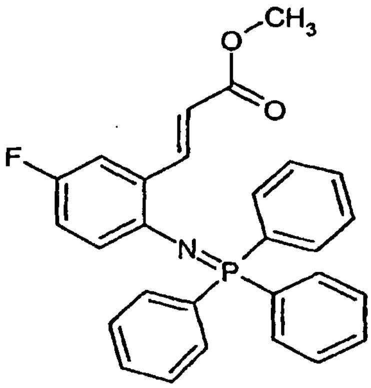

Ausgehend von 50.0 g (256.2 mmol) Aminverbindung aus Beispiel 9A werden nach der allgemeinen Arbeitsvorschrift [E] 89.6 g (77% d. Th.) Produkt erhalten.
HPLC (Methode 1): $R_t$ = 4.36 min
MS (ESI-pos): m/z = 456 $(M+H)^+$

**Beispiel 12A**

(2E)-3-{S-Cyano-2-[(triphenylphosphoranyliden)amino]phenyl}-propensäuremethylester

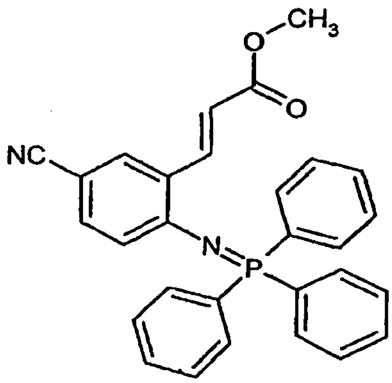

Ausgehend von 1.24 g (4.60 mmol) Aminverbindung aus Beispiel 5A werden nach der allgemeinen Arbeitsvorschrift [E] 2.12 g (92% d. Th.) Produkt erhalten.
HPLC (Methode 1): $R_t$ = 4.42 min
MS (ESI-pos): m/z = 463 $(M+H)^+$

**Allgemeine Arbeitsvorschrift [F]: Synthese von Phenylpiperazinen via Buchwald-Hartwig-Reaktion**

Zur Reaktionsvorbereitung wird der Reaktionskolben am Hochvakuum gründlich ausgeheizt und beim Belüften mit Argon gefüllt. In den Kolben werden 1.0 Äquivalente Bromarlyverbindung und 6.0 Äquivalente Piperazin in absolutem Toluol vorgelegt (0.2-0.3M Lösung der Bromverbindung). Dann werden 0.01 Äquivalente Tris(dibenzylidenaceton)dipalladium sowie 0.03 Äquivalente BINAP zugegeben. Das Reaktionsgemisch wird 16 h unter Rückfluss gerührt. Anschließend wird der Ansatz einmal mit Wasser extrahiert, die organische Phase zweimal mit 1N Salzsäure extrahiert, die wässrige Phase mit 1N Natronlauge auf pH 8 gestellt und dreimal mit Dichlormethan extrahiert. Die vereinigten organischen Phasen werden über Natriumsulfat getrocknet, filtriert, das Lösungsmittel im Vakuum entfernt und das Produkt über Nacht im Hochvakuum getrocknet.

**Beispiel 13A**

N-(4-Fluor-3-methylphenyl)-piperazin

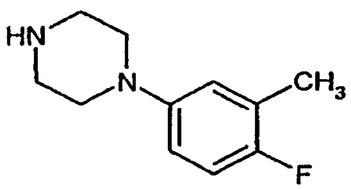

Ausgehend von 5.0 g (26.5 mmol) 4-Fluor-3-methyl-1-brombenzol werden nach der allgemeinen Arbeitsvorschrift [F] 4.52 g (83% d. Th.) Produkt erhalten.
HPLC (Methode 1): $R_t$= 3.54 min
MS (ESI pos): m/z = 195 $(M+H)^+$

**Beispiel 14A**

*N*-(4-Fluorpenyl)-3-methylpiperazin

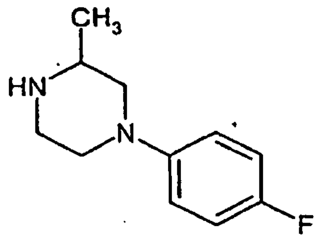

Ausgehend von 1.0 g (5.71 mmol) 4-Fluor-3-methyl-1-brombenzol werden nach der allgemeinen Arbeitsvorschrift [F] 0.57 g (49% d. Th.) Produkt erhalten.
HPLC (Methode 1): $R_t$ = 3.37 min
MS (DCI pos): m/z = 195 $(M+H)^+$

**Beispiel 15A**

1-(3-Fluorphenyl)-piperazin

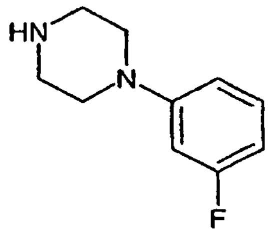

In 20 ml Toluol werden 1 g (5.71 mmol) 3-Fluorbrombenzol und 2.95 g (34.29 mmol) Piperazin gelöst und mit 0.77 g (8 mmol) Natrium-tert.-butylat versetzt. Anschließend wird in Anwesenheit von 0.11 g (0.17 mmol) BINAP und 0.05 g (0.06 mmol) Tris(dibenylidenaceton)dipalladium über Nacht unter Rückfluss gerührt. Nach Abkühlen wird Essigsäureethylester zugegeben und mit Wasser gewaschen. Anschließend wird mit 1 N Salzsäure extrahiert und die wässrige Phase mit Essigsäureethylester gewaschen. Nach Einstellen des pH-Werts auf 8-9 wird mit Dichlormethan extrahiert. Nach Trocknen der organischen Phase über Magnesiumsulfat und Entfernen des Lösungsmittels wird die Zielverbindung erhalten.
Ausbeute: 0.8 g (78% d. Th.)
HPLC (Methode 1): $R_t$= 3.4 min
MS (ESI-pos): m/z = 181 $(M+H)^+$

**Beispiel 16A**

1-(3,4-Difluorphenyl)-piperazin

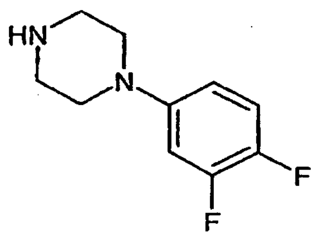

In 100 ml Toluol werden 5 g (25.91 mmol) 3,4-Difluorbrombenzol mit 13.39 g (155.45 mmol) Piperazin, 3.49 g (36.27 mmol) Natrium-tert.-butylat, 0.24 g (0.26 mmol) Tris(dibenylidenaceton)-dipalladium und 0.48 g (0.78 mmol)

BINAP über Nacht unter Rückfluss gerührt. Nach Zugabe von Essigsäureethylester wird mit Wasser gewaschen und die organische Phase mit 1 N Salzsäure extrahiert. Die wässrige Phase wird nun mit Essigsäureethylester gewaschen und anschließend auf pH 8 eingestellt. Das Produkt wird mit Dichlormethan aus der wässrigen Phase extrahiert. Anschließend wird über Magnesiumsulfat getrocknet, das Lösungsmittel entfernt und die Zielverbindung im Vakuum getrocknet.
Ausbeute: 3.85 g (75%.d. Th.)
HPLC (Methode 1): $R_t$= 3.4 min
MS (DCI): m/z = 199 $(M+H)^+$

**Beispiel 17A**

2-Isocyanato-1-methoxy-4-(trifluormethyl)benzol

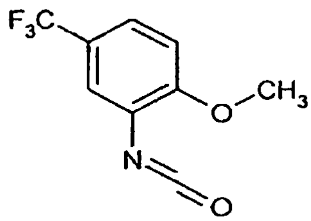

In 100 ml Dichlormethan werden 3 g (15.69 mmol) 2-Methoxy-5-trifluormethylanilin gelöst und mit 6.73 g (31.39 mmol) 1,8-Bis(dimethylamino)naphthalin versetzt. Bei 0-5°C werden 2.24 g (11.3 mmol) Chlorameisensäuretrichlormethylester, gelöst in 50 ml Dichlormethan, zugetropft und 30 min bei 0°C sowie 60 min bei Raumtemperatur gerührt. Bei 0°C wird mit 1N Salzsäure, Eiswasser und Natriumhydrogencarbonat-Lösung gewaschen. Nach Trocknen über Magnesiumsulfat und Abdestillieren des Lösungsmittels wird das Produkt erhalten. Das Isocyanat wird anschließend ohne weitere Reinigung in den folgenden Reaktionen umgesetzt.
Ausbeute: 3.00 g (88% d. Th.)

**Beispiel 18A**

(2E)-3-{3-Fluor-2-[({[2-methoxy-5-(trifluormethyl)phenyl]-imino}-methylen)-amino]phenyl)-2-propensäuremethylester

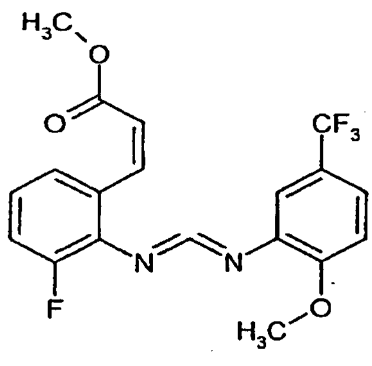

In 50 ml Dichlormethan werden 5.0 g (10.98 mmol) (2E)-3-{3-Fluor-2-[(triphenylphosphoranyliden)amino]-phenyl}-2-propensäuremethylester (Beispiel 10A) vorgelegt und mit 2.5 g (11.53 mmol) 2-Isocyanato-1-methoxy-4-(trifluormethyl)benzol (Beispiel 17A) über Nacht bei Raumtemperatur gerührt. Nach Abdestillieren des Lösungsmittels wird das Produkt durch Chromatographie an Kieselgel (Isohexan/Dichlormethan 2:1; 1:1) gereinigt und aus Isohexan umkristallisiert.
Ausbeute: 2.69 g (62% d. Th.)
HPLC (Methode 1): $R_t$= 5.6 min
MS (ESI-pos): m/z = 395 $(M+H)^+$

**Allgemeine Arbeitsvorschrift [G]: Umsetzung des Iminophosphorans mit einem Isocyanat und anschließende Umsetzung zum Dihydrochinazolin-Derivat mit einem Amin**

1.0 Äquivalente des Iminophosphorans werden in 20 ml Dichlormethan gelöst (0.1-0.2M Lösung). Danach werden 1.05 Äquivalente eines substituierten Isocyanats hinzugefügt, und man lässt bis zur Beendigung der Reaktion bei RT rühren. Eine Reaktionskontrolle erfolgt durch DC oder analytische HPLC.

Die so erhaltene Lösung des Carbodiimids in Dichlormethan wird mit 1.0 Äquivalenten Amin sowie einer Spatelspitze Kieselgel versetzt und bei Raumtemperatur bis zur vollständigen Umsetzung gerührt. Nach beendeter Reaktion (Reaktionskontrolle mittels DC oder HPLC) wird der Ansatz eingeengt und durch präparative HPLC an RP-Phase gereinigt.

Unter Umständen zeigt das NMR noch einen schwankenden Anteil an nicht-cyclisiertem Reaktionsprodukt an. In diesen Fällen wird das Gemisch aus cyclisiertem und nicht cyclisiertem Produkt in Dioxan aufgenommen, mit einer Spatelspitze Kieselgel versetzt und unter Rückfluss 30 min bis 16 h gerührt. Das Kieselgel wird abfiltriert und die Lösung für weitere Umsetzungen verwendet.

Sollen die enantiomerenreinen Verbindungen erhalten werden, so erfolgt die chromatografische Trennung auf dieser Stufe.

**Beispiel 19A**

{8-Fluor-2-[4-(4-fluorphenyl)-1-piperazinyl]-3-[3-(trifluormethyl)phenyl]-3,4-dihydro-4-chinazolinyl}essigsäuremethylester

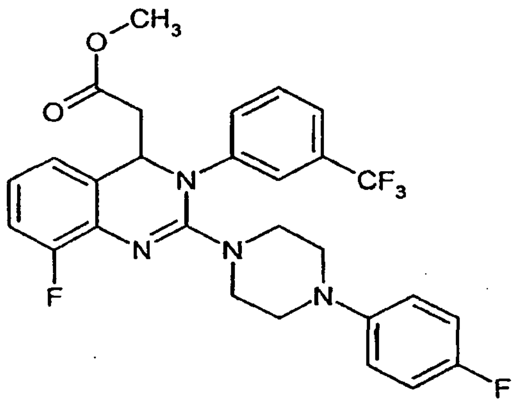

Ausgehend von 92.5 mg (0.2 mmol) Iminophosphoran aus Beispiel 10A werden nach der allgemeinen Arbeitsvorschrift [G] 50 mg (45% d. Th.) Produkt erhalten.
HPLC (Methode 1): $R_t$ = 4.81 min

**Beispiel 20A**

{8-Fluor-2-[4-(4-fluorphenyl)-1-piperazinyl)-3-(3-(trifluormethyl)phenyl]-3,4-dihydro-4-chinazolinyl}essigsäuremethylester

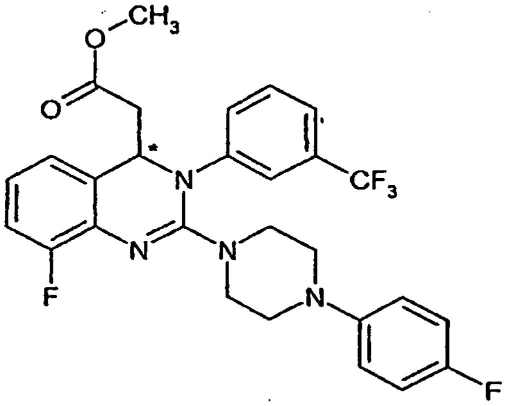

Diese Verbindung wird als Enantiomer A nach der Enantiomerentrennung von 3.84 g Beispiel 19A erhalten (715 mg, 14% d. Th.).
HPLC (Methode 1): $R_t$ = 4.81 min
MS (ESI-pos): m/z = 544.9 $(M+H)^+$

## Beispiel 21A

{6-Fluor-2-[4-(4-fluorphenyl)-1-piperazinyl]-3-[3-(trifluormethyl)phenyl]-3,4-dihydro-4-chinazolinyl}essigsäuremethylester

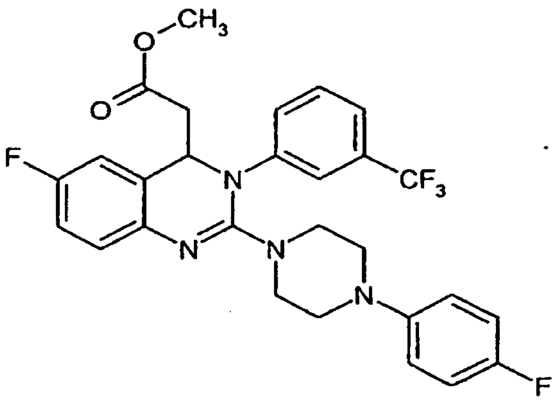

Ausgehend von 100 mg (0.28 mmol) Iminophosphoran aus Beispiel 11A werden nach der allgemeinen Arbeitsvorschrift [G] 58 mg (39% d. Th.) Produkt erhalten.
HPLC (Methode 1): $R_t$ = 4.80 min

## Beispiel 22A

{6-Fluor-2-(4-(4-fluorphenyl)-1-piperazinyl)-3-[3-(trifluonnethyl)phenyl]-3,4-dihydro-4-chinazolinyl}essigsäuremethylester

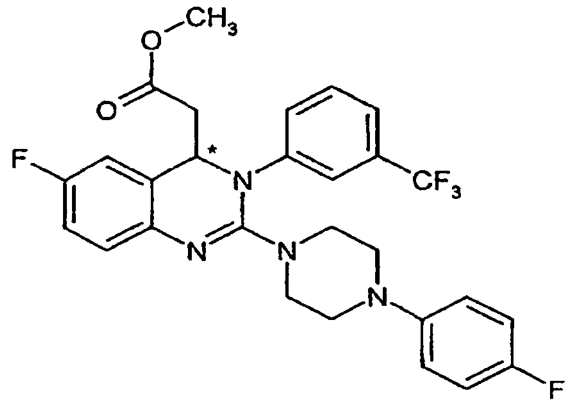

Diese Verbindung wird als Enantiomer A nach der Enantiomerentrennung von 832 mg Beispiel 21A erhalten (368 mg, 17% d. Th.).
HPLC (Methode 1): $R_t$ = 4.77 min
MS (ESI-pos): m/z= 544.9 $(M+H)^+$

**Beispiel 23A**

{8-Fluor-2-[4-(3-methylphenyl)-1-piperazinyl]-3-[3-(trifluormethyl)phenyl]-3,4-dihydro-4-chinazolinyl}essigsäuremethylester

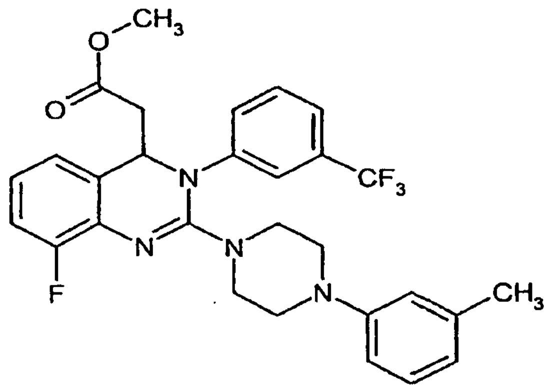

Ausgehend von 93 mg (0.2 mmol) Iminophosphoran aus Beispiel 10A werden nach der allgemeinen Arbeitsvorschrift [G] 43 mg (39% d. Th.) Produkt erhalten.
HPLC (Methode 1): $R_t$ = 4.80 min
MS (ESI-pos): m/z= 541.0 $(M+H)^+$

**Beispiel 24A**

{8-Fluor-2-[4-(3-methylphenyl)-1-piperazinyl]-3-[3-(trifluormethyl)phenyl]-3,4-dihydro-4-chinazolinyl}essigsäuremethylester

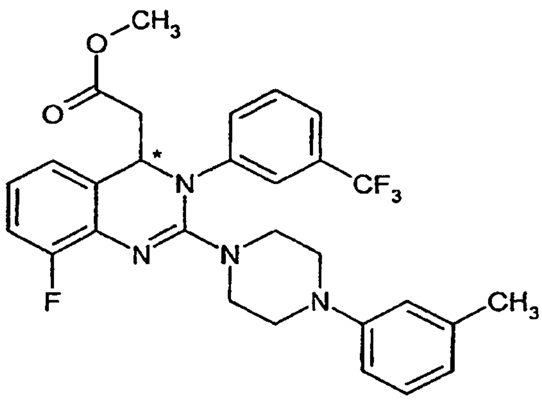

Diese Verbindung wird als Enantiomer A nach der Enantiomerentrennung von 3.31 g Beispiel 23A erhalten (1.18 g, 22% d. Th.).
HPLC (Methode 1): $R_t$ = 4.80 min
MS (ESI-pos): m/z = 541.0 $(M+H)^+$

## Beispiel 25A

{8-Fluor-2-[4-(3-methoxyphenyl)-1-piperazinyl]-3-[3-(trifluormethyl)phenyl]-3,4-dihydro-4-chinazolinyl}essigsäuremethylester

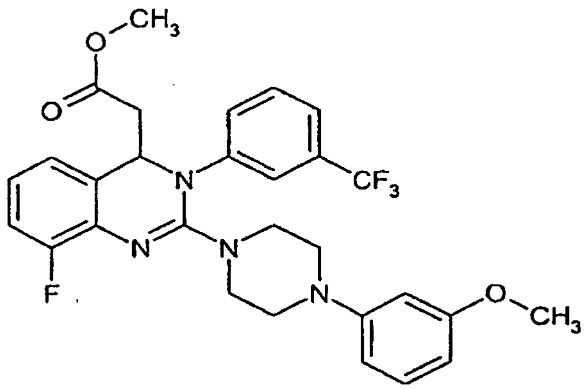

Ausgehend von 93 mg (0.2 mmol) Iminophosphoran aus Beispiel 10A werden nach der allgemeinen Arbeitsvorschrift [G] 51 mg (45% d. Th.) Produkt erhalten.
HPLC (Methode 1): $R_t$ = 4.62 min
MS (ESI-pos): m/z = 556.7 $(M+H)^+$

## Beispiel 26A

{8-Fluor-2-[4-(3-methoxyphenyl)-1-piperazinyl]-3-[3-(trifluormethyl)phenyl]-3,4-dihydro-4-chinazolinyl}essigsäuremethylester

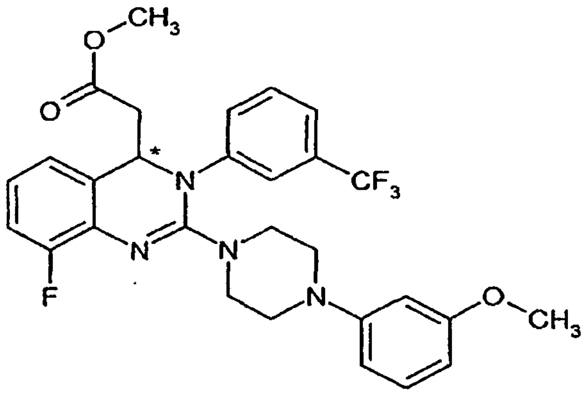

Diese Verbindung wird als Enantiomer A nach der Enantiomerentrennung von 5.11 g Beispiel 25A erhalten (0.49 g, 9% d. Th.).
HPLC (Methode 1): $R_t$ = 4.71 min
MS (ESI-pos): m/z = 556.8 $(M+H)^+$

## Beispiel 27A

{8-Fluor-2-[4-(4-fluör-3-methylphenyl)-1-piperazinyl]-3-[6-methoxy-3-(trifluor-methyl)phenyl]-3,4-dihydro-4-chinazolinyl}essigsäuremethylester

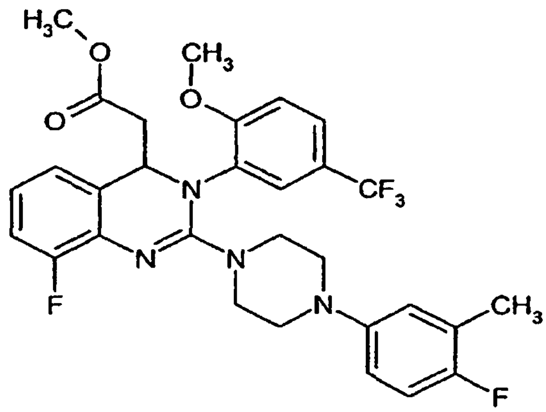

Ausgehend von 1.0 g (2.2 mmol) Iminophosphoran aus Beispiel 10A, 500 mg (2.31 mmol) 2-Isocyanato-1-methoxy-4-(trifluormethyl)benzol (Beispiel 17A) und 427 mg (2.2 mmol) Phenylpiperazin aus Beispiel 13A werden nach Filtration über Kieselgel (Cyclohexan/Essigsäureethylester 2:1 (v/v)) 1.03 g (79% d. Th.) Rohprodukt erhalten. Dieses wird ohne weitere Reinigung weiter umgesetzt.
LC-MS (Methode 3): $R_t$ = 2.55 min, 2.66 min
MS (ESI-pos): m/z = 589.3 $(M+H)^+$

## Beispiel 28A

{8-Fluor-2-[4-(4-fluor-3-methylphenyl)-1-piperazinyl]-3-[6-methoxy-3-1-methyl-phenyl]-3,4-dihydro-4-chinazolinyl}essigsäuremethylester

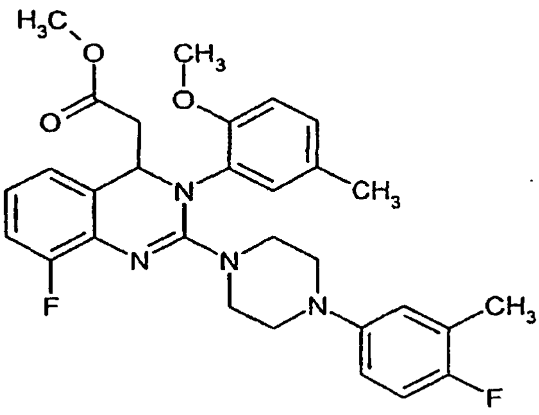

Ausgehend von 0.60 g (1.76 mmol) Iminophosphoran aus Beispiel 10A, 376 mg (2.31 mmol) 2-Methoxy-5-methylphenylisocyanat und 342 mg (1.76 mmol) Phenylpiperazin aus Beispiel 13A werden nach Reinigung mittels präparativer HPLC (Methode 5) 183 mg (16% d. Th.) Produkt erhalten.
HPLC (Methode 1): $R_t$ = 4.77 min
MS (ESI-pos): m/z = 535.2 $(M+H)^+$

## Beispiel 29A

{8-Fluor-2-[4-(4-fluorphenyl)-1-piperazinyl]-3-[6-methoxy-3-chlorphenyl]-3,4-dihydro-4-chinazolinyl}essigsäuremethylester

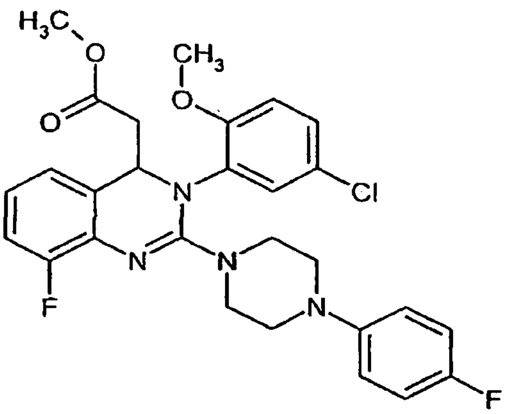

Ausgehend von 1.0 g (2.2 mmol) Iminophosphoran aus Beispiel 10A, 423 mg (2.31 mmol) 2-Methoxy-5-chlorphenylisocyanat und 396 mg (2.2 mmol) 4-Fluorphenylpiperazin werden nach Reinigung mittels präparativer HPLC (Methode 5) 621 mg (52% d. Th.) Produkt erhalten.
HPLC (Methode 1): $R_t$ = 4.75 min
MS (ESI-pos): m/z = 541.2 $(M+H)^+$

## Beispiel 30A

{8-Fluor-2-[4-(4-fluorphenyl)-1-piperazinyl]-3-[2-methoxy-5-(trifluormethyl)-phenyl]-3,4-dihydro-4-chinazolinyl}essigsäuremethylester

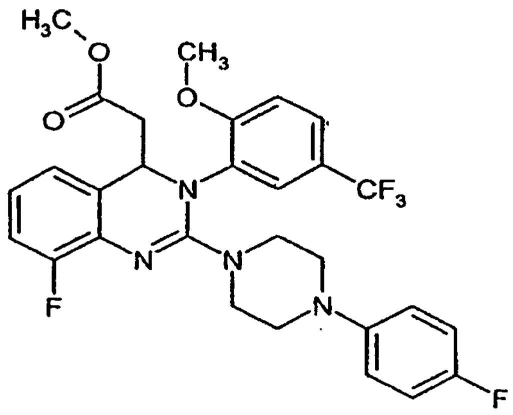

In 15 ml Dichlormethan werden 550 mg (1.39 mmol) (2E)-3-{3-Fluor-2-[({[2-methoxy-5-(trifluormethyl)phenyl]-imino}-methylen)amino]phenyl}-2-propensäuremethylester (Beispiel 18A) und 251 mg (1.39 mmol) 1-(4-Fluorphenyl)piperazin in Gegenwart einer Spatelspitze Kieselgel 1 Stunde gerührt. Nach 90 Stunden Rühren unter Rückfluss wird das Produkt durch Chromatographie an Kieselgel (Dichlormethan, Dichlormethan/Essigsäureethylester 10:1) gereinigt.
Ausbeute: 769 mg (96% d. Th.)
HPLC (Methode 1): $R_t$= 4.8 min
MS (ESI-pos): m/z = 575 $(M+H)^+$

## Beispiel 31A

{8-Fluor-2-[4-(3-methoxyphenyl)-1-piperazinyl]-3-[2-methoxy-5-(trifluormethyl)-phenyl]-3,4-dihydro-4-chinazolinyl}essigsäuremethylester

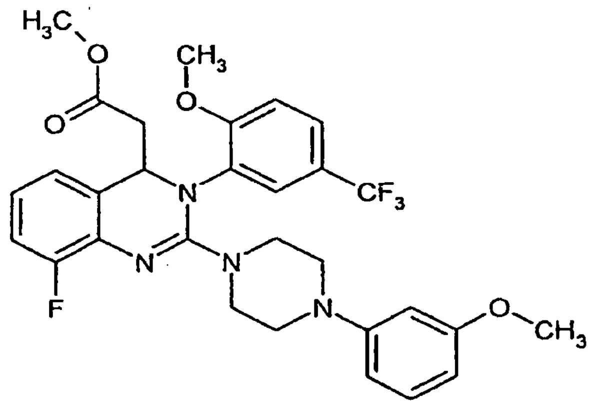

In 20 ml Dichlormethan werden 700 mg (1.78 mmol) (2E)-3-{3-Fluor-2-[({[2-methoxy-5-(trifluormethyl)phenyl]-imino}-methylen)amino]phenyl}-2-propensäuremethylester (Beispiel 18A) mit 341 mg (1.78 mmol) 1-(3-Methoxyphenyl)-piperazin und einer Spatelspitze Kieselgel eine Stunde bei Raumtemperatur und 35 Stunden unter Rückfluss gerührt. Nach Reinigung an Kieselgel (Dichlormethan, Dichlormethan/Essigsäureethylester 10:1) wird die Zielverbindung erhalten.
Ausbeute: 1012 mg (97% d. Th.)
HPLC (Methode 6): $R_t$ = 4.8 min
MS (ESI-pos): m/z = 587 $(M+H)^+$

**Beispiel 32A**

{8-Fluor-2-[4-(3,4-difluorphenyl)-1-piperazinyl]-3-[2-methoxy-5-(trifluormethyl)-phenyl]-3,4-dihydro-4-chinazolinyl}essigsäuremethylester

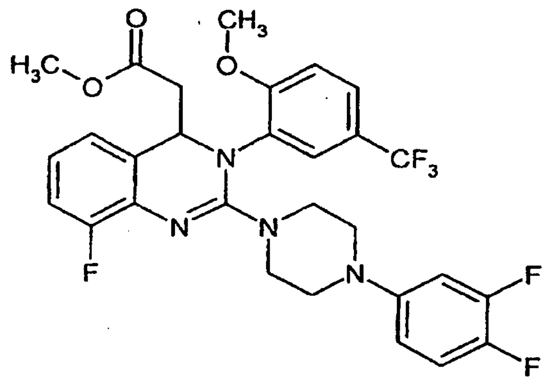

In 20 ml Dichlormethan werden 700 mg (1.78 mmol) (2E)-3-{3-Fluor-2-[({[2-methoxy-5-(trifluormethyl)phenyl]-imino}-methylen)amino]phenyl}-2-propensäuremethylester (Beispiel 18A) und 352 mg (1.78 mmol) 1-(3,4-Difluorphenyl)-piperazin (Beispiel 16A) mit einer Spatelspitze Kieselgel 1 Stunde bei Raumtemperatur und 20 Stunden unter Rückfluss gerührt. Anschließend wird die Zielverbindung durch Chromatographie an Kieselgel gereinigt (Dichlormethan, Dichlormethan/ Essigsäureethylester 10:1).
Ausbeute: 1027 mg (97% d. Th.)
HPLC (Methode 1): $R_t$ = 4.8 min
MS (ESI-pos): m/z=593 $(M+H)^+$

**Beispiel 33A**

(8-Fluor-2-[4-(3-methylphenyl)-1-piperazinyl]-3-[2-methoxy-5-(tritluormethyl)-phenyl]-3,4-dihydro-4-chinazolinyl}essigsäuremethylester

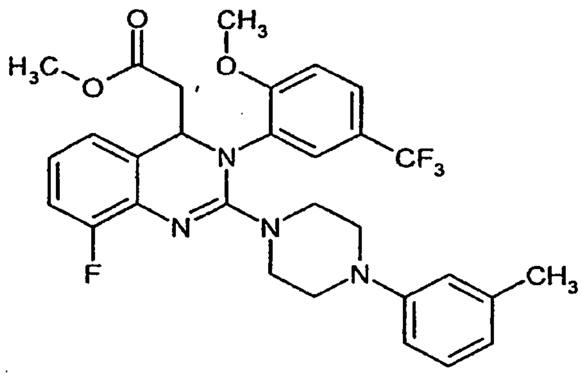

In 300 ml Dichlormethan werden 11.5 g (29.16 mmol) (2E)-3-{3-Fluor-2-[({[2anethoxy-5-(trifluormethyl)phenyl]-imino}-methylen)amino]phenyl}-2-propensäuremethylester (Beispiel 18A) und 5.14 g (29.16 mmol) 1-(3-Methylphenyl)-piperazin mit einer Spatelspitze Kieselgel 1 Stunde bei Raumtemperatur und 20 Stunden unter Rückfluss gerührt. Nach Chromatographie an Kieselgel (Dichlormethan, Dichlormethan/Essigsäureethylester 10:1, 5:1) wird das Produkt erhalten.
Ausbeute: 15.8 g (95% d. Th.)
HPLC (Methode 1): $R_t$=4.8 min
MS (ESI-pos): m/z= 571 $(M+H)^+$

**Beispiel 34A**

{8-Fluor-2-[4-(3-fluorphenyl)-1-piperazinyl]-3-[2-methoxy-5-(trifluonnethyl)-phenyl]-3,4-dihydro-4-chinazolinyl}essigsäuremethylester

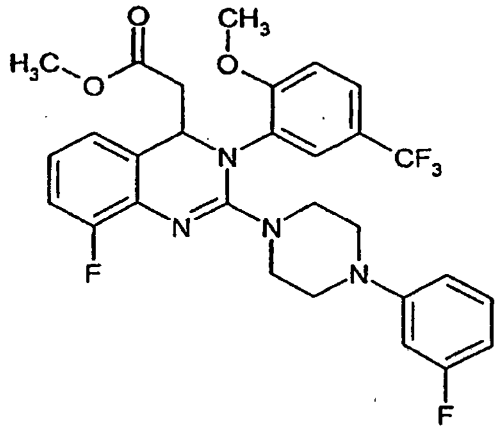

In 15 ml Dichlormethan werden 100 mg (0.25 mmol) (2E)-3-{3-Fluor-2-[({[2-methoxy-5-(trifluormethyl)phenyl]-imino}-methylen)amino]phenyl}-2-propensäuremethylester (Beispiel 18A) und 45.7 mg (0.25 mmol) 1-(3-Fluorphenyl)-piperazin (Beispiel 15A) mit einer Spatelspitze Kieselgel eine Stunde bei Raumtemperatur und 20 Stunden unter Rückfluss gerührt. Nach Chromatographie an Kieselgel (Dichlormethan, Dichlormethan/Essigsäureethylester 10:1) wird die Zielverbindung erhalten.
Ausbeute: 139.2 mg (96% d. Th.)
HPLC (Methode 1): $R_t$=4.8 min
MS (ESI-pos): m/z = 575 $(M)^+$

**Beispiel 35A**

(8-Fluor-2-[4-(3-chlorphenyl)-1-piperazinyl]-3-[3-(trifluormethyl)phenyl]-3,4-dihydro-4-chinazolinyl}essigsäuremethylester

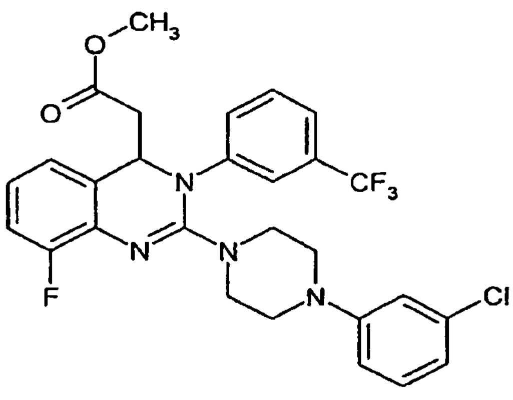

Ausgehend von 93 mg (0.2 mmol) Iminophosphoran aus Beispiel 10A werden nach der allgemeinen Arbeitsvorschrift [G] 51 mg (45% d. Th.) Produkt erhalten.
LC-MS (Methode 3): $R_t$ = 4.78 min
MS (ESI-pos): m/z = 561 $(M+H)^+$

**Beispiel 36A**

(8-Fluor-2-[4-(1,3-benzodioxol-5-yl)-1-piperazinyl]-3-[3-(trifluormethyl)phenyl]-3,4-dihydro-4-chinazolinyl}essigsäuremethylester

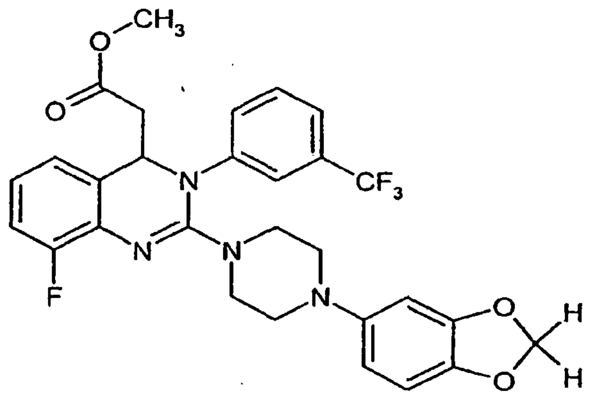

Ausgehend von 4.19 g (9.2 mmol) Iminophosphoran aus Beispiel 10A werden nach der allgemeinen Arbeitsvorschrift [G] 3.67 g (70% d. Th.) Produkt erhalten.
HPLC (Methode 1): $R_t$ = 4.67 min
MS (ESI-pos): m/z = 571 $(M+H)^+$

**Beispiel 37A**

4-Amino-3-[(1E)-3-tert.-butoxy-3-oxoprop-1-en-1-yl]benzoesäuremethylester

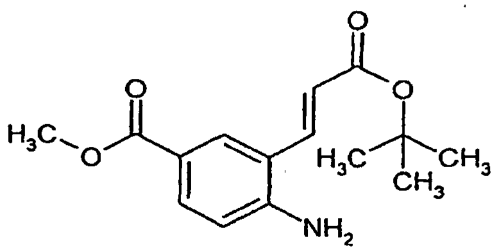

Ausgehend von 25.0 g (90.2 mmol) 4-Amino-3-iodbenzoesäuremethylester werden nach der allgemeinen Arbeitsvorschrift [A] 24.3 g (88% d. Th.) Produkt erhalten.
HPLC (Methode 1): $R_t$ = 4.71 min
MS (DCI-pos): m/z = 295 $(M+NH_4)^+$

**Beispiel 38A**

(2E)-3-(4-Cyano-2-nitrophenyl)-2-propensäuremethylester

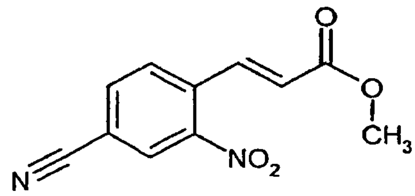

Ausgehend von 3.00 g (17.0 mmol) 4-Cyano-2-nitrobenzaldehyd werden nach der allgemeinen Arbeitsvorschrift [B] und Umkristallisation aus Methanol 2.51 g (63% d. Th.) Produkt erhalten.
HPLC (Methode 1): $R_t$= 4.06 min
MS (ESI-pos): m/z = 233 $(M+H)^+$

**Beispiel 39A**

3-[2-Amino-7-cyanophenyl]-propensäuremethylester

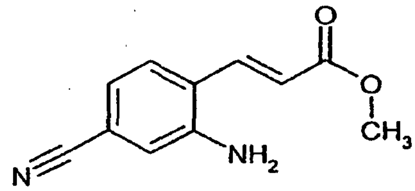

Ausgehend von 1.0 g (4.31 mmol) Nitroverbindung aus Beispiel 38A werden nach der allgemeinen Arbeitsvorschrift [D] (allerdings ohne Kochen über Aktivkohle) 793 mg (89% d. Th.) Produkt erhalten.
HPLC (Methode 1): $R_t$=3.99 min

**Beispiel 40A**

(2E)-3-{6-Cyano-2-[(triphenylphosphoranyliden)amino]phenyl}-propensäuremethylester

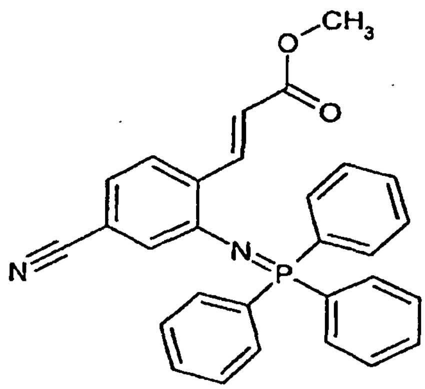

Ausgehend von 0.75 g (3.71 mmol) Aminverbindung aus Beispiel 39A werden nach der allgemeinen Arbeitsvorschrift [E] 1.09 g (62% d. Th.) Produkt erhalten.
HPLC (Methode 1): $R_t$ = 4.30 min
MS (ESI-pos): m/z = 463 $(M+H)^+$

**Beispiel 41A**

3-[(1E)-3-tert.-Butoxy-3-oxoprop-1-en-1-yl]-4-[(triphenylphosphoranyliden)amino]benzoesäuremethylester

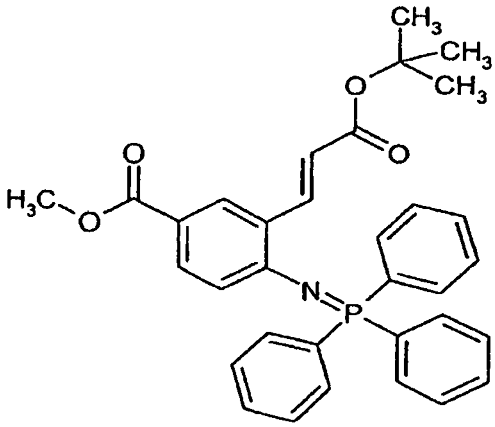

Ausgehend von 19.0 g (68.5 mmol) Aminverbindung aus Beispiel 37A werden nach der allgemeinen Arbeitsvorschrift [E] 31.4 g (85% d. Th.) Produkt erhalten.
HPLC (Methode 1): $R_t$ = 4.69 min
MS (ESI-pos): m/z = 538 $(M+H)^+$

**Beispiel 42A**

{8-Fluor-2-[4-(4-fluorphenyl)-1-piperazinyl]-3-[2-methoxy-5-(trifluormethyl)-phenyl]-3,4-dihydro-4-chinazolinyl}essigsäuremethylester

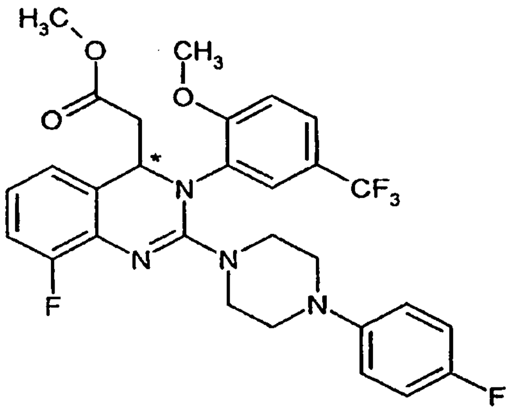

Die Verbindung erhält man als Enantiomer A, indem man das Racemat aus Beispiel 30A chromatographisch gemäß Methode 15 in die Enantiomeren auftrennt. Ausgehend von 231 g Racemat erhält man 120 g des Zielproduktes, das direkt weiter umgesetzt wird.
MS (ESI-pos): m/z = 575 $(M+H)^+$

**Beispiel 43A**

{8-Fluor-2-[4-(3-methoxyphenyl)-1-piperazinyl]-3-[2-methoxy-5-(trifluormethyl)-phenyl]-3,4-dihydro-4-chinazolinyl}essigsäuremethylester

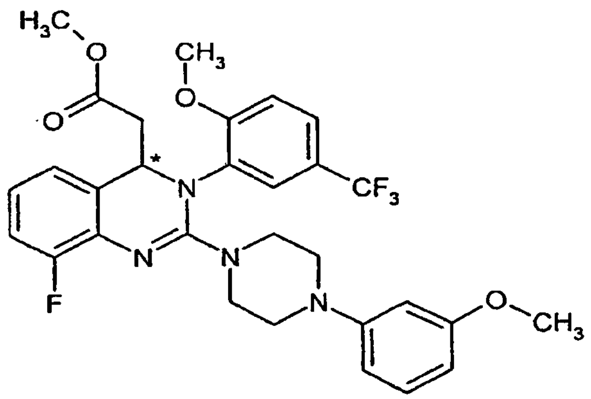

Die Verbindung erhält man als Enantiomer A, indem man das Racemat aus Beispiel 31A chromatographisch gemäß Methode 15 in die Enantiomeren auftrennt. Ausgehend von 231 g Racemat erhält man 111 g (48% d. Th.) des Zielproduktes.
MS (ESI-pos): m/z = 587 $(M+H)^+$

**Beispiel 44A**

(6-Cyano-2-[4-(4-fluorphenyl)-1-piperazinyl]-3-[3-(trifluormethyl)phenyl]-3,4-dihydro-4-chinazolinyl}essigsäuremethylester

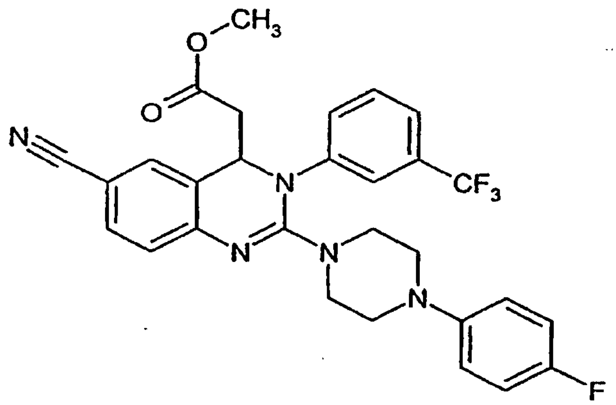

Ausgehend von 400 mg (0.6 mmol) Iminophosphoran aus Beispiel 12A werden nach der allgemeinen Arbeitsvorschrift [G] 166 mg (48% d. Th.) Produkt erhalten.
HPLC (Methode 1): $R_t$ = 4.65 min
MS (ESI-pos): m/z = 552 $(M+H)^+$

**Beispiel 45A**

{7-Cyano-2-[4-(4-fluorphenyl)-1-piperazinyl]-3-[3-(trifluormethyl)phenyl]-3,4-dihydro-4-chinazolinyl}essigsäuremethylester

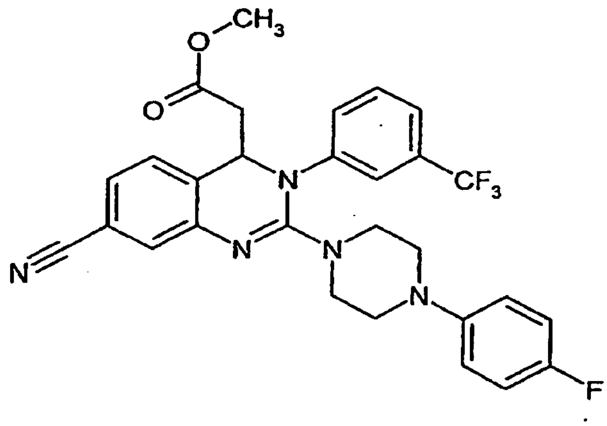

Ausgehend von 1.0 g (2.16 mmol) Iminophosphoran aus Beispiel 40A werden nach der allgemeinen Arbeitsvorschrift [G] 1.07 g (98% d. Th.) Produkt erhalten.
HPLC (Methode 1): $R_t$ = 4.72 min
MS (ESI-pos): m/z = 552 $(M+H)^+$

**Beispiel 46A**

4-(2-tert.-Butoxy-2-oxoethyl)-2-[4-(4-fluorphenyl)piperazin-1-yl]-3-[3-(trifluormethyl)phenyl]-3,4-dihydrochinazolin-6-carbonsäuremethylester

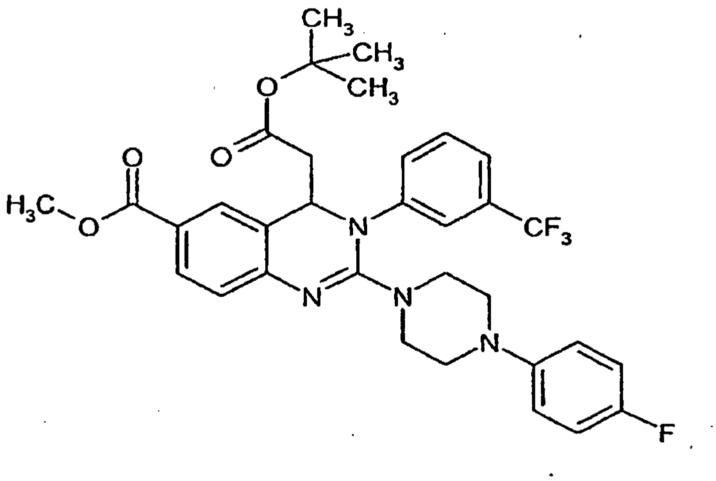

Ausgehend von 4.2 g (9.3 mmol) Iminophosphoran aus Beispiel 41A werden nach der allgemeinen Arbeitsvorschrift [G] 3.9 g (51% d. Th.) Produkt erhalten.
HPLC (Methode 1): $R_t$ = 5.03 min
MS (ESI-pos): m/z = 627 $(M+H)^+$

**Beispiel 47A**

{8-Fluor-2-[4-(4-fluorphenyl)-1-piperazinyl]-3-[3-(trifluormethyl)phenyl]-3,4-dihydro-4-chinazolinyl}essigsäuremethylester

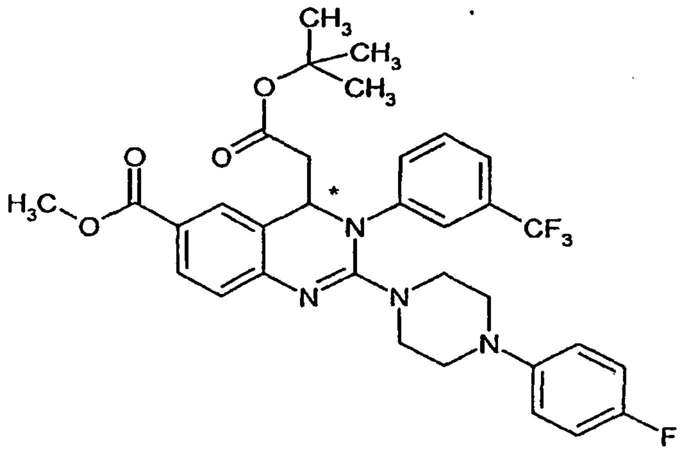

Diese Verbindung wird als Enantiomer A nach der Enantiomerentrennung von 3.5 g Beispiel 46A erhalten (1.4 mg, 20% d. Th.).
HPLC (Methode 1): $R_t$ = 4.9l min
MS (ESI-pos): m/z = 627 $(M+H)^+$

**Beispiel 48A**

4-(2-tert.-Butoxy-2-oxoethyl)-2-[4-(4-fluorphenyl)piperazin-1-yl]-3-[3-(trifluormethyl)phenyl]-3,4-dihydrochinazolin-6-carbonsäure

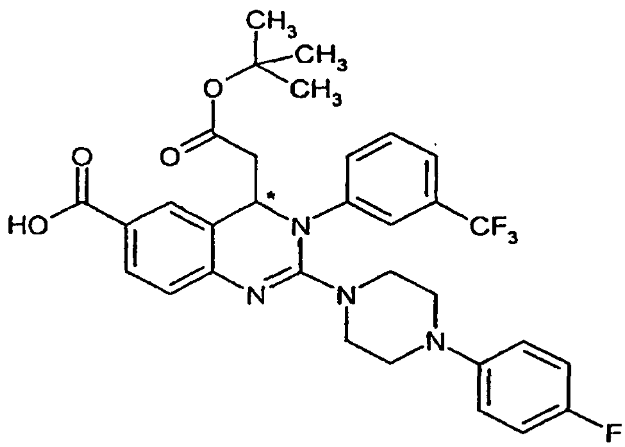

1.3 g (2.0 mmol) Carbonsäuremethylester aus Beispiel 47A werden in 12 ml Dioxan gelöst, mit 2.4 ml einer 1 N wässrigen Kaliumhydroxid-Lösung versetzt und 5 Stunden bei 60°C gerührt. Mit 1N wässriger Salzsäure-Lösung wird pH=4 eingestellt, das Reaktionsgemisch eingeengt und per präparativer HPLC gereinigt. Man erhält 580 mg (48% d. Th.) des Produktes.
HPLC (Methode 1): $R_t$ = 4.85 min
MS (ESI-pos): m/z = 613 $(M+H)^+$

**Beispiel 49A**

{6-(Aminocarbonyl)-2-[4-(4-fluorphenyl)piperazin-1-yl]-3-[3-(trifluormethyl)phenyl]-3,4-dihydrochinazolin-4-yl}essigsäure-tert.-butylester

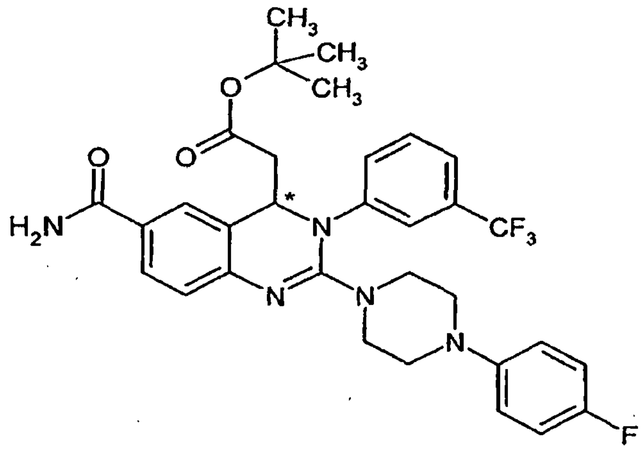

560 mg (0.9 mmol) Carbonsäure aus Beispiel 48A werden mit 2.6 mmol Aluminiumchlorid, 1.1 mmol 1-Hydroxy-1H-benzotriazol-Hydrat und 1.1 mmol N-(3-Dimethylaminopropyl)-N'-ethyl-carbodiimid in DMF suspendiert. Man setzt 2.6 mmol N,N-Diisopropylamin zu und rührt 16 Stunden bei Raumtemperatur. Das Reaktionsgemisch wird mit 20 ml Essigsäureethylester versetzt und mit einer gesättigten, wässrigen Natriumhydrogencarbonat-Lösung und einer gesättigten, wässrigen Natriumchlorid-Lösung gewaschen. Die vereinigten wässrigen Phasen werden auf pH=8 eingestellt und mit Essigsäureethylester extrahiert. Die vereinigten organischen Phasen werden abschließend mit einer gesättigten, wässrigen Natriumchlorid-Lösung gewaschen, über Natriumsulfat getrocknet und eingeengt. Man erhält 548 mg (97% d. Th.) Produkt.

HPLC (Methode 1): $R_t$ = 4.73 min

MS (ESI-pos): m/z = 612 $(M+H)^+$

Die Beispiele 50A bis 112A der Tabelle **1** können nach den allgemeinen Arbeitsvorschriften [A] bis [G] aus den entsprechenden Ausgangsverbindungen hergestellt werden.

**Tabelle 1**

| Beispiel Nr. | Struktur | $R_t$ [min] | HPLC Methode | MS ESIpos. [M+H]+ |
|---|---|---|---|---|
| 50A | 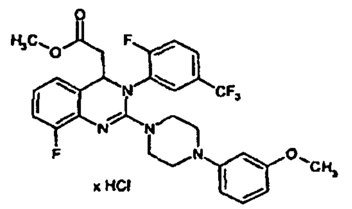 | 4.53 | 1 | 561 $[M+H-HCl]^+$ |
| 51A | 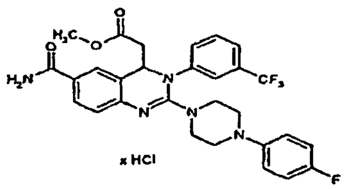 | 4.22 | 1 | 556 $[M+H-HCl]^+$ |

(fortgesetzt)

| Beispiel Nr. | Struktur | $R_t$ [min] | HPLC Methode | MS ESIpos. [M+H]+ |
|---|---|---|---|---|
| 52A | 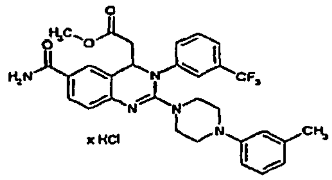 | 4.36 | 1 | 552 $[M+H-HCl]^+$ |
| 53A | 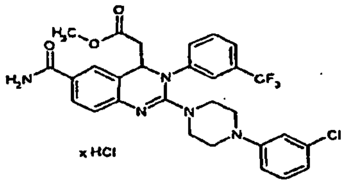 | 4.37 | 1 | 572 $[M+H-HCl]^+$ |
| 54A | 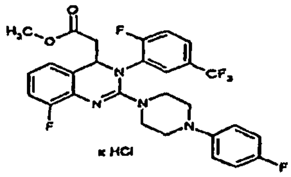 | 4.54 | 1 | 549 $[M+H-HCl]^+$ |
| 55A | 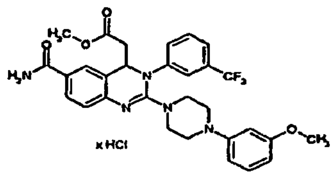 | 4.27 | 1 | 568 $[M+H-HCl]^*$ |
| 56A | 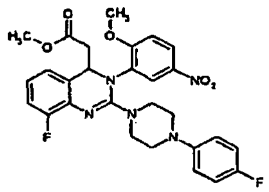 | 4.30 | 1 | 538 |
| 57A | 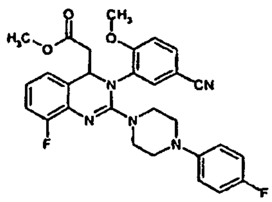 | 4.28 | 1 | 518 |
| 58A | 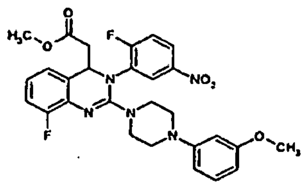 | 4.41 | 1 | 538 |
| 59A | 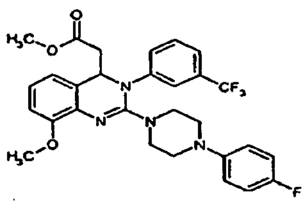 | 4.82 | 1 | 557 |

(fortgesetzt)

| Beispiel Nr. | Struktur | $R_t$ [min] | HPLC Methode | MS ESIpos. [M+H]+ |
|---|---|---|---|---|
| 60A | 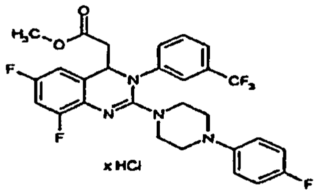 | 4.61 | 1 | 549 [M+H-HCl]* |
| 61A | 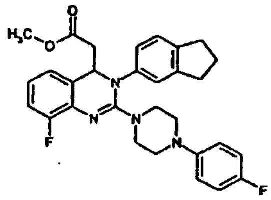 | 4.89 | 1 | 517 |
| 62A | 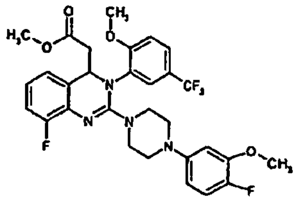 | 4.81 | 6 | 605 |
| 63A | 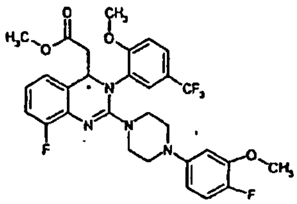 | 4.60 | 6 | 591 |
| 64A | 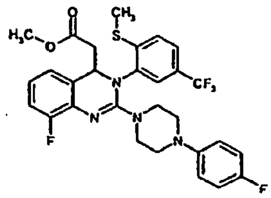 | 4.85 | 6 | 591 |
| 65A | 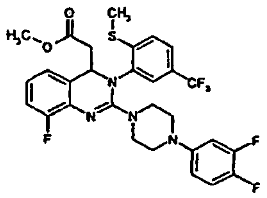 | 4.92 | 6 | 609 |
| 66A | 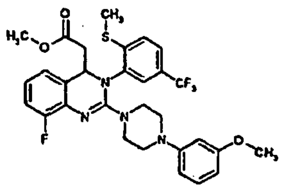 | 4.83 | 1 | 603 |

(fortgesetzt)

| Beispiel Nr. | Struktur | $R_t$ [min] | HPLC Methode | MS ESIpos. [M+H]+ |
|---|---|---|---|---|
| 67A | 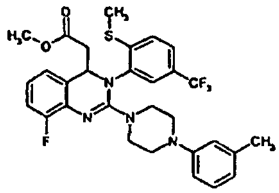 | 4.78 | 1 | 587 |
| 68A | 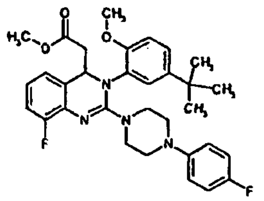 | 5.13 | 1 | 563 |
| 69A | 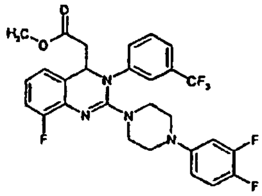 | 4.76 | 1 | 563 |
| 70A | 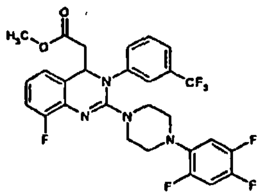 | 4.81 | 1 | 581 |
| 71A | 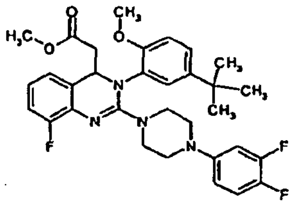 | 5.21 | 1 | 581 |
| 72A | 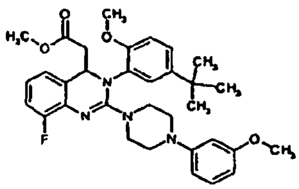 | 5.12 | 1 | 575 |
| 73A | 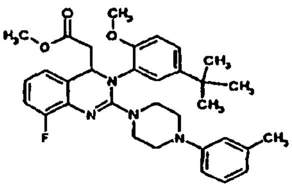 | 4.98 | 1 | 559 |

(fortgesetzt)

| Beispiel Nr. | Struktur | $R_t$ [min] | HPLC Methode | MS ESIpos. [M+H]+ |
|---|---|---|---|---|
| 74A | 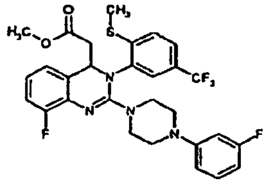 | 4.86 | 1 | 591 |
| 75A | 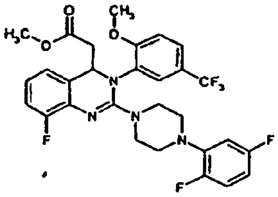 | 4.86 | 6 | 593 |
| 76A | 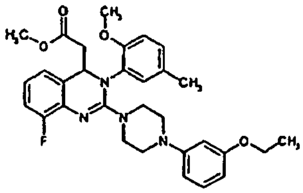 | 4.94 | 1 | 547 |
| 77A | 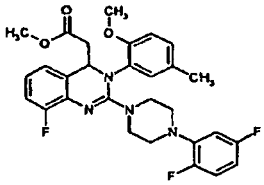 | 4.82 | 1 | 539 |
| 78A | 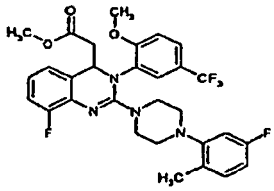 | 4.92 | 1 | 589 |
| 79A | 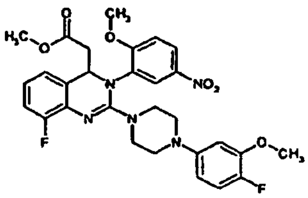 | 4.57 | 1 | 582 |
| 80A | 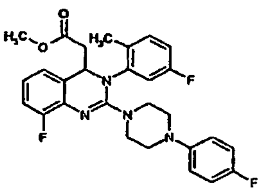 | 2.38 | 3 | 495 |

(fortgesetzt)

| Beispiel Nr. | Struktur | $R_t$ [min] | HPLC Methode | MS ESIpos. [M+H]+ |
|---|---|---|---|---|
| 81A | 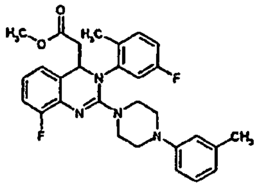 | 1.95 | 9 | 491 |
| 82A | 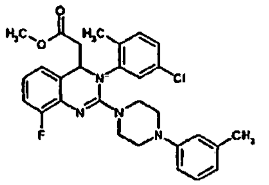 | 1.97 | 9 | 507 |
| 83A | 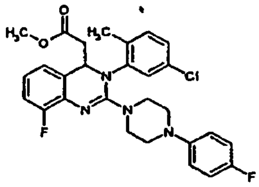 | 1.93 | 9 | 511 |
| 84A | 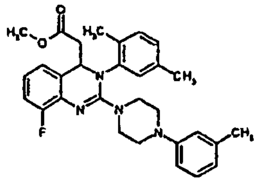 | 1.90 | 9 | 487 |
| 85A | 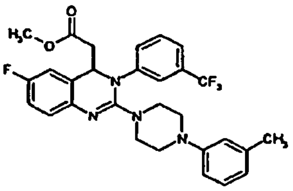 | 4.87 | 1 | 541 |
| 86A | 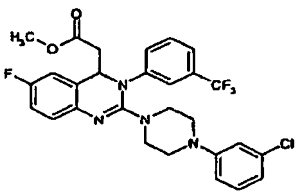 | 4.91 | 1 | 561 |
| 87A | 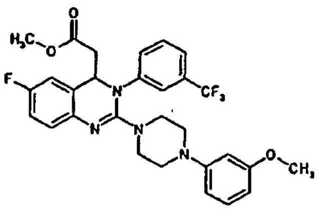 | 4.76 | 1 | 557 |

(fortgesetzt)

| Beispiel Nr. | Struktur | $R_t$ [min] | HPLC Methode | MS ESIpos. [M+H]+ |
|---|---|---|---|---|
| 88A | 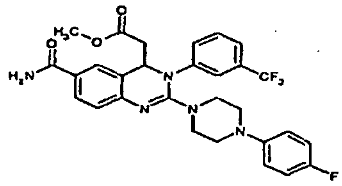 | 4.65 | 1 | 552 |
| 89A | 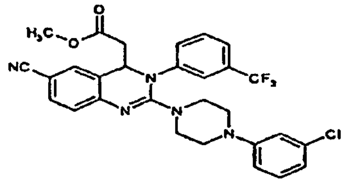 | 4.77 | 1 | 568 |
| 90A | 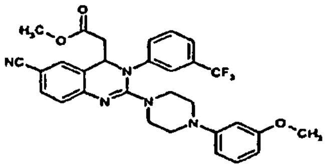 | 4.62 | 1 | 564 |
| 91A | 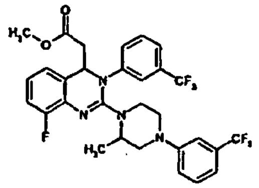 | 5.00 | 1 | 609 |
| 92A | 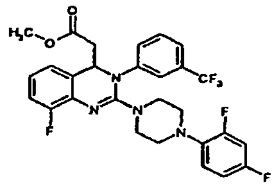 | 4.70 | 1 | 563 |
| 93A | 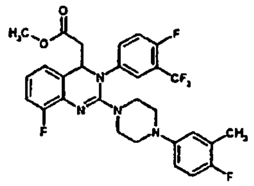 | | | 577 |
| 94A | 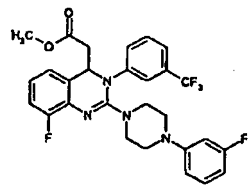 | 4.74 | 1 | 545 |
| 95A | 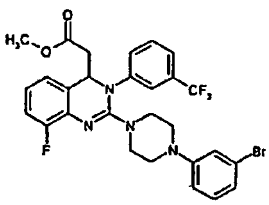 | 4.90 | 1 | 605 |

(fortgesetzt)

| Beispiel Nr. | Struktur | $R_t$ [min] | HPLC Methode | MS ESIpos. [M+H]+ |
|---|---|---|---|---|
| 96A | 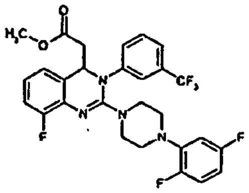 | 4.83 | 1 | 563 |
| 97A | 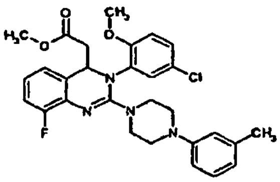 | 4.82 | 1 | 537 |
| 98A | 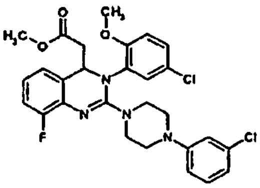 | 4.90 | 1 | 557 |
| 99A | 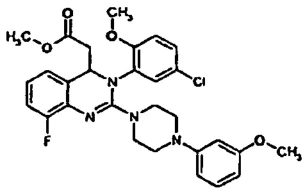 | 4.81 | 1 | 553 |
| 100A | 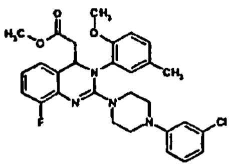 | | | |
| 101A | 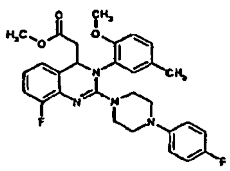 | 4.78 | 1 | 521 |
| 102A | 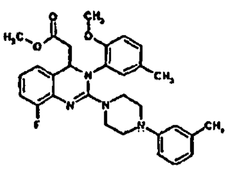 | 4.73 | 1 | 517 |
| 103A | 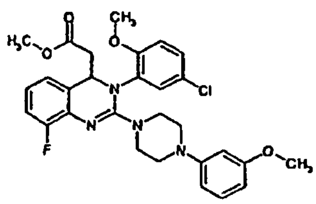 | 3.10 | 16 | 533 |

(fortgesetzt)

| Beispiel Nr. | Struktur | $R_t$ [min] | HPLC Methode | MS ESIpos. [M+H]+ |
|---|---|---|---|---|
| 104A | 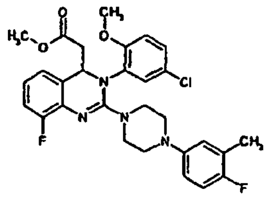 | 2.75 | 17 | 555 |
| 105A | 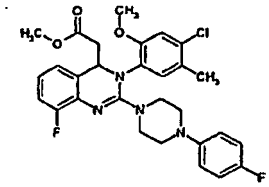 | 2.95 | 17 | 555 |
| 106A | 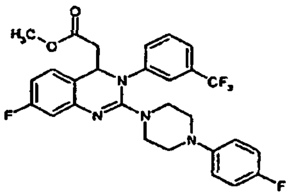 | 4.74 | 1 | 545 |
| 107A | 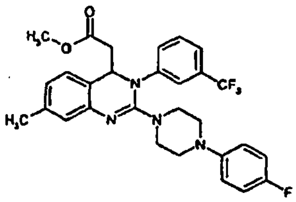 | 4.93 | 1 | 541 |
| 108A | 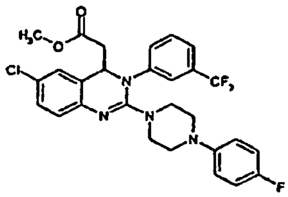 | 5.08 | 1 | 575 |
| 109A | 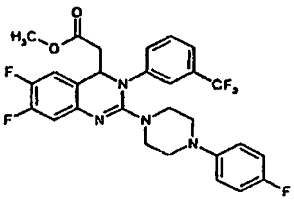 | 4.88 | 1 | 563 |
| 110A | 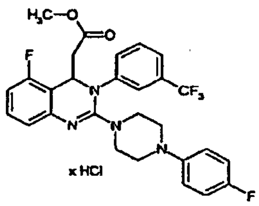 | 4.54 | 1 | 531 $[M+H-HCl]^+$ |

(fortgesetzt)

| Beispiel Nr. | Struktur | $R_t$ [min] | HPLC Methode | MS ESIpos. [M+H]+ |
|---|---|---|---|---|
| 111A | 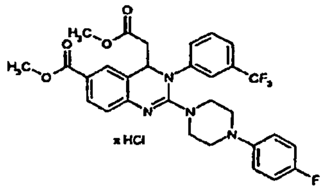 | 4.54 | 1 | 571 [M+H-HCl]+ |

## Ausführungsbeispiele

### Allgemeine Arbeitsvorschrift [H]: Esterverseifung der Chinazolylessigsäurester

Es werden 1.0 Äquivalente des Chinazolylessigsäuresters in Dioxan gelöst und 5.0 Äquivalente 1N Natronlauge hinzugefügt. Man lässt für 16 Stunden bei 80°C rühren und nach beendeter Reaktion (Reaktionskontrolle mittels analytischer HPLC) wird der Ansatz eingeengt. Der Rückstand wird dann in Wasser aufgenommen und mit 1N Salzsäure auf pH 5 gestellt. Man filtriert den entstehenden Niederschlag ab, wäscht ihn mit wenig Wasser und Diethylether und trocknet ihn im Hochvakuum bei Raumtemperatur. Alternativ kann der Niederschlag über eine Extrelutkartusche filtriert, mit Essigsäureethylester nachgewaschen und das Filtrat eingeengt werden. Falls die Reinheit des Produktes nicht hoch genug ist, wird es entweder über präparative HPLC an RP-Phase (Methode 2 oder Methode 5) oder an Kieselgel mit Cyclohexan/Essigsäureethylester-Gemischen gereinigt.

### Beispiel 1

{8-Fluor-2-[4-(4-fluorphenyl)-1-piperazinyl]-3-[3-(trifluormethyl)phenyl]-3,4-dihydro-4-chinazolinyl}essigsäure

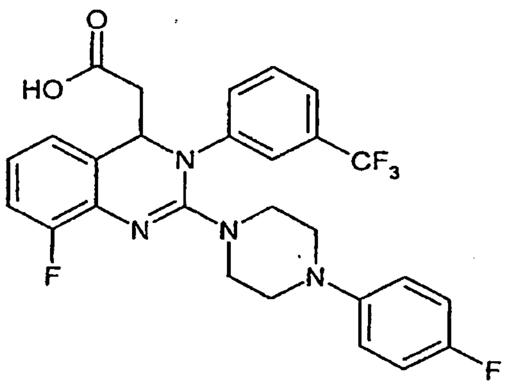

Ausgehend von 37 mg (0.07 mmol) Methylester aus Beispiel 19A werden nach der allgemeinen Arbeitsvorschrift [H] 29 mg (80% d. Th.) Produkt erhalten.
HPLC (Methode 1): $R_t$= 4.49 min
MS (ESI-pos): m/z = 530.7 $(M+H)^+$
$^1$H-NMR (400MHz, $CD_3CN$): δ [ppm] = 7.59 (s, 1H); 7.45 (t, 1H); 7.37 (t, 2H); 7.02-6.95 (m, 3H); 6.93-6.85 (m, 4H); 5.24 (dd, 1H); 2.98 ($d_b$, 4H); 2.91 ($d_b$, 4H); 2.73 (dd, 1H); 2.54 (dd, 1H).

### Beispiel 2

{8-Fluor-2-[4-(4-fluorphenyl)-1-piperazinyl]-3-[3-(trifluormethyl)phenyl]-3,4-dihydro-4-chinazolinyl}essigsäure

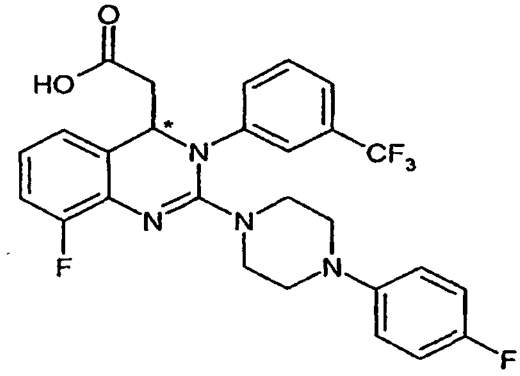

Ausgehend von 695 mg (1.27 mmol) Methylester aus Beispiel 20A werden nach der allgemeinen Arbeitsvorschrift [H] 488 mg (64% d. Th.) Produkt erhalten.
HPLC (Methode 1): $R_t$=4.59 min
MS (ESI-pos): m/z = 530.8 $(M+H)^+$
$^1$H-NMR (400MHz, $CD_3CN$): δ [ppm] = 7.60 (s, 1H); 7.47-7.40 (m, 3H); 7.03-6.86 (m, 7H); 5.26-5.23 (m, 1H); 3.60-3.52 (m, 4H); 2.99-2.90 (m, 4H); 2.75 (dd, 1H); 2.56 (dd, 1H).

## Beispiel 3

{8-Fluor-2-[4-(3-methylphenyl)-1-piperazinyl]-3-[3-(trifluormethyl)phenyl]-3,4-dihydro-4-chinazolinyl}essigsäure

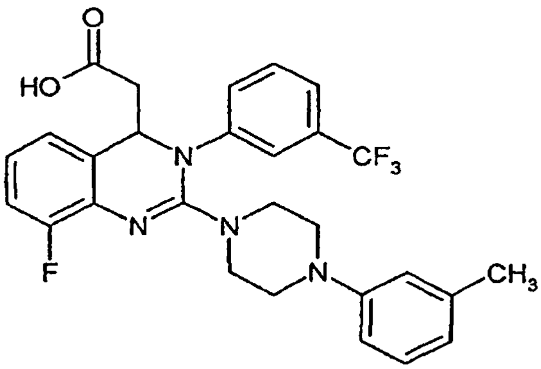

Ausgehend von 34 mg (0.06 mmol) Methylester aus Beispiel 23A werden nach der allgemeinen Arbeitsvorschrift [H] 30 mg (90% d. Th.) Produkt erhalten.
HPLC (Methode 1): $R_t$=4.56 min
MS (ESI-pos): m/z = 526.9 $(M+H)^+$
$^1$H-NMR (200MHz, DMSO-$d_6$): δ [ppm] = 7.64 (s, 1H); 7.53 (t, 1H); 7.44-7.34 (m, 2H); 7.11-6.90 (m, 3H); 6.72-6.59 (m, 4H); 5.33-5.25 (m, 1H); 3.52 ($d_b$, 4H); 3.02 ($d_b$, 4H); 2.69-2.55 (m, 2H, teilweise unter DMSO-Signal); 2.23 (s, 3H).

## Beispiel 4

{8-Fluor-2-[4-(3-methoxyphenyl)-1-piperazinyl]-3-[3-(trifluormethyl)phenyl]-3,4-dihydro-4-chinazolinyl} essigsäure

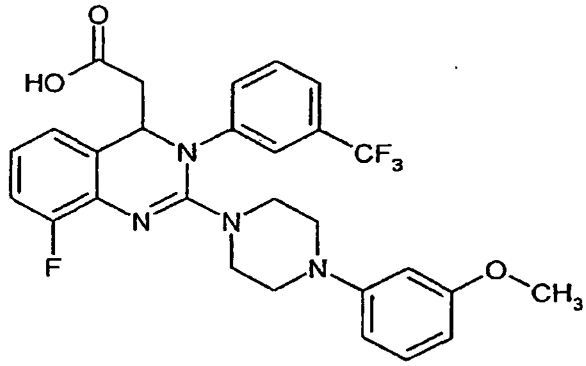

Ausgehend von 36 mg (0.07 mmol) Methylester aus Beispiel 25A werden nach der allgemeinen Arbeitsvorschrift [H] und nach Chromatographie (Methode 2) 28 mg (77% d. Th.) Produkt erhalten.
HPLC (Methode 1): $R_t$=4.46 min
MS (ESI-pos): m/z = 542.9 $(M+H)^+$
$^1$H-NMR (200MHz, DMSO-$d_6$): $\delta$ [ppm] = 7.67 (s, 1H); 7.54 (t, 1H); 7.45-7.38 (m, 2H); 7.14-6.94 (m, 3H); 6.51-6.35 (m, 4H); 5.35-5.25 (m, 1H); 3.69 (s, 3H); 3.50 ($d_b$, 4H); 3.06 ($d_b$, 4H); 2.58-2.52 (m, 2H).

**Beispiel** 5

{8-Fluor-2-[4-(3-chlorphenyl)-1-piperazinyl]-3-[3-(trifluormethyl)phenyl]-3,4-dihydro-4-chinazolinyl}essigsäure

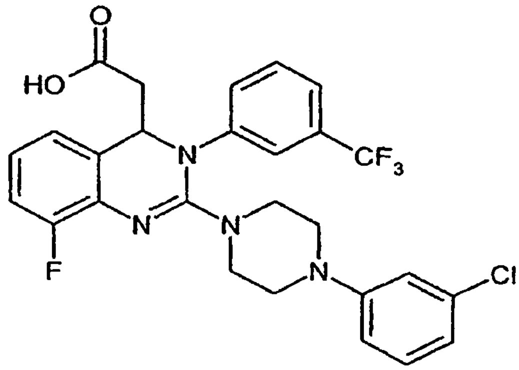

Ausgehend von 38 mg (0.07 mmol) Methylester aus Beispiel 35A werden nach der allgemeinen Arbeitsvorschrift [H] 25 mg (66% d. Th.) Produkt erhalten.
HPLC (Methode 1): $R_t$=4.64 min
MS (ESI-pos): m/z = 546.9 $(M+H)^+$
$^1$H-NMR (200MHz, DMSO-$d_6$): $\delta$ [ppm] = 7.66 (s, 1H); 7.52 (t, 1H); 7.38 (dd, 2H); 7.20 (t, 1H); 7.10-6.78 (m, 6H); 5.33-5.26 (m, 1H); 3.51 ($d_b$, 4H); 3.11 ($d_b$, 4H); 2.61-2.55 (m, 2H).

**Beispiel 6**

{8-Fluor-2-[4-(1,3-benzodioxol-5-yl)-1-piperazinyl]-3-[3-(trifluormethyl)phenyl]-3,4-dihydro-4-chinazolinyl}essigsäure

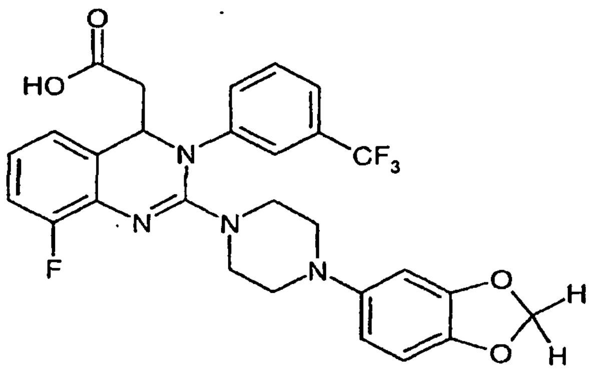

Ausgehend von 173 mg (0.30 mmol) Methylester aus Beispiel 36A werden nach der allgemeinen Arbeitsvorschrift [H] 79 mg (46% d. Th.) Produkt erhalten.
HPLC (Methode 1): $R_t$=4.44 min
MS (ESI-pos): m/z = 557.2 $(M+H)^+$
$^1H$-NMR (300MHz, $CDCl_3$): $\delta$ [ppm] = 7.47 (s, 1H); 7.42-7.34 (m, 3H); 7.03-6.89 (m, 2H); 6.79 (d, 1H); 6.64 (d, 1H); 6.41 (d, 1H); 6.22 (dd, 1H); 5.87 (s, 2H); 5.20-5.15 (m, 1H); 3.59 ($s_b$, 3H); 2.94-2.85 (m, 5H); 2.59 (dd, 1H).

## Beispiel 7

{6-Fluor-2-[4-(4-fluorphenyl)-1-piperazinyl]-3-[3-(trifluormethyl)phenyl]-3,4-dihydro-4-chinazolinyl}essigsäure

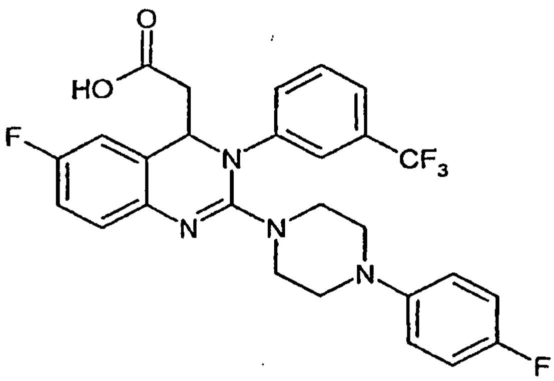

Ausgehend von 42 mg (0.08 mmol) Methylester aus Beispiel 21Awerden nach der allgemeinen Arbeitsvorschrift [H] 34 mg (76% d. Th.) Produkt erhalten.
HPLC (Methode 1): $R_t$=4.63 min
MS (ESI-pos): m/z = 530.9 $(M+H)^+$

## Beispiel 8

{6-Fluor-2-[4-(4-fluorphenyl)-1-piperazinyl]-3-[3-(trifluormethyl)phenyl]-3,4-dihydro-4-chinazolinyl)essigsäure Hydrochlorid

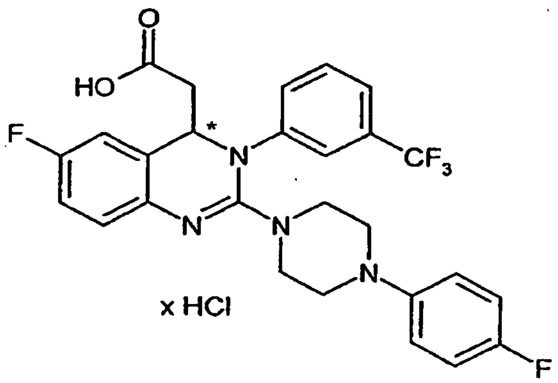

Ausgehend von 350 mg (0.64 mmol) Ester aus Beispiel 22A werden nach der allgemeinen Arbeitsvorschrift [H] 284 mg (83% d. Th.) Produkt erhalten.
HPLC (Methode 1): $R_t$=4.53 min
MS (ESI-pos): m/z=530.8 $(M+H-HCl)^+$
$^1$H-NMR (400MHz, $CD_3CN$): δ [ppm] = 7.62 (s, 1H); 7.51-7.48 (m, 1H); 7.43-7.41 (d, 1H); 7.26-7.23 (m, 1H); 7.04-6.95 (m, 2H); 6.91-6.85 (m, 3H); 5.23 (dd, 1H); 3.55 ($s_b$, 3H); 3.02-2.99 (m, 1H); 2.94 ($s_b$, 4H); 2.80 (dd, 1H).

**Beispiel 9**

{8-Fluor-2-[4-(3-methylphenyl)-1-piperazinyl]-3-[3-(trifluormethyl)phenyl]-3,4-dihydro-4-chinazolinyl}essigsäure Hydrochlorid

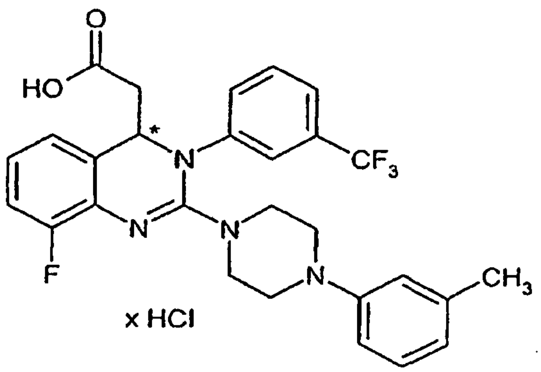

Ausgehend von 1.10g (1.93 mmol) Ester aus Beispiel 24A werden nach der allgemeinen Arbeitsvorschrift [H] 1.04 g (91% d. Th.) Produkt erhalten. Nach der Enantiomerentrennung nach Methode 4 wird das Produkt als Enantiomer A erhalten.
HPLC (Methode 1): $R_t$=4.68 min
MS (ESI-pos): m/z = 526.9 $(M+H-HCl)^+$
$^1$H-NMR (400MHz, $CD_3CN$): δ [ppm] = 7.61 (s, 1H); 7.49-7.38 (m, 3H); 7.10-6.89 (m, 4H); 6.71-6.65 (m, 3H); 5.26 (dd, 1H); 3.60-3.52 (m, 4H); 3.03-2.95 (m, 4H); 2.76 (dd, 1H); 2.57 (dd, 1H); 2.25 (s, 3H).

**Beispiel 10**

{8-Fluor-2-[4-(3-methoxyphenyl)-1-piperazinyl]-3-[3-(triflluormethyl)phenyl]-3,4-dihydro-4-chinazolinyl}essigsäure Hydrochlorid

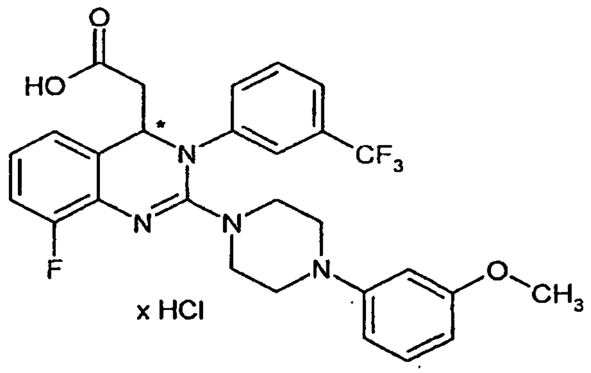

Ausgehend von 437 mg (0.79 mmol) Ester aus Beispiel 26A werden nach der allgemeinen Arbeitsvorschrift [H] 344 mg (72% d. Th.) Produkt erhalten.
HPLC (Methode 1): $R_t$=4.48 min
MS (ESI-pos): m/z = 543.0 $(M+H-HCl)^+$
$^1$H-NMR (400MHz, $CD_3CN$): δ [ppm] = 7.61 (s, 1H); 7.49-7.38 (m, 3H); 7.14-6.89 (m, 4H); 6.47-6.39 (m, 3H); 5.26 (dd, 1H); 3.72 (s, 1H); 3.60-3.54 (m, 4H); 3.07-3.00 (m, 4H); 2.77 (dd, 1H); 2.57 (dd, 1 H).

**Beispiel 11**

{8-Fluor-2-[4-(4-fluor-3-methylphenyl)-1-piperazinyl]-3-[6-methoxy-3-(trifluormethyl)phenyl]-3,4-dihydro-4-chinazolinyl} essigsäure Hydrochlorid

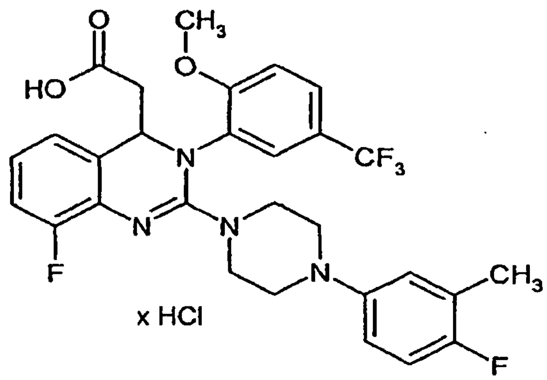

Ausgehend von 1.03 g (1.75 mmol) Rohprodukt des Esters aus Beispiel 27A werden nach der allgemeinen Arbeitsvorschrift [H] und nach Chromatographie nach Methode 5 sowie anschließendem Aufnehmen des Produktes in Methanol/1N Salzsäure und erneutem Abdampfen des Lösungsmittels 283 mg (22% d. Th.) Hydrochlorid erhalten.
HPLC (Methode 1): $R_t$=4.58 min
MS (ESI-pos): m/z = 575.2 $(M+H-HCl)^+$
$^1$H-NMR (400MHz, $CD_3CN$): δ [ppm] = 8.17 (s, 0.66H); 7.69 (d, 1H); 7.55-7.30 (m, 1H); 7.27-7.24 (m, 2H); 7.16 (d, 0.6H); 7.09-7.04 (m, 2H); 5.33-5.27, 5.12-5.06 (2x m, 1H); 4.08-3.35 (m, 4H); 3.69 (s, 3H); 3.30-3.22 (m, 1H); 2.80-2.76 (m, 1H); 2.25 (s, 3H).

**Beispiel 12**

{8-Fluor-2-[4-(4-fluor-3-methylphenyl)-1-piperazinyl]-3-[6-methoxy-3-(trifluormethyl)phenyl]-3,4-dihydro-4-chinazolinyl} essigsäure Hydrochlorid

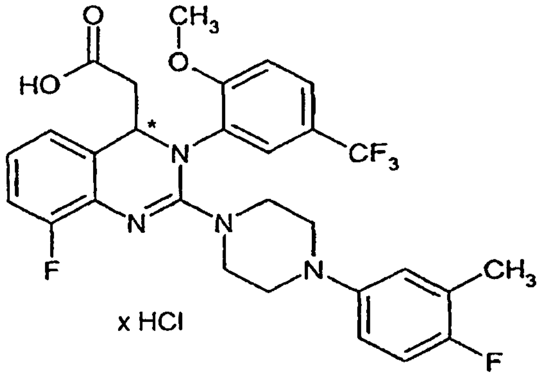

Vor der Enantiomerentrennung werden 268 mg Hydrochlorid aus Beispiel 11 in Dichlormethan aufgenommen und die organische Phase zweimal mit gesättigter Natriumhydrogencarbonat-Lösung extrahiert. Die vereinigten wässrigen Phasen werden einmal mit Dichlormethan extrahiert, die vereinigten organischen Phasen über Natriumsulfat getrocknet, filtriert und im Vakuum das Lösungsmittel entfernt. Man erhält 204 mg (86% d. Th.) der freien Base. Davon ausgehend erhält man nach Enantiomerentrennung (Methode 4) und erneuter Reinigung mittels präparativer HPLC (Methode 5) sowie anschließendem Aufnehmen des Produktes in Methanol/1N Salzsäure und erneutem Abdampfen des Lösungsmittels 80 mg (78% d. Th.) des Enantiomers A.
HPLC (Methode 6): $R_t$=4.66 min
MS (ESI-pos): m/z = 575.2 $(M+H-HCl)^+$
$^1$H-NMR (400MHz, $CD_3CN$): δ [ppm] = 8.17 (s, 0.66H); 7.69 (d, 1H); 7.45-7.30 (m, 1H); 7.24 (d, 2H); 7.15 (d, 0.7H); 7.08-7.01 (m, 2H); 5.32-5.27, 5.11-5.07 (2x m, 1H); 4.06-3.50 (m, 4H); 3.68 (s, 3H); 3.33-3.24 (m, 1H); 2.77-2.72 (m, 1H); 2.24, 2.23 (2x s, 3H).

## Beispiel 13

{8-Fluor-2-[4-(4-fluor-3-rnethylphenyl)-1-piperazinyl]-3-[6-methoxy-3-methyl-phenyl]-3,4-dihydro-4-chinazolinyl}essigsäure Hydrochlorid

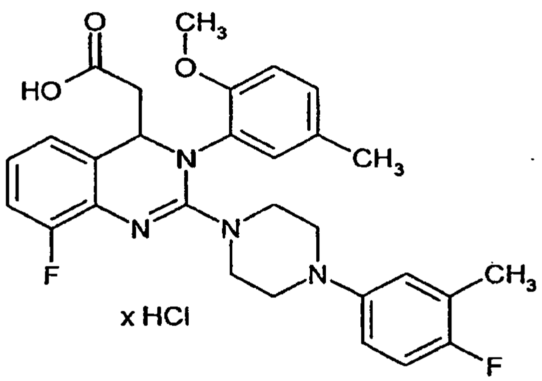

Ausgehend von 183 mg (0.34 mmol) Rohprodukt des Esters aus Beispiel 28A werden nach der allgemeinen Arbeitsvorschrift [H] und nach Chromatographie nach Methode 5 sowie anschließendem Aufnehmen des Produktes in Methanol/1N Salzsäure und erneutem Abdampfen des Lösungsmittels 135 mg (67% d. Th.) Hydrochlorid erhalten.
HPLC (Methode 1): $R_t$=4.67 min
MS (ESI-pos): m/z = 521.2 $(M+H-HCl)^+$
$^1$H-NMR (400MHz, $CD_3CN$): δ [ppm] = 7.69-7.42 (m, 4H); 7.25-7.06 (m, 5H); 6.93-6.78 (m, 1H); 5.24-5.21, 5.06-5.03 (2x m, 1H); 4.00-3.35 (m, 8H); 3.21-3.08 (m, 1H); 3.01-2.77 (m, 1H); 2.34, 2.20 (2xs, 3H); 2.26 (s, 3H).

**Beispiel 14**

{8-Fluor-2-[4-(3-methoxyphenyl)-1-piperazinyl]-3-[2-methoxy-5-(trifluormethyl)-phenyl]-3,4-dihydro-4-chinazolinyl}essigsäure

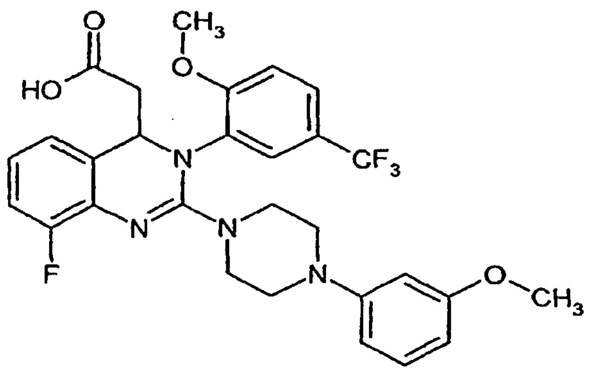

In 40 ml Dioxan werden 878 mg (1.5 mmol) {8-Fluor-2-[4-(3-methoxyphenyl)-1-piperazinyl]-3-[2-methoxy-5-(trifluormethyl)phenyl]-3,4-dihydro-4-chinazolinyl}essigsäuremethylester (Beispiel 31A) bei Raumtemperatur mit 179.6 mg (4.49 mmol) Natriumhydroxid versetzt und 2 Stunden bei 50°C gerührt. Anschließend wird auf pH 4-5 gebracht. Das Produkt wird abfiltriert, mit Wasser gewaschen und im Vakuum getrocknet.
Ausbeute: 801 mg (93% d. Th.)
HPLC (Methode 1): $R_t$=4.5 min
MS (ESI-pos): m/z = 573 $(M+H)^+$

**Beispiel 15**

{8-Fluor-2-[4-(3-methoxyphenyl)-1-piperazinyl]-3-[2-methoxy-5-(trifluormethyl)-phenyl]-3,4-dihydro-4-chinazolinyl}essigsäure

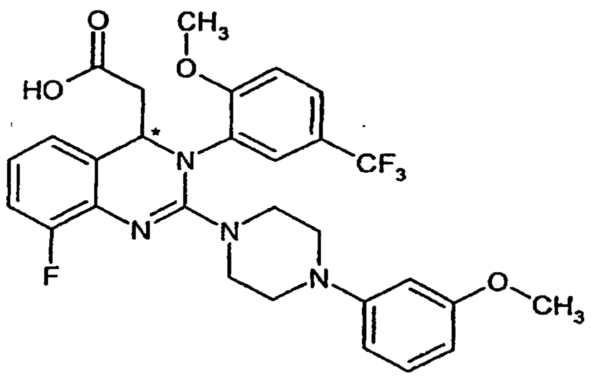

Nach Enantiomerentrennung (Methode 11) von 500 mg Racemat (Beispiel 14) wird das Rohprodukt durch Chromatographie an Kieselgel gereinigt und anschließend in 1 N Natronlauge gelöst und mit Diethylether extrahiert. Nach Ansäuren mit 1 N Salzsäure wird das Produkt abfiltriert und im Vakuum getrocknet.
Ausbeute: 105 mg (21% d. Th.)
MS (ESI-pos): m/z=573 $(M+H)^+$
$^1$H-NMR (300 MHz, DMSO-$d_6$): $\delta$ [ppm] = 2.4-2.5 (m, 1H); 2.7-3.1 (m, 5H); 3.3-3.6 (m, 4H); 3.7 (s, 3H); 3.7-3.9 ($s_b$ 3H); 4.8-5.05 ($s_b$ 1H); 6.3-6.4 (m, 2H); 6.4-6.5 (m, 1H); 6.8-7.65 (m, 6H); 12.5 ($S_b$, 1H).

Alternativ erhält man das Zielprodukt, indem man den enantiomerenreinen Ester aus Beispiel 43A gemäß der allgemeinen Arbeitsvorschrift [H] umsetzt. Ausgehend von 111 g (0.19 mol) Ester erhält man 69 g (63% d. Th.) Zielprodukt.

**Beispiel 16**

{8-Fluor-2-[4-(3,4-difluorphenyl)-1-piperazinyl]-3-[2-methoxy-5-(trifluormethyl)-phenyl]-3,4-dihydro-4-chinazolinyl}essigsäure

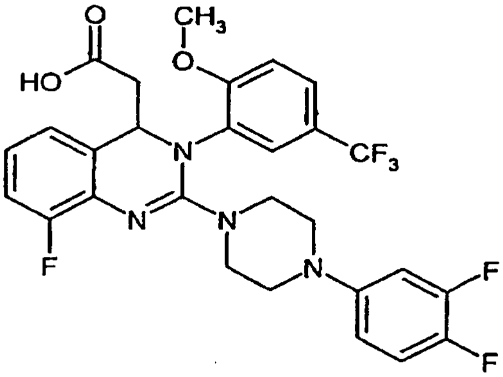

In 40 ml Dioxan werden 881 mg (1.49 mmol) {8-Fluor-2-[4-(3,4-difluorphenyl)-1-piperazinyl]-3-[2-methoxy-5-(trifluormethyl)phenyl]-3,4-dihydro-4-chinazolinyl}essigsäuremethylester (Beispiel 32A) mit 178 mg (4.46 mmol) Natriumhydroxid zwei Stunden bei 50°C gerührt. Nach Ansäuern mit 1 N Salzsäure wird das Produkt abgesaugt, mit Wasser gewaschen und im Vakuum getrocknet.
Ausbeute: 775 mg (90% d. Th.)
HPLC (Methode 1): $R_t$=4.5 min
MS (ESI-pos): m/z = 579 $(M+H)^+$

**Beispiel** 17

{8-Fluor-2-[4-(3,4-difluorphenyl)-1-piperazinyl]-3-[2-methoxy-5-(trifluormethyl)-phenyl]-3,4-dihydro-4-chinazolinyl}essigsäure

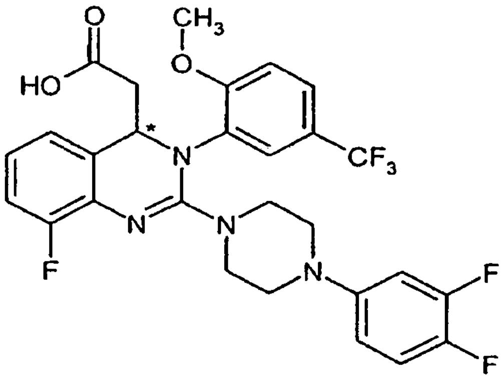

Nach Enantiomerentrennung (Methode 12) von 500 mg (0.86 mmol) Racemat (Beispiel 16) wird das Rohprodukt durch Chromatographie an Kieselgel (Dichlormethan, Dichlormethan/Methano120:1, 10:1) gereinigt, in 1N Natronlauge gelöst und mit Diethylether extrahiert. Die wässrige Phase wird mit 1 N Salzsäure auf pH 4-5 gebracht, das Produkt abfiltriert, mit Wasser gewaschen und im Vakuum getrocknet.
Ausbeute: 86 mg (17% d. Th.)
MS (ESI-pos): m/z = 579 $(M+H)^+$
$^1$H-NMR (300 MHz, DMSO-$d_6$): $\delta$ [ppm] = 2.6-3.1 (m, 6H); 3.25-3.6 (m, 4H); 3.75 ($s_b$, 3H); 4.85 ($S_b$, 1H); 6.6-6.7 (m, 1H); 6.7-7.7 (m, 9H); 12.5 ($s_b$, 1H).

**Beispiel 18**

{8-Fluor-2-[4-(4-fluorphenyl)-1-piperazinyl]-3-[2-methoxy-5-(trifluormethyl)-phenyl]-3,4-dihydro-4-chinazolinyl}essigsäure

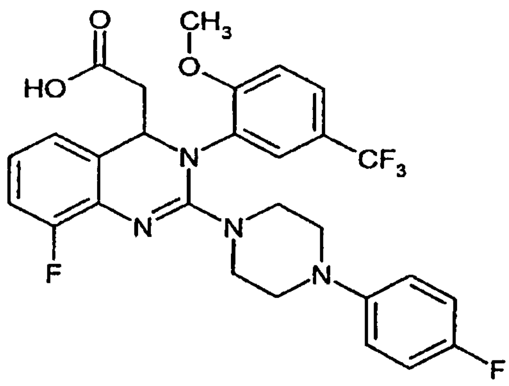

In 800 ml Dioxan werden 15g (26.11 mmol) {8-Fluor-2-[4-(4-fluorphenyl)-1-piperazinyl]-3-[2-methoxy-5-(trifluormethyl)phenyl]-3,4-dihydro-4-chinazolinyl}essigsäuremethylester (Beispiel 30A) mit 3.13 g (78.32 mmol) Natriumhydroxid 4 Stunden bei 50°C gerührt. Nach Abdestillieren des Lösungsmittels wird der Rückstand in 500 ml Wasser gelöst, angesäuert und der Niederschlag abgesaugt. Das Produkt wird mit Wasser gewaschen und im Vakuum getrocknet.
Ausbeute: 14.5 g (99% d. Th.)
HPLC (Methode 1): $R_t$=4.5 min
MS (ESI-pos): m/z= 561 $(M+H)^+$

**Beispiel 19**

{8-Fluor-2-[4-(4-fluorphenyl)-1-piperazinyl]-3-[2-methoxy-5-(trifluormethyl)-phenyl]-3,4-dihydro-4-chinazolinyl}essigsäure

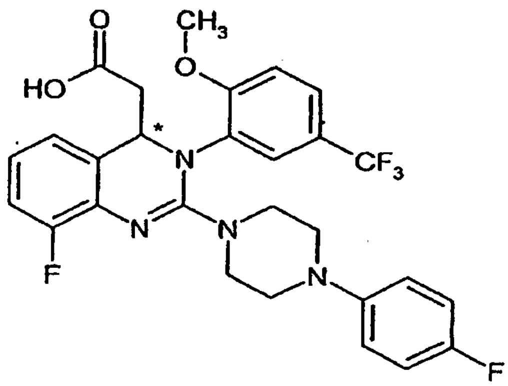

Es wird 14.2 g (25.33 mmol) Racemat (Beispiel 18) getrennt (Methode 13). Das Rohprodukt wird in 250 ml 0.5 N Natriumhydroxid-Lösung gelöst und anschließend durch Extraktion mit Diethylether gereinigt. Nach Ansäuern der wässrigen Phase mit Salzsäure wird das Produkt abfiltriert, mit Wasser gewaschen und im Vakuum getrocknet.
Ausbeute: 5.85 g (41% d. Th.)
MS (ESI-pos): m/z = 561 $(M+H)^+$
HPLC (Methode 1): $R_t$=4.5 min
$^1$H-NMR (400MHz, DMSO-$d_6$): $\delta$ [ppm] = 2.6-3.0 (m, 6H); 3.3-3.6 (m, 4H); 3.6-4.0 ($s_b$, 3H); 4.8-5.2 ($s_b$, 1H); 6.7-7.75 (m, 10H); 12.2-12.8 ($s_b$, 1H).

Alternativ erhält man das Zielprodukt, indem man den enantiomerenreinen Ester aus Beispiel 42A gemäß der allgemeinen Arbeitsvorschrift [H] umsetzt. Ausgehend von 120g (0.21 mol) Ester erhält man 96 g (81 % d. Th.) Zielprodukt.

## Beispiel 20

{8-Fluor-2-[4-(3-methylphenyl)-1-piperazinyl]-3-[2-methoxy-5-(trifluormethyl)-phenyl]-3,4-dihydro-4-chinazolinyl}essigsäure

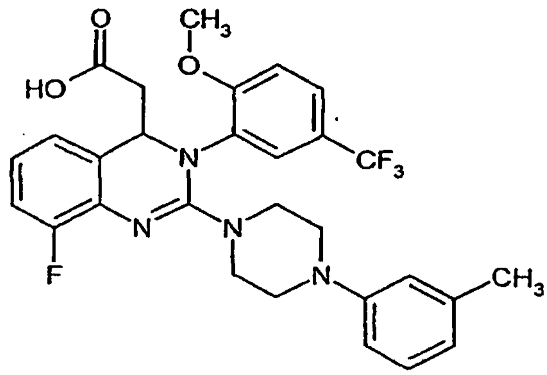

In 40 ml Dioxan werden 892 mg (1.56 mmol) {8-Fluor-2-[4-(3-methylphenyl)-1-piperazinyl]-3-[2-methoxy-5-(trifluormethyl)phenyl]-3,4-dihydro-4-chinazolinyl}essigsäuremethylester (Beispiel 33A) mit 187.6 mg (4.69 mmol) Natriumhydroxid 2 Stunden bei 50°C gerührt. Nach Entfernen des Lösungsmittels wird der Rückstand in Wasser aufgenommen und mit 1 N Salzsäure auf pH 4-5 eingestellt. Nach Abfiltrieren wird das Produkt mit Wasser gewaschen und im Vakuum getrocknet.
Ausbeute: 788 mg (91% d. Th.)
MS (ESI-pos): m/z = 557 $(M+H)^+$
HPLC (Methode 6): $R_t$=4.5 min

## Beispiel 21

{8-Fluor-2-[4-(3-methylphenyl)-1-piperazinyl]-3-[2-methoxy-5-(trifluormethyl)-phenyl]-3,4-dihydro-4-chinazolinyl}essigsäure

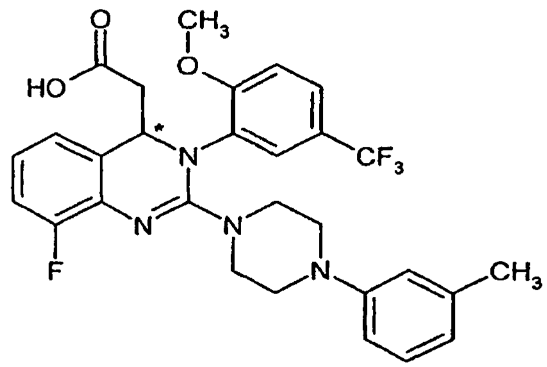

Die Enantiomerentrennung (Methode 13) erfolgt von 500 mg (0.9 mmol) Racemat (Beispiel 20). Anschließend wird das Rohprodukt in 1 N Natronlauge gelöst, mit Diethylether extrahiert und die wässrige Phase mit 1 N Salzsäure auf pH 4-5 gebracht. Das Produkt wird abgesaugt, mit Wasser gewaschen und im Vakuum getrocknet.
Ausbeute: 104 mg (21% d. Th.)
MS (ESI-pos): m/z = 557 $(M+H)^+$
$^1$H-NMR (400 MHz, DMSO-$d_6$): $\delta$ [ppm] = 2.2 ($s_b$, 3 H); 2.35-2.5 (m, 1H); 2.6-3.1 (m, 5 H); 3.3-3.6 (m, 4 H); 3.8 ($s_b$, 3

H); 4.9 ($s_b$, 1H); 6.5-6.7 (m, 3 H); 6.8-7.7 (m, 7H); 12.6 ($s_b$, 1H).

**Beispiel 22**

{8-Fluor-2-[4-(4-fiuorphenyl)-1-piperazinyl]-3-[6-methoxy-3-chlorphenyl]-3,4-dihydro-4-chinazolinyl}essigsäure Hydrochlorid

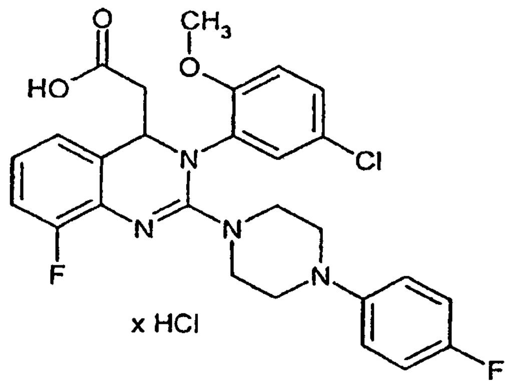

Ausgehend von 621 mg (1.15 mmol) Ester aus Beispiel 29A werden nach der allgemeinen Arbeitsvorschrift [H] und nach Reinigung mittels präparativer HPLC (Methode 5) und Koevaporieren mit Methanol/1N Salzsäure 330 mg (51% d. Th.) Produkt erhalten.
HPLC (Methode 1): $R_t$=4.58 min
MS (ESI-pos): m/z= 527.0 $(M+H-HCl)^+$

**Beispiel 23**

{8-Fluor-2-[4-(4-fluorphenyl)-1-piperazinyl]-3-[6-methoxy-3-chlorphenyl]-3,4-dihydro-4-chinazolinyl}essigsäure Hydrochlorid

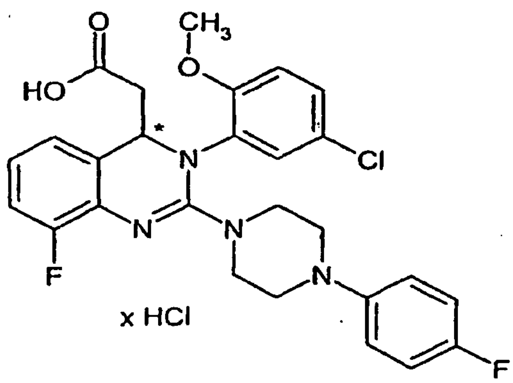

Ausgehend von 320 mg (0.06 mmol) des Racemats aus Beispiel 22 werden nach chromatographischer Enantiomerentrennung (Methode 4) sowie anschließendem Aufnehmen des Produktes in Methanol/1N Salzsäure und erneutem Abdampfen des Lösungsmittels 174 mg (50% d. Th.) Hydrochlorid erhalten.
HPLC (Methode 1): $R_t$=4.51 min
MS (ESI-pos): m/z = 527.1 $(M+H-HCl)^+$
$^1$H-NMR (400MHz, $CD_3CN$): $\delta$ [ppm] = 7.29 (dd, 1H); 7.19-7.11 (m, 2H); 7.01-6.94 (m, 4H); 6.87-6.83 (m, 2H); 5.08 (t, 1H); 3.67 (s, 3H); 3.56 (s, 4H); 3.03-2.92 (m, 5H); 2.72 (dd, 1H).

**Beispiel 24**

{8-Fluor-2-[4-(3-fluorphenyl)-1-piperazinyl]-3-[2-methoxy-5-(trifluormethyl)-phenyl]-3,4-dihydro-4-chinazolinyl}essigsäure

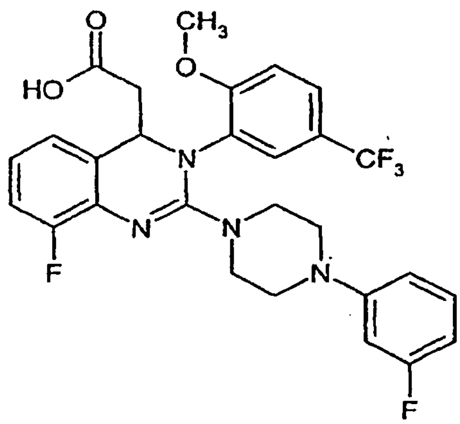

In 15ml Dioxan werden 117 mg (0.2 mmol) {8-Fluor-2-[4-(3-fluorphenyl)-1-piperazinyl]-3-[2-methoxy-5-(trifluonnethyl)phenyl-3,4-dihydro-4-chinazolinyl}essigsäuremethylester (Beispiel 34A) mit 0.61 ml 1 N Natronlauge versetzt und 3 Stunden bei 50°C gerührt. Nach Entfernen des Lösungsmittels wird der Rückstand in Wasser aufgenommen und mit 1 N Salzsäure auf pH 3-4 eingestellt. Der Niederschlag wird abgesaugt, mit Wasser gewaschen und im Vakuum getrocknet
Ausbeute: 76 mg (67% d. Th.)
HPLC (Methode 1): $R_t$=4.6 min
MS (ESI-pos): m/z = 561 $(M+H)^+$

**Beispiel 25**

{8-Fluor-2-[4-(3-fluorphenyl)-1-piperazinyl]-3-[2-methoxy-5-(trifluormethyl)-phenyl]-3,4-dihydro-4-chinazolinyl}essigsäure

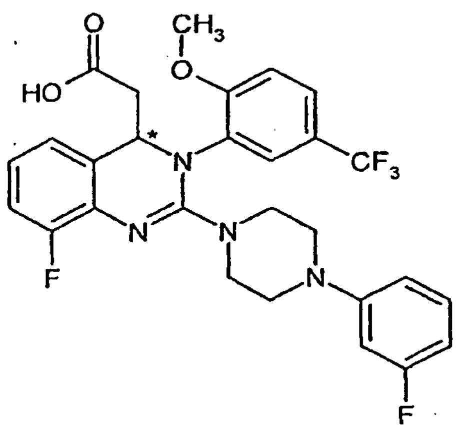

Es werden 52 mg (0.09 mmol) des Racemats (Beispiel 24) in die Enantiomere getrennt (Methode 13). Anschließend wird das Rohprodukt durch Chromatographie an Kieselgel (Essigsäure, Dichlormethan/Methanol 10:1) gereinigt und im Vakuum getrocknet.
Ausbeute: 12.3 mg (24% d. Th.)
LC-MS (Methode 7): $R_t$=2.50 min

MS (ESI-pos): m/z= 561 $(M+H)^+$
$^1$H-NMR (400 MHz, DMSO-$d_6$): δ [ppm] = 2.35-2.5 (m, 1H); 2.7-3.1 (m, 5H); 3.3-3.6 (m, 4H); 3.8 ($S_b$, 3H); 4.8-4.9 (m, 1H); 6.45-6.6 (m, 1H); 6.6-6.7 (m, 2H); 6.8-6.9 (m, 2H); 6.98-7. 1 (m, 1H); 7.1-7.6 (m, 4H); 12.4 ($S_b$, 1H).

Die Beispiele 26 bis 34 und 36 bis 89 der Tabelle 2 können nach den allgemeinen Arbeitsvorschriften [A] bis [H] aus den entsprechenden Ausgangsverbindungen und Beispiel 35 wie im Anschluss an Tabelle 2 beschrieben hergestellt werden.

**Tabelle 2**

| Beispiel Nr. | Struktur | Molekulargewicht [g/mol] | AusgangsVerbindung Beispiel | $R_t$ [min] | HPLC Methode | MS |
|---|---|---|---|---|---|---|
| 26 | 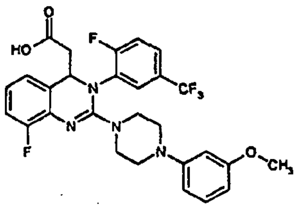 | 597.0 | 50A | 4.53 | 1 | 561 $[M+H-HCl]^+$ |
| 27 | 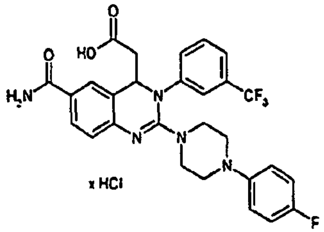 | 592.0 | 51A | 4.22 | 1 | 556 $[M+H-HCl]^+$ |
| 28 | 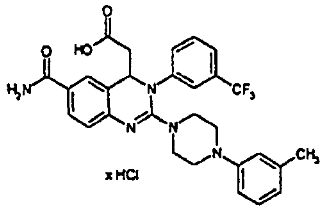 | 588.0 | 52A | 4.36 | 1 | 552 $[M+H-HCl]^+$ |
| 29 | 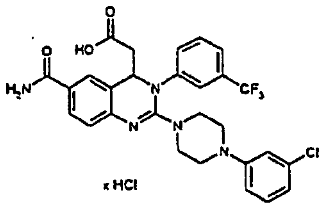 | 608.4 | 53A | 4.37 | 1 | 572 $[M+H-HCl]^*$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 30 | 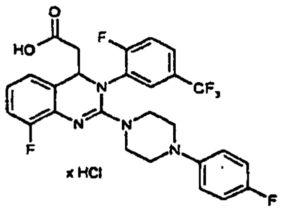 x HCl | 584.9 | 54A | 4.54 | 1 | 548 $[M+H-HCl]^+$ |
| 31 | 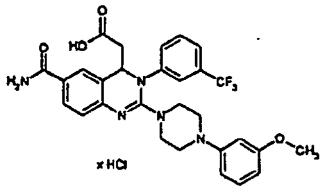 x HCl | 604.0 | 55A | 4.27 | 1 | 568 $[M+H-HCl]^*$ |
| 32 | 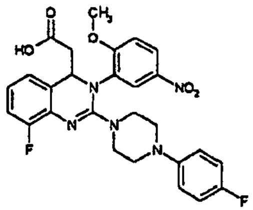 | 537.5 | 56A | 4.30 | 1 | 538 [M+H]+ |
| 33 | 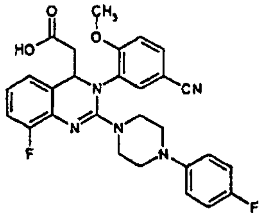 | 517.5 | 57A | 4.28 | 1 | 518 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 34 | 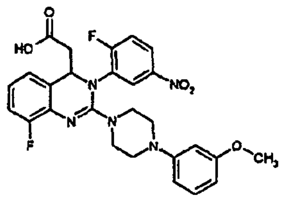 | 537.5 | 58A | 4.41 | 1 | 538 $[M+H]^+$ |
| 35 | 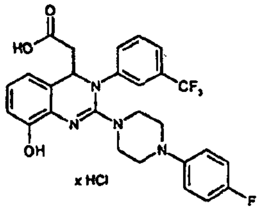 | 565.0 | 89 | 4.47 | 1 | 529 $[M+H-HCl]^+$ |
| 36 | 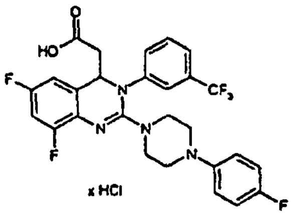 | 584.9 | 60A | 4.61 | 1 | 549 $[M+H-HCl]^+$ |
| 37 | 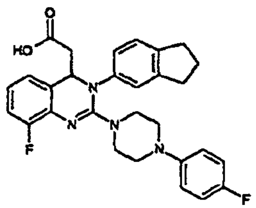 | 502.6 | 61A | 4.6 | 1 | 503 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 38 | 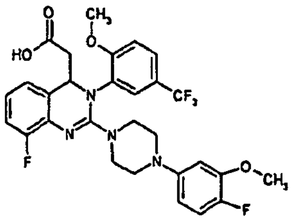 | 590.6 | 62A | 4.6 | 6 | 591 $[M+H]^+$ |
| 39 | 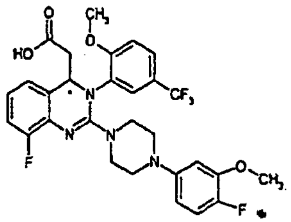 | 590.6 | 63A | 4.53 | 1 | 591 591 $[M+H]^+$ |
| 40 | 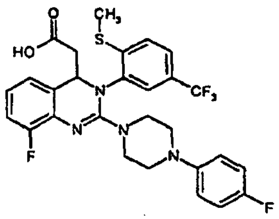 | 576.6 | 64A | 4.5 | 6 | 577 $[M+H]^+$ |
| 41 | 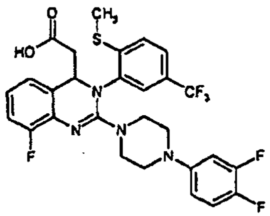 | 594.6 | 65A | 4.5 | 6 | 595 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 42 | 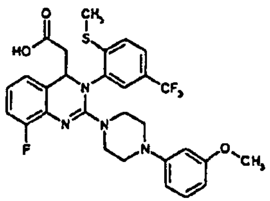 | 588.6 | 66A | 4.4 | 6 | 589 $[M+H]^+$ |
| 43 | 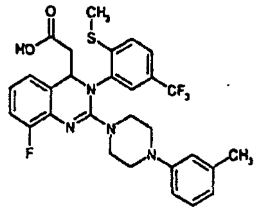 | 572.6 | 67A | 4.5 | 6 | 573 $[M+H]^+$ |
| 44 | 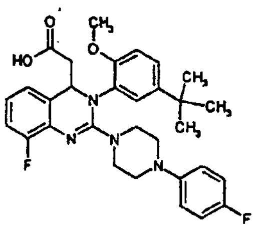 | 548.6 | 68A | 4.9 | 1 | 549 $[M+H]^+$ |
| 45 | 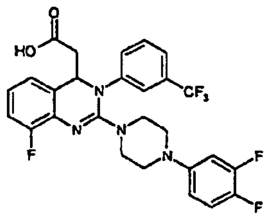 | 548.5 | 69A | 4.67 | 1 | 549 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 46 | 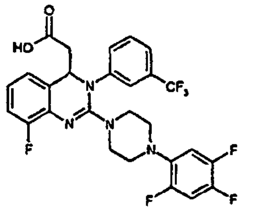 | 566.5 | 70A | 4.60 | 1 | 567 $[M+H]^+$ |
| 47 | 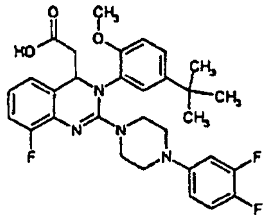 | 566.6 | 71A | 4.9 | 1 | 567 567 $[M+H]^+$ |
| 48 | 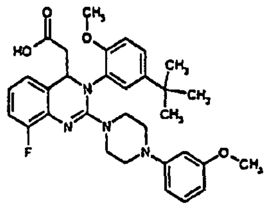 | 560.7 | 72A | 4.8 | 1 | 561 $[M+H]^+$ |
| 49 | 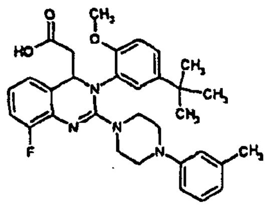 | 544.7 | 73A | 5.0 | 1 | 545 $[M+M]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 50 | 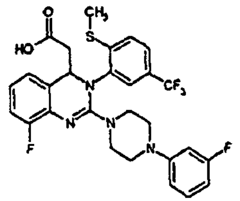 | 576.6 | 74A | 4.6 | 1 | 577 $[M+H]^+$ |
| 51 | 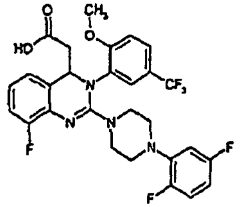 | 578.5 | 75A | 4.7 | 1 | 579 $[M+H]^+$ |
| 52 | 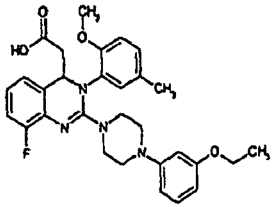 | 532.6 | 76A | 4.6 | 1 | 561 $[M+H]^+$ |
| 53 | 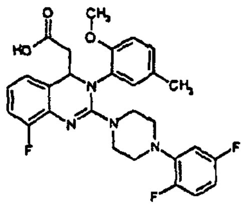 | 524.5 | 77A | 4.5 | 1 | 525 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 54 | 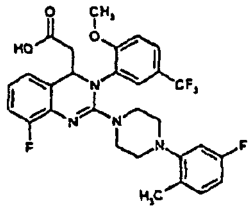 | 574.6 | 78A | 4.7 | 1 | 575 $[M+H]^+$ |
| 55 | 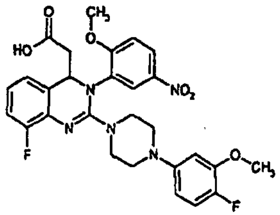 | 567.6 | 79A | 4.3 | 1 | 568 $[M+H]^+$ |
| 56 | 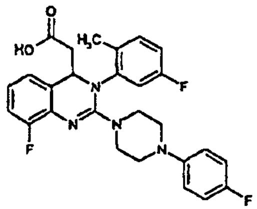 | 494.5 | 80A | 2.77 | 10 | 495 $[M+H]^+$ |
| 57 | 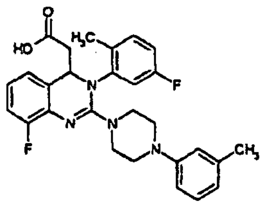 | 490.6 | 81A | 1.94 | 9 | 491 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 58 | 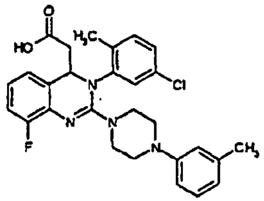 | 507.0 | 82A | 1.97 | 9 | 507 $[M+H]^+$ |
| 59 | 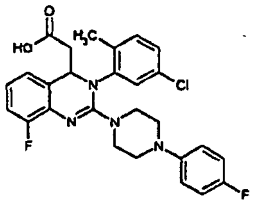 | 511.0 | 83A | 1.93 | 9 | 511 $[M+H]^+$ |
| 60 | 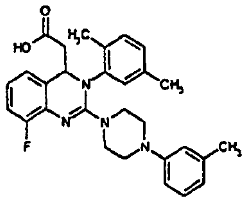 | 486.6 | 84A | 1.90 | 9 | 487 $[M+H]^+$ |
| 61 | 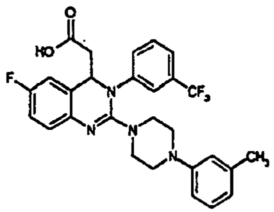 | 526.5 | 85A | 4.69 | 1 | 527 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 62 | 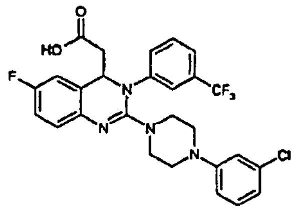 | 545.0 | 86A | 3.57 | 8 | 547 $[M+H]^+$ |
| 63 | 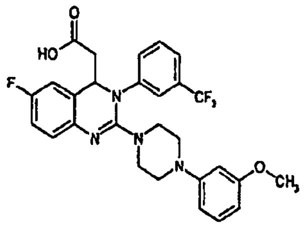 | 542.5 | 87A | 3.37 | 8 | 543 $[M+H]^+$ |
| 64 | 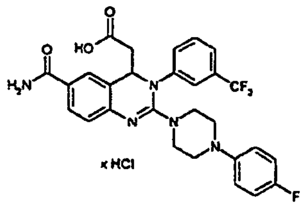 x HCl | 574.0 | 88A | 4.43 | 1 | 538 $[M+H-HCl]^+$ |
| 65 | 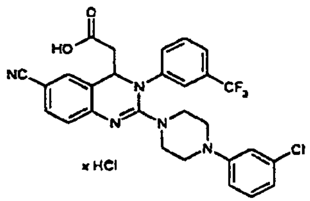 x HCl | 590.4 | 89A | 4.58 | 1 | 554 $[M+H-HCl]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 66 | 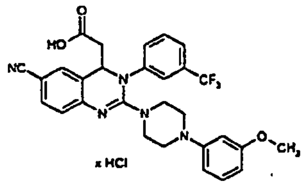 | 586.0 | 90A | 4.41 | 1 | 550 $[M+H-HCl]^+$ |
| 67 | 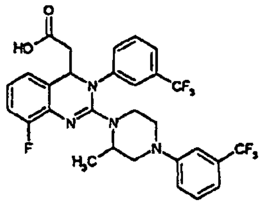 | 594.5 | 91A | 4.82 | 1 | 595 595 $[M+H]^+$ |
| 68 | 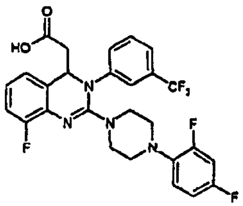 | 548.5 | 92A | 4.66 | 1 | 549 $[M+H]^+$ |
| 69 | 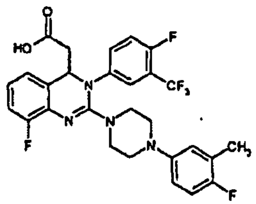 | 562.5 | 93A | 4.74 | 1 | 563 563 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 70 | 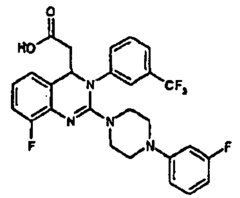 | 530.5 | 94A | 4.62 | 1 | 531 [M+H]+ |
| 71 | 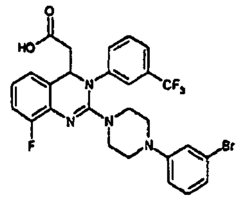 | 591.4 | 95A | 4.76 | 1 | 591 $[M+H]^+$ |
| 72 | 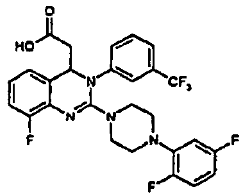 | 548.5 | 96A | 4.63 | 1 | 549 $[M+H]^+$ |
| 73 | 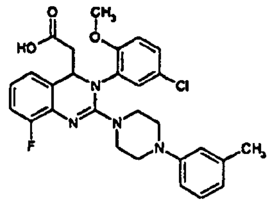 | 523.0 | 97A | 4.65 | 1 | 523 $[M+H]^+$ |
| 74 | 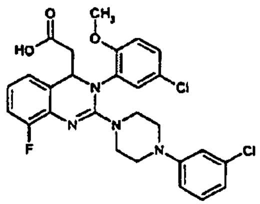 | 543.4 | 98A | 4.67 | 6 | 543 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 75 | 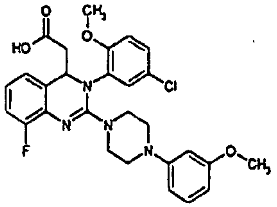 | 539.0 | 99A | 4.56 | 6 | 539 $[M+H]^+$ |
| 76 | 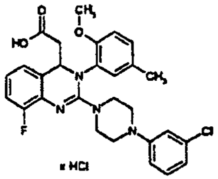 x HCl | 559.5 | 100A | 4.63 | 6 | 523 $[M+H-HCl]^+$ |
| 77 | 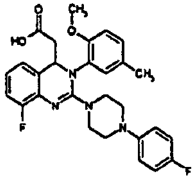 | 506.6 | 101A | 4.52 | 6 | 507 $[M+H]^+$ |
| 78 | 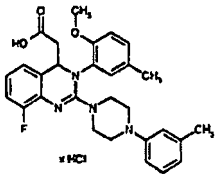 x HCl | 539.0 | 102A | 4.63 | 6 | 503 $[M+H-HCl]^+$ |
| 79 | 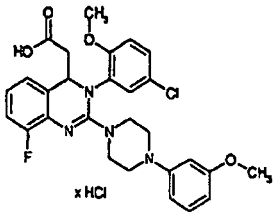 x HCl | 555.0 | 103A | 4.41 | 6 | 519 $[M+H-HCl]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 80 | 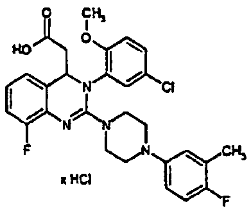 | 577.5 | 104A | 4.53 | 6 | 541 $[M+H-HCl]^+$ |
| 81 | 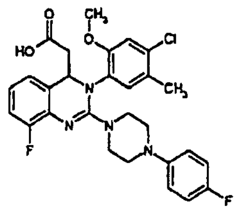 | 541.0 | 105A | 4.68 | 1 | 541 $[M+H]^+$ |
| 82 | 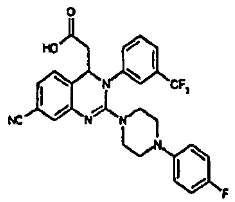 | 537.5 | 45A | 4.52 | 1 | 538 $[M+H]^+$ |
| 83 | 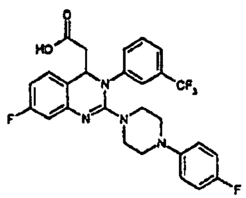 | 530.5 | 106A | 4.51 | 1 | 531 $[M+H]^+$ |
| 84 | 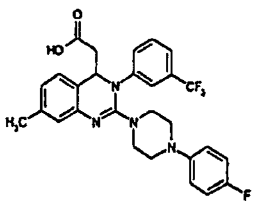 | 526.5 | 107A | 4.70 | 1 | 527 $[M+H]^+$ |

(fortgesetzt)

| Beispiel Nr. | Struktur | Molekulargewicht [g/mol] | AusgangsVerbindung Beispiel | $R_t$ [min] | HPLC Methode | MS |
|---|---|---|---|---|---|---|
| 85 | 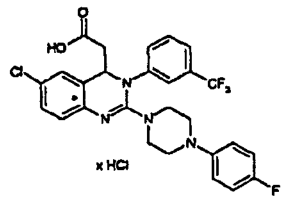 | 583.4 | 108A | 4.61 | 1 | 547 $[M+H-HCl]^+$ |
| 86 | 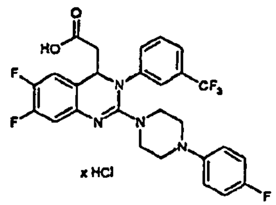 | 585.0 | 109A | 4.82 | 1 | 549 $[M+H-HCl]^*$ |
| 87 | 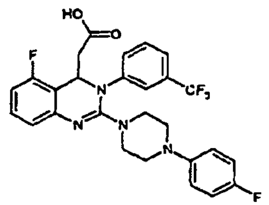 | 530.5 | 110A | 4.54 | 1 | 531 $[M+H]^+$ |
| 88 | 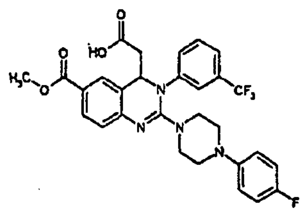 | 570.6 | 111A | 4.54 | 1 | 571 $[M+H]^+$ |
| 89 | 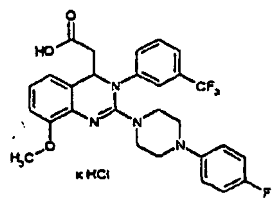 | 579.0 | 59A | 4.60 | 1 | 543 $[M+H-HCl]^+$ |

**Beispiel 35**

{2-[4-(4-Fluorphenyl)piperazin-1-yl]-8-hydroxy-3-[3-(trifluormethyl)phenyl]-3,4-dihydrochinazolin-4-yl}essigsäure

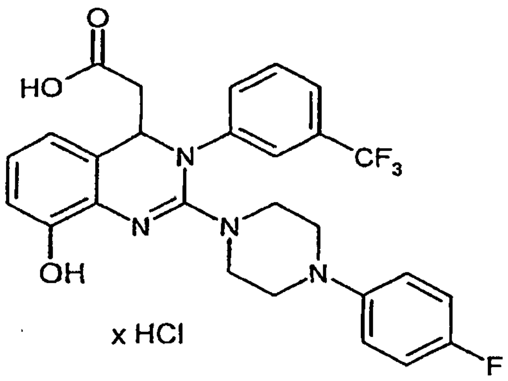

80 mg (0.14 mmol) Methylether (Beispiel 89) werden in 2 ml Dichlormethan gelöst und bei 0°C mit 0.41 mmol 1M Bortribromid-Lösung in Dichlormethan versetzt. 16 Stunden wird bei Raumtemperatur gerührt, weitere 0.82 mmol Bortribromid-Lösung addiert und nach 24 Stunden weitere 1.23 mmol zugesetzt. Man rührt 24 Stunden bei Raumtemperatur, gießt das Reaktionsgemisch anschließend auf Eis und setzt 5 ml einer 1N wässrigen Salzsäure-Lösung zu. Man extrahiert mit 25 ml Essigsäureethylester. Die organische Phase wird mit einer gesättigten, wässrigen Natriumchlorid-Lösung gewaschen, über Natriumsulfat getrocknet, eingeengt und per präparativer HPLC gereinigt. Man erhält 50 mg (63% d. Th.) Produkt.
HPLC (Methode 1): $R_t$= 4.47 min
MS (ESI-pos): m/z = 529 $(M+H-HCl)^+$

**Beispiel 90**

{7-Hydroxcarbonyl-2-[4-(4-fluorphenyl)-1-piperazinyl]-3-[5-(trifluormethyl)-phenyl]-3,4-dihydro-4-chinazolinyl}essigsäure

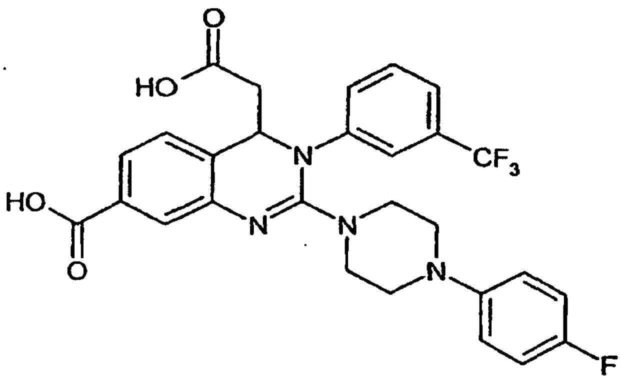

Es werden 100 mg (0.16 mmol) des Esters aus Beispiel 45A in halbkonzentrierter Salzsäure suspendiert und das Reaktionsgemisch 42 Stunden bei 90°C gerührt. Nach dem Erkalten wird mit 20%iger Natronlauge auf pH=4 eingestellt, der sich bildende Rückstand abfiltriert, mit Wasser gewaschen und im Vakuum getrocknet.
Ausbeute: 64 mg (66% d. Th.)
HPLC (Methode 1): $R_t$= 4.38 min
MS (ESI-pos): m/z = 557 $(M+H)^+$

**Beispiel 91**

{6-(Aminocarbonyl)-2-[4-(4-fluorphenyl)piperazin-1-yl]-3-[3-(trifluormethyl)phenyl]-3,4-dihydrochinazolin-4-yl}essigsäure Hydrochlorid

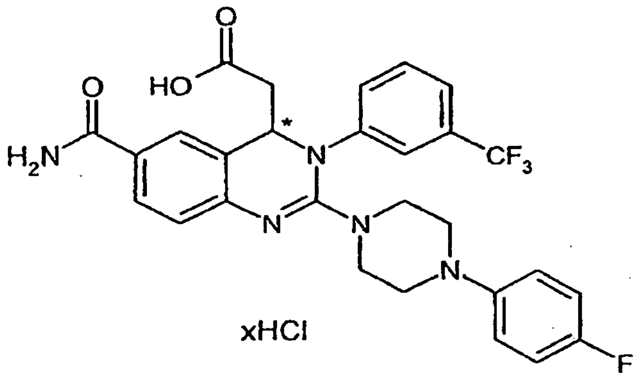

Es werden 500 mg (0.8 mmol) des tert.-Butylesters aus Beispiel 49A mit 8 ml einer 4M Lösung von Chlorwasserstoff in Dioxan suspendiert und das Reaktionsgemisch 16 Stunden bei Raumtemperatur gerührt. Die Suspension wird eingeengt und im Vakuum getrocknet.
Ausbeute: 564 mg (99% d. Th.)
HPLC (Methode 1): $R_t$=4.25 min
MS (ESI-pos): m/z = 556 $(M+H-HCl)^+$
$^1$H-NMR (300 MHz, DMSO-$d_6$): $\delta$ [ppm] = 12.94 (brs, 1H); 8.11 (s, 1H); 8.03-7.95 (m, 2H); 7.92-7.65 (m, 4H); 7.09-6.91 (m, 4H); 5.50 (dd, 1H); 4.38-4.12 (m, 4H); 3.17-3.06 (m, 5H); 2.81 (dd, 1H).

**B. Bewertung der physiologischen Wirksamkeit**

Die *in vitro*-Wirkung der erfindungsgemäßen Verbindungen kann in folgenden Assays gezeigt werden:

**Anti-HCMV-(Anti-Humanes Cytomegalo-Virus) Zytopathogenitätstests**

Die Testverbindungen werden als 50 millimolare (mM) Lösungen in Dimethysulfoxid (DMSO) eingesetzt. Ganciclovir®, Foscarnet® und Cidofovir® dienen als Referenzverbindungen. Nach der Zugabe von jeweils 2 µl der 50, 5, 0,5 und 0,05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x $10^4$ Zellen (humane Vorhautfibroblasten [NHDF]) pipettiert(Reihe 1 = Zellkontrolle) bzw. in die Reihen 2-12 ein Gemisch von HCMV-infizierten und nichtinfizierten NHDF-Zellen (M.O.I. = 0,001 - 0,002), d.h. 1-2 infizierte Zellen auf 1000 nicht-infizierte Zellen. Die Reihe 12 (ohne Substanz) dient als Viruskontrolle. Die End-Testkonzentrationen liegen bei 250-0,0005 µM. Die Platten werden 6 Tage bei 37°C /5 % $CO_2$ inkubiert, d.h. bis in den Viruskontrollen alle Zellen infiziert sind (100 % cytopathogener Effekt [CPE]). Die Wells werden dann durch Zugabe eines Gemisches von Formalin und Giemsa's Farbstoff fixiert und gefärbt (30 Minuten), mit aqua bidest, gewaschen und im Trockenschrank bei 50°C getrocknet. Danach werden die Platten mit einem Overhead-Mikroskop (Plaque Multiplier der Firma Technomara) visuell ausgewertet.
Die folgenden Daten können von den Testplatten ermittelt werden:

$CC_{50}$ (NHDF) = Substanzkonzentration in µM, bei der im Vergleich zur unbehandelten Zellkontrolle keine sichtbaren cytostatischen Effekte auf die Zellen erkennbar sind;

$EC_{50}$ (HCMV) = Substanzkonzentration in µM, die den CPE (cytopathischen Effekt) um 50 % im Vergleich zur unbehandelten Viruskontrolle hemmt;

$$SI \text{ (Selektivitätsindex)} = CC_{50} \text{ (NHDF)} / EC_{50} \text{ (HCMV)}.$$

Repräsentative in-vitro-Wirkdaten für die erfindungsgemäßen Verbindungen sind in Tabelle Awiedergegeben:

**Tabelle A**

| Beispiel Nr. | NHDF $CC_{50}$ [µM] | HCMV $EC_{50}$ [µM] | SI HCMV |
|---|---|---|---|
| 2 | 12 | 0.016 | 750 |
| 9 | 15 | 0.02 | 750 |
| 15 | 31 | 0.002 | 15500 |
| 19 | 17 | 0.002 | 8947 |
| 23 | 24 | 0.002 | 12632 |
| 29 | 47 | 0.07 | 671 |

Die Eignung der erfindungsgemäßen Verbindungen zur Behandlung von HCMV-Infektionen kann im folgenden Tiermodell gezeigt werden:

**HCMV Xenograft-Gelfoam®-Modell**

Tiere:

3-4 Wochen alte weibliche immundefiziente Mäuse (16-18 g), Fox Chase SCID oder Fox Chase SCID-NOD oder SCID-beige werden von kommerziellen Züchtern (Taconic M+B, Jackson, USA) bezogen. Die Tiere werden unter sterilen Bedingungen (einschließlich Streu und Futter) in Isolatoren gehalten.

Virusanzucht:

Humanes Cytomegalovirus (HCMV), Stamm Davis oder AD169, wird *in vitro* auf humanen embryonalen Vorhautfibroblasten (NHDF-Zellen) angezüchtet. Nach Infektion der NHDF-Zellen mit einer Multiplizität der Infektion (M.O.I) von 0,01-0,03 werden die virusinfizierten Zellen 5-10 Tage später geerntet und in Gegenwart von Minimal Essential Medium (MEM), 10 % foetalem Kälberserum (FKS) mit 10 % DMSO bei -40'C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot.

Vorbereitung der Schwämme, Transplantation, Behandlung und Auswertung:

1x1x1 cm große Kollagenschwämme (Gelfoam®; Fa. Peasel & Lorey, Best.-Nr. 407534; K.T. Chong et al., Abstracts of 39th Interscience Conference on Antimicrobial Agents and Chemotherapy, 1999, S. 439) werden zunächst mit Phosphat-gepufferter Saline (PBS) benetzt, die eingeschlossenen Luftblasen durch Entgasen entfernt und dann in MEM + 10 % FKS aufbewahrt. 1 x $10^6$ virusinfizierte NHDF-Zellen (Infektion mit HCMV-Davis oder HCMV AD169 M.O.I = 0.03) werden 3 Stunden nach Infektion abgelöst und in 20 µl MEM, 10 % FKS auf einen feuchten Schwamm getropft. Ca. 16 Stunden später werden die mit den infizierten Zellen beladenen Schwämme mit 25 µl PBS / 0,1 % BSA /1 mM DTT mit 5 ng/µl basic Fibroblast Growth Factor (bFGF) inkubiert. Zur Transplantation werden die immundefizienten Mäuse mit Avertin oder mit einer Ketamin/Xylazin/Azepromazin Mischung narkotisiert, das Rückenfell mit Hilfe eines Rasierers entfernt, die Oberhaut 1-2 cm geöffnet, entlastet und die feuchten Schwämme unter die Rückenhaut transplantiert. Die Operationswunde wird mit Gewebekleber verschlossen. 6 Stunden nach der Transplantation können die Mäuse zum ersten Mal behandelt werden (am Tag der Operation wird einmal behandelt). An den folgenden Tagen wird über einen Zeitraum von 8 Tagen dreimal täglich (7.00 Uhr und 14.00 Uhr und 19.00 Uhr), zweimal täglich (8 Uhr und 18 Uhr) oder einmal täglich (14 Uhr) peroral mit Substanz behandelt. Die Tagesdosis beträgt beispielsweise 3 oder 10 oder 30 oder 60 oder 100 mg/kg Körpergewicht, das Applikationsvolumen 10 ml/kg Körpergewicht. Die Formulierung der Substanzen erfolgt in Form einer 0,5 %-igen Tylosesuspension mit 2 % DMSO oder einer 0,5 %-igen Tylosesuspension. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U/1,5 ml) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well-Platten konfluenter NHDF-Zellen nach Vitalfärbung mit Neutralrot. Ermittelt wird die Anzahl infizierter Zellen bzw. infektiöser Viruspartikel (infectious center assay) nach Substanzbehandlung im Vergleich zur placebobehandelten

Kontrollgruppe.

## CYP-Inhibitions-Assay

Zur Untersuchung der mechanism-based (irreversiblen) Inhibition von CYP3A4 wird die Testsubstanz in verschiedenen Konzentrationen mit Humanlebermikrosomen (2mg/ml mikrosomales Protein) in Kaliumphosphatpuffer pH 7.4 unter Zusatz von NADPH-generierendem System (NADP+, Glucose-6-phosphat und Glucose-6-phosphatdehydrogenase) bei 37°C inkubiert. Zu verschiedenen Zeitpunkten werden 2 Aliqouts aus der Inkubation entnommen.
Das erste Aliqout wird 1:50 in eine neue Inkubationslösung (Phosphatpuffer, NADPH-generierendes System und 10 μM Midazolam) für weitere 10 min bei 37°C inkubiert. Danach wird die Inkubation mit Acetonitril auf Eis gestoppt, in der Zentrifuge bei 15000g das Protein pelletiert, und der Überstand mit HPLC/MS nach Standardmethoden auf die Bildung von 1'-Hydroxymidazolam analysiert.
Das zweite Aliquot wird mit Acetonitril auf Eis gestoppt und mit HPLC/UV/MS auf verbleibenden Testsubstanz analysiert.
Aus beiden analytischen Datensätzen werden für irreversible Inhibition typische Parameter ($k_{inact}$, $K_i$ und partition ratio r) bestimmt und die Testsubstanz damit bewertet (vgl. A. Madan, et al., in A.D. Rodrigues (ed.) "Drug-Drug Interaction" in "Drugs and the Pharmaceutical Science", Vol. 116, , ISBN 0-8247-0283.2, Marcel Dekker Inc., New York, 2002.).

## C. Ausführungsbeispiele für pharmazeutische Zusammensetzungen

Die erfindungsgemäßen Verbindungen können folgendermaßen in pharmazeutische Zubereitungen überführt werden:

### Tablette:

Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

Herstellung :

Die Mischung aus Wirkstoff, Lactose und Stärke wird mit einer 5 %-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

Zusammensetzung:

1000 mg der Verbindung von Beispiel 1, 1000 mg Ethanol (96 %), 400 mg Rhodigel (Xanthan gum der Fa. FMC, Pennsylvania, USA) und 99 g Wasser.
Einer Einzeldosis von 100 mg der erfindungsgemäßen Verbindung entsprechen 10 ml orale Suspension.

Herstellung.

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h geührt.

### Intravenös applizierbare Lösung :

Zusammencetsung:

10-500 mg der Verbindung von Beispiel 1.15 g Polyethylenglykol 400 und 250 g Wasser für Injektionszwecke.

Herstellung:

Die Verbindung von Beispiel 1 wird zusammen mit Polyethlenglykol 400 in dem Wasser unter Rühren gelöst. Die Lösung wird sterifitriert (Porendurchmesser 0.22 μm) und unter aseptischen Bedingungen in hitzesterilisierte Infusionsflachen abgefüllt. Diese werden mit Infusionnsstopfen und Bördelkappen verschlossen.

**Patentansprüche**

1. Verbindung der Formel

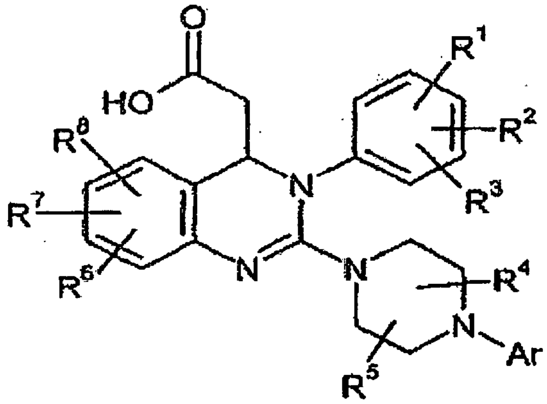

(I);

in welcher

Ar für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Formyl, Carboxyl, Alkylcarbonyl, Alkoxycarbonyl, Trifluormethyl, Halogen, Cyano, Hydroxy, Amino, Alkylamino, Aminocarbonyl und Nitro,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,
oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls vorhandener dritter Substituent unabhängig davon aus der genannten Gruppe ausgewählt wird,
$R^1$ für Wasserstoff, Amino, Alkyl, Alkoxy, Alkylamino, Alkylthio, Cyano, Halogen, Nitro oder Trifluormethyl steht,
$R^2$ für Wasserstoff, Alkyl, Alkoxy, Alkylthio, Cyano, Halogen, Nitro oder trifluormethyl steht,
$R^3$ für Animo, Alkyl, Alkoxy, Alkylamino, Alkylthio, Cyano. Halogen, Nitro, Trifluormethyl, Alkylsulfonyl oder Alkylaminosulfonyl steht

oder
einer der Reste $R^1$, $R^2$ und $R^3$ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Diaxolan, einen Cyrlopentan-Ring oder einen Cyclohexan-Ring bilden.

$R^4$ für Wasserstoff oder Alkyl steht,
$R^5$ für Wasserstoff oder Alkyl steht

oder die Reste $R^4$ und $R^5$ im Piperazin-Ring an genau gegenüberliegenden Kohlenstoffatomen gebunden sind und eine gegebenenfalls mit 1 bis 2 Methylgruppen substituierte Methylen-Brücke bilden.

$R^6$ für Alkyl, Alkoxy, Alkylthio, Formyl, Carboxyl, Aminocarbonyl, Alkylcarbonyl, Alkoxycarbonyl, Trifluormethyl, Halogen, Cyano, Hydroxy oder Nitro steht,
$R^7$ für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht
und
$R^6$ für Wasserstoff, Alkyl, Alkoxy, Alkylthio, Formyl, Carboxyl, Alkylcarbonyl, Alkoxycarbonyl, Trifluormethyl,

Halogen, Cyano, Hydroxy oder Nitro steht,

und deren Salze, Solvate und Solvate der Salze.

**2.** Verbindung nach Anspruch 1, **dadurch gekennzeichnet, dass**

Ar für Phenyl steht, worin Phenyl substitulert sein kann mit 1 bis 3 Substituenten, wobei die Substituenten unabhängig voneinander ausgewählt werden aus der Gruppe, bestehend aus $C_1$-$C_6$-Alkyl, $C_1$-$C_6$-Alkoxy, Carboxyl, $C_1$-$C_6$-Alkylcarbonyl, $C_1$-$C_6$-Alkoxycarbonyl, Trifluormethyl, Fluor, Chlor, Brom, Cyano, Hydroxy, Amino, $C_1$-$C_6$-Alkylamino und Nitro.
oder zwei der Substituenten am Phenyl zusammen mit den Kohlenstoffatomen, an die sie gebunden sind, ein 1,3-Dioxolan bilden und ein gegebenenfalls vorhandener dritter Substituent unabhängig davon aus der genannten Gruppe ausgewählt wird.
$R^1$ für Wasserstoff, $C_1$-$C_3$-Alkyl, $C_1$-$C_3$-Alkoxy, $C_1$-$C_3$-Alkylthio, Fluor oder Chlor steht,
$R^2$ für Wasserstoff, $C_1$-$C_3$-Alkyl, $C_1$-$C_3$-Alkoxy, $C_1$-$C_3$-Alktylthio, Fluor oder Chlor steht,
$R^3$ für $C_1$-$C_4$-Alkyl, Cyano, Fluor, Chlor, Nitro, Trifluormethyl oder $C_1$-$C_3$-Alkylsulfonyl steht,

oder
einer der Reste $R^1$, $R^2$ und $R^3$ für Wasserstoff, $C_1$-$C_3$-Alkyl, $C_1$-$C_3$-Alkoxy, Cyano, Hatogen, Nitro oder Trifluormethyl steht und die anderen beiden zusammen mit den Kohlenstoffatomen, an die sie gebunden sind, einen Cyclopentan-Ring oder einen Cyclohexan-Ring bilden,

$R^4$ für Wasserstoff oder Methyl steht,
$R^5$ für Wasserstoff steht,
$R^6$ für $C_1C_3$ Alkyl, $C_1$-$C_3$-Alkoxy, Carboxyl. Aminocarbonyl, Trifluormethyl, Fluor, Chlor, Cyano, Hydroxy oder Nitro steht,
$R^7$ für Wasserstoff, $C_1$-$C_3$-Alkyl, $C_1$-$C_3$-Alkoxy, Fluor, Chlor, Cyano oder Hydroxy steht
und
$R^8$ für Wasserstoff, $C_1$-$C_3$-Alkyl, $C_1$-$C_3$-Alkoxy, Fluor, Chlor, Cyano oder Hydroxy steht.

**3.** Verbindung nach Anspruch 1 oder 2, **dadurch gekennzeichnet, dass**

Ar für Phenyl steht, worin Phenyl substituiert sein kann mit 1 bis 2 Substituenten, wobei die Subslituenten unabhängig voneinander ausgewählt werden aus der Gruppe, bestehend aus Methyl, Methoxy, Fluor und Chlor,
$R^1$ für Wasserstoff, Methyl, Methoxy, Methylthio, Fluor oder Chlor steht,
$R^2$ für Wasserstoff steht,
$R^3$ für Methyl, iso-Propyl, tert-Butyl, Cyano, Fluor, Nitro oder Trifluormethyl steht,
$R^4$ für Wasserstoff steht,
$R^5$ für Wasserstoff steht,
$R^6$ für Aminocarbonyl, Fluor, Chlor, Cyano oder Hydroxy steht,
$R^7$ für Wasserstoff steht
**und**
$R^8$ für Wasserstoff, Fluor oder Chlor steht.

**4.** Verbindung nach einem der Ansprüche 1 bis 3. **dadurch gekennzeichnet, dass** $R^1$ für Wasserstoff, Methyl, Methoxy oder Fluor steht.

**5.** Verbindung nach einem der Ansprüche 1 bis 4, **dadurch gekennzeichnet, dass** $R^1$ für Methoxy steht.

**6.** Verbindung nach einem der Ansprüche 1 bis 5, **dadurch gekennzeichnet, dass** $R^1$ über die ortho-Position zur Verknüpfungsstelle des Phenylrings an den Phenylring gebunden ist.

**7.** Verbindung nach einem der Ansprüche 1, 2 und 4 bis 6, **dadurch gekennzeichnet, dass** $R^2$ für Wasserstoff steht.

**8.** Verbindung nach einem der Ansprüche 1 bis 7, **dadurch gekennzeichnet, dass** $R^3$ für Trifluormethyl, Chlor, Methyl, iso-Propyl oder tert-Butyl steht.

**9.** Verbindung nach einem der Ansprüche 1 bis 8, welche ist

{8-Fluor-2-[4-(3-methoxyphenyl)-1-piperazinyl]-3-[2-methoxy-5-(trifluormethyl)-phenyl]-3,4-dihydro-4-chinazolinyl}essigsäure

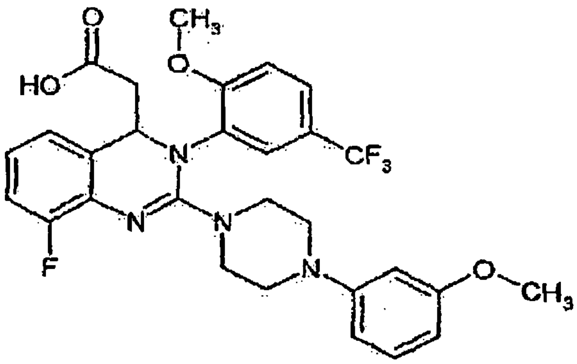

**10.** Verbindung nach einem der Ansprüche 1 bis 8, **dadurch gekennzeichnet, dass** $R^1$ über die ortho-Position zur Verknüpfungsstelle des Phenylrings an den Phenylring gebunden ist und $R^9$ über die $R^1$ gegenüberliegende meta-Position zur Verknüpfungsstelle des Phenylrings an den Phenylring gebunden ist.

**11.** Verbindung nach einem der Ansprüche 1, 2 und 4 bis 8 und 10, **dadurch gekennzeichnet, dass** $R^4$ und $R^5$ für Wasserstoff stehen.

**12.** Verbindung nach einem der Ansprüche 1 bis 8 und 10 bis 11, **dadurch gekennzeichnet, dass** $R^6$ für Fluor steht.

**13.** Verbindung nach einem der Ansprüche 1, 2 und 4 bis 8 und 10 bis 12. **dadurch gekennzeichnet, dass** $R^7$ für Wasserstoff steht.

**14.** Verbindung nach einem der Ansprüche 1, 2 und 4 bis 8 und 10 bis 13, **dadurch gekennzeichnet, dass** $R^8$ für Wasserstoff, Methyl oder Fluor steht.

**15.** Verbindung nach einem der Ansprüche 1, 2 und 4 bis 8 und 10bis 14, **dadurch gekennzeichnet, dass** Ar für Phenyl steht, worin Phenyl substituiert sein kann mit 1 bis 2 Substituenten, wobei die Substituenten unabhängig voneinander ausgewählt werden aus der Gruppe, bestehend aus Methyl, Methoxy, Fluor und Chlor.

**16.** Verfahren zur Herstellung einer Verbindung der Formel (I) nach Anspruch 1. **dadurch gekennzeichnet, dass** eine Verbindung der Formel

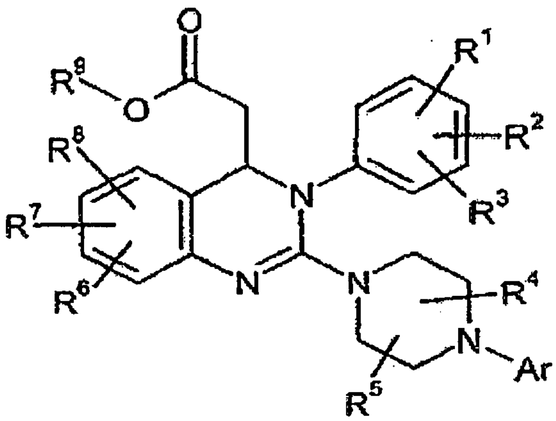

(II),

in welcher

Ar, $R^1$, $R^2$, $R^3$, $R^4$, $R^5$, $R^6$, $R^7$ und $R^9$ die In Anspruch 1 angegebene Bedeutung haben und

$R^9$ für Alkyl, bevorzugt für Methyl oder Ethyl oder für tert.-Butyl, steht,

mit einer Base oder einer Säure umgesetzt wird.

**17.** Verbindung nach einem der Ansprüche 1 bis 15 zur Behandlung und/oder Prophylaxe von Krankheiten.

**18.** Arzneimittel enthaltend eine Verbindung nach einem der Ansprüche 1 bis 15 in Kombination mit einem inerten, nichttoxischen, pharmazeutisch geeigneten Hilfsstoff

**19.** Verwendung einer Verbindung nach einem der Ansprüche 1 bis 15 zur Herstellung eines Arzneimittels zur Behandlung und/oder Prophylaxe von Virusinfektionen.

**20.** Verwendung nach Anspruch 119, **dadurch gekennzeichnet, dass** die Virusinfektion cinc Infektion mit dem humanen Cytomegalovirus (HCMV) oder einem anderen Vertreter der Gruppe der Herpes viridae ist.

**21.** Arzneimittel nach Anspruch 18 zur Behandlung und/oder Prophylaxe von Virusinfektionen.

## Claims

**1.** Compound of the formula

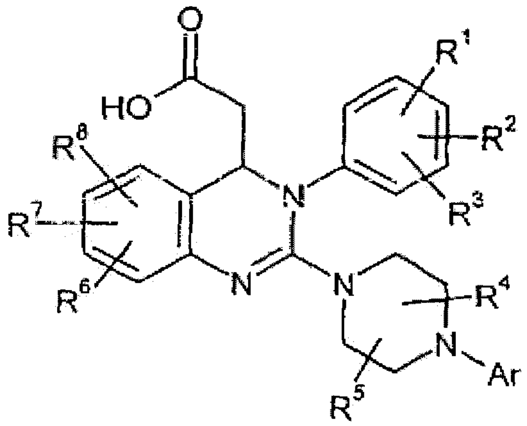

(I),

in which

Ar represents aryl which may be substituted by 1 to 3 substituents, where the substituents are selected independently of one another from the group consisting of alkyl, alkoxy, formyl, carboxyl, alkylcarbonyl, alkoxycarbonyl, trifluoromethyl, halogen, cyano, hydroxyl, amino, alkylamino, aminocarbonyl and nitro,
where alkyl may be substituted by 1 to 3 substituents, where the substituents are selected independently of one another from the group consisting of halogen, amino, alkylamino, hydroxyl and aryl,
or two of the substituents on the aryl radical together with the carbon atoms to which they are attached form a 1,3-dioxolane, a cyclopentane ring or a cyclohexane ring, and any third substituent present is selected independently from the group mentioned,
$R^1$ represents hydrogen, amino, alkyl, alkoxy, alkylamino, alkylthio, cyano, halogen, nitro or trifluoromethyl,
$R^2$ represents hydrogen, alkyl, alkoxy, alkylthio, cyano, halogen, nitro or trifluoromethyl,
$R^3$ represents amino, alkyl, alkoxy, alkylamino, alkylthio, cyano, halogen, nitro, trifluoromethyl, alkylsulfonyl or alkylaminosulfonyl

or

one of the radicals $R^1$, $R^2$ and $R^3$ represents hydrogen, alkyl, alkoxy, cyano, halogen, nitro or trifluoromethyl and the other two together with the carbon atoms to which they are attached form a 1,3-dioxolane, a cyclopentane ring or a cyclohexane ring,

$R^4$ represents hydrogen or alkyl,

$R^5$ represents hydrogen or alkyl
or
the radicals $R^4$ and $R^5$ are attached to carbon atoms directly opposing each other in the piperazine ring and form a methylene bridge which is optionally substituted by 1 or 2 methyl groups,
$R^6$ represents alkyl, alkoxy, alkylthio, formyl, carboxyl, aminocarbonyl, alkylcarbonyl, alkoxycarbonyl, trifluoromethyl, halogen, cyano, hydroxyl or nitro,
$R^7$ represents hydrogen, alkyl, alkoxy, alkylthio, formyl, carboxyl, alkylcarbonyl, alkoxycarbonyl, trifluoromethyl, halogen, cyano, hydroxyl or nitro
and
$R^8$ represents hydrogen, alkyl, alkoxy, alkylthio, formyl, carboxyl, alkylcarbonyl, alkoxycarbonyl, trifluoromethyl, halogen, cyano, hydroxyl or nitro,

and their salts, solvates and solvates of the salts.

**2.** Compound according to Claim 1, **characterized in that**
Ar represents phenyl which may be substituted by 1 to 3 substituents, where the substituents are selected independently of one another from the group consisting of $C_1$-$C_6$-alkyl, $C_1$-$C_6$-alkoxy, carboxyl, $C_1$-$C_6$-alkylcarbonyl, $C_1$-$C_6$-alkoxycarbonyl, trifluoromethyl, fluorine, chlorine, bromine, cyano, hydroxyl, amino, $C_1$-$C_6$-alkylamino and nitro,
or two of the substituents on the phenyl radical together with the carbon atoms to which they are attached form a 1,3-dioxolane and any third substituent present is selected independently from the group mentioned,
$R^1$ represents hydrogen, $C_1$-$C_3$-alkyl, $C_1$-$C_3$-alkoxy, $C_1$-$C_3$-alkylthio, fluorine or chlorine,
$R^2$ represents hydrogen, $C_1$-$C_3$-alkyl, $C_1$-$C_3$-alkoxy, $C_1$-$C_3$-alkylthio, fluorine or chlorine,
$R^3$ represents $C_1$-$C_4$-alkyl, cyano, fluorine, chlorine, nitro, trifluoromethyl or $C_1$-$C_3$-alkylsulfonyl,
or
one of the radicals $R^1$, $R^2$ and $R^3$ represents hydrogen, $C_1$-$C_3$-alkyl, $C_1$-$C_3$-alkoxy, cyano, halogen, nitro or trifluoromethyl and the other two together with the carbon atoms to which they are attached form a cyclopentane ring or a cyclohexane ring,
$R^4$ represents hydrogen or methyl,
$R^5$ represents hydrogen,
$R^6$ represents $C_1$-$C_3$-alkyl, $C_1$-$C_3$-alkoxy, carboxyl, aminocarbonyl, trifluoromethyl, fluorine, chlorine, cyano, hydroxyl or nitro;
$R^7$ represents hydrogen, $C_1$-$C_3$-alkyl, $C_1$-$C_3$-alkoxy, fluorine, chlorine, cyano or hydroxyl
and
$R^8$ represents hydrogen, $C_1$-$C_3$-alkyl, $C_1$-$C_3$-alkoxy, fluorine, chlorine, cyano or hydroxyl.

**3.** Compound according to Claim 1 or 2, **characterized in that**
Ar represents phenyl which may be substituted by 1 or 2 substituents, where the substituents are selected independently of one another from the group consisting of methyl, methoxy, fluorine and chlorine,
$R^1$ represents hydrogen, methyl, methoxy, methylthio, fluorine or chlorine,
$R^2$ represents hydrogen,
$R^3$ represents methyl, isopropyl, tert-butyl, cyano, fluorine, chlorine, nitro or trifluoromethyl, $R^4$ represents hydrogen,
$R^5$ represents hydrogen,
$R^6$ represents aminocarbonyl, fluorine, chlorine, cyano or hydroxyl,
$R^7$ represents hydrogen
and
$R^8$ represents hydrogen, fluorine or chlorine.

**4.** Compound according to any of Claims 1 to 3, **characterized in that** $R^1$ represents hydrogen, methyl, methoxy or fluorine.

**5.** Compound according to any of Claims 1 to 4, **characterized in that** $R^1$ represents methoxy.

**6.** Compound according to any of Claims 1 to 5, **characterized in that** $R^1$ is attached to the phenyl ring via the position ortho to the point of attachment of the phenyl ring.

**7.** Compound according to any of Claims 1, 2 and 4 to 6, **characterized in that** $R^2$ represents hydrogen.

**8.** Compound according to any of Claims 1 to 7, **characterized in that** $R^3$ represents trifluoromethyl, chlorine, methyl, isopropyl or tert-butyl.

**9.** Compound according to any of Claims 1 to 8, that is {8-fluoro-2-[4-(3-methoxyphenyl)piperazine-1-yl]-3-[2-methoxy-5-(trifluoromethylphenyl]-3,4-dihydroquinazoline-4-yl}acetic acid

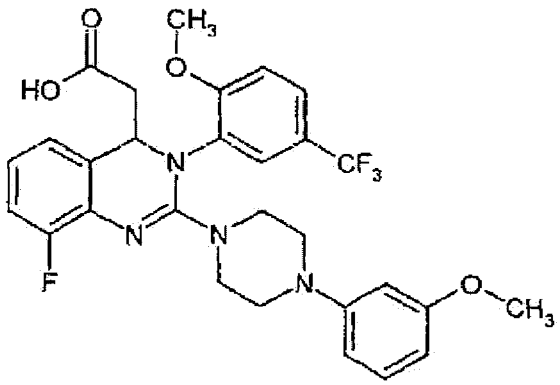

**10.** Compound according to any of Claims 1 to 8, **characterized in that** $R^1$ is attached to the phenyl ring via the position ortho to the point of attachment of the phenyl ring and $R^3$ is attached to the phenyl ring via the position meta to the point of attachment of the phenyl ring, which position is opposite to that of $R^1$.

**11.** Compound according to any of Claims 1, 2 and 4 to 8 and 10, **characterized in that** $R^4$ and $R^5$ represent hydrogen.

**12.** Compound according to any of Claims 1 to 8 and 10 to 11, **characterized in that** $R^6$ represents fluorine.

**13.** Compound according to any of Claims 1, 2 and 4 to 8 and 10 to 12, **characterized in that** $R^7$ represents hydrogen.

**14.** Compound according to any of Claims 1, 2 and 4 to 8 and 10 to 13, **characterized in that** $R^8$ represents hydrogen, methyl or fluorine.

**15.** Compound according to any of Claims 1, 2 and 4 to 8 and 10 to 14, **characterized in that** Ar represents phenyl which may be substituted by 1 or 2 substituents, where the substituents are selected independently of one another from the group consisting of methyl, methoxy, fluorine and chlorine.

**16.** Process for preparing a compound of the formula (I) according to Claim 1, **characterized in that** a compound of the formula

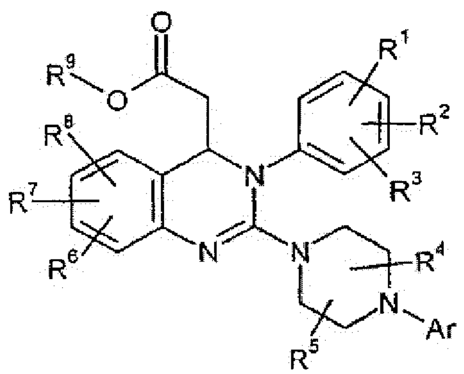

(II),

in which

Ar, $R^1$, $R^2$, $R^3$, $R^4$, $R^5$, $R^6$, $R^7$ and $R^8$ are as defined in claim 1
and

$R^9$ represents alkyl, preferably methyl or ethyl or tert-butyl,
is reacted with a base or an acid.

**17.** Compound according to any of Claims 1 to 15 for the treatment and/or the prophylaxis of diseases.

**18.** Medicament comprising a compound according to any of Claims 1 to 15 in combination with an inert non-toxic pharmaceutically acceptable auxiliary.

**19.** Use of a compound according to any of Claims 1 to 15 for preparing a medicament for the treatment and/or prophylaxis of viral diseases.

**20.** Use according to Claim 19, **characterized in that** the viral infection is an infection with the human cytomegalovirus (HCMV) or another representative of the group of the Herpes viridae.

**21.** Medicament according to Claim 18 for the treatment and/or prophylaxis of viral infections.

**Revendications**

**1.** Composé de formule

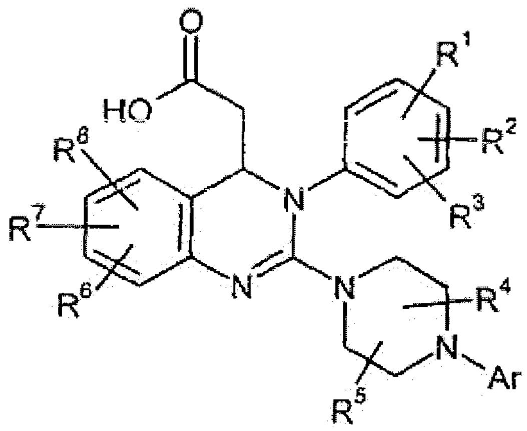

(I),

dans laquelle

Ar représente un reste aryle, lequel reste aryle peut être substitué avec 1 à 3 substituants, les substituants étant choisis indépendamment les uns des autres dans le groupe constitué des substituants alkyle, alkoxy, formyle, carboxyle, alkylcarbonyle, alkoxycarbonyle, trifluorométhyle, halogéno, cyano, hydroxy, amino, alkylamino, aminocarbonyle et nitro,
où un reste alkyle peut être substitué avec 1 à 3 substituants, les substituants étant choisis indépendamment les uns des autres dans le groupe constitué des substituants halogéno, amino, alkylamino, hydroxy et aryle,
ou bien deux des substituants portés par le reste aryle forment, conjointement avec les atomes de carbone auxquels ils sont liés, un groupement 1,3-dioxolanne, un noyau cyclopentane ou un noyau cyclohexane, et un troisième substituant éventuellement présent est choisi indépendamment d'eux dans le groupe mentionné,
$R^1$ représente l'hydrogène, un reste amino, alkyle, alkoxy, alkylamino, alkylthio, cyano, halogéno, nitro, ou trifluorométhyle,
$R^2$ représente l'hydrogène, un reste alkyle, alkoxy, alkylthio, cyano, halogéno, nitro ou trifluorométhyle,
$R^3$ représente un reste amino, alkyle, alkoxy, alkylamino, alkylthio, cyano, halogéno, nitro, trifluorométhyle, alkylsulfonyle ou alkylaminosulfonyle,

ou bien
l'un des restes R1, R2 et R3 représente l'hydrogène, un reste alkyle, alkoxy, cyano, halogéno, nitro ou trifluorométhyle et les deux autres forment, conjointement avec les atomes de carbone auxquels ils sont liés, un groupement 1,3-dioxolanne, un noyau cyclopentane ou un noyau cyclohexane,

$R^4$ représente l'hydrogène ou un reste alkyle,
$R^5$ représente l'hydrogène ou un reste alkyle,

ou bien
les restes $R^4$ et $R^5$ dans le noyau pipérazine sont liés à des atomes de carbone exactement opposés et forment un pont méthylène éventuellement substitué avec 1 ou 2 groupes méthyle,

$R^6$ représente un reste alkyle, alkoxy, alkylthio, formyle, carboxyle, aminocarbonyle, alkylcarbonyle, alkoxycarbonyle, trifluorométhyle, halogéno, cyano, hydroxy ou nitro, R7 représente l'hydrogène, un reste alkyle, alkoxy, alkylthio, formyle, carboxyle, alkylcarbonyle, alkoxycarbonyle, trifluorométhyle, halogéno, cyano, hydroxy ou nitro
et
$R^8$ représente l'hgydrogène, un reste alkyle, alkoxy, alkylthio, formyle, carboxyle, alkylcarbonyle, alkoxycarbonyle, trifluorométhyle, halogéno, cyano, hydroxy ou nitro,

et leurs sels, leurs produits de solvatation et les produits de solvatation de leurs sels.

**2.** Composé suivant la revendication 1, **caractérisé en ce que**
Ar représente un reste phényle, lequel reste phényle peut être substitué avec 1 à 3 substituants, les substituants étant choisis, indépendamment les uns des autres, dans le groupe constitué des substituants alkyle en $C_1$ à $C_6$, alkoxy en $C_1$ à $C_6$, carboxyle, (alkyle en $C_1$ à $C_6$) -carbonyle, (alkoxy en $C_1$ à $C_6$)-carbonyle, trifluorométhyle, fluoro, chloro, bromo, cyano, hydroxy, amino, alkylamino en $C_1$ à $C_6$ et nitro, ou bien deux des substituants portés par le reste phényle forment, conjointement avec les atomes de carbone auxquels ils sont liés, un groupe 1,3-dioxolanne, et un troisième substituant éventuellement présent est choisi indépendamment d'eux dans le groupe mentionné,

$R^1$ représente l'hydrogène, un reste alkyle en $C_1$ à $C_3$, alkoxy en $C_1$ à $C_3$, alkylthio en $C_1$ à $C_3$, le fluor ou le chlore,
$R^2$ représente l'hydrogène, un reste alkyle en $C_1$ à $C_3$, alkoxy en $C_1$ à $C_3$, alkylthio en $C_1$ à $C_3$, le fluor ou le chlore,
$R^3$ représente un reste alkyle en $C_1$ à $C_4$, cyano, le fluor, le chlore, un reste nitro, trifluorométhyle ou alkylsulfonyle en $C_1$ à $C_3$,

ou bien
l'un des restes $R^1$, $R^2$ et $R^3$ représente l'hydrogène, un reste alkyle en $C_1$ à $C_3$, alkoxy en $C_1$ à $C_3$, cyano, halogéno, nitro ou trifluorométhyle et les deux autres forment, conjointement avec les atomes de carbone auxquels ils sont liés, un noyau cyclopentane ou un noyau cyclohexane,

$R^4$ représente l'hydrogène ou un groupe méthyle,
$R^5$ représente l'hydrogène,
$R^6$ représente un reste alkyle en $C_1$ à $C_3$, alkoxy en $C_1$ à $C_3$, carboxyle, aminocarbonyle, trifluorométhyle, le fluor, le chlore, un reste cyano, hydroxy ou nitro,
$R^7$ représente l'hydrogène, un reste alkyle en $C_1$ à $C_3$, alkoxy en $C_1$ à $C_3$, le fluor, le chlore, un reste cyano

ou hydroxyl
et
$R^8$ représente l'hydrogène, un reste alkyle en $C_1$ à $C_3$, alkoxy en $C_1$ à $C_3$, le fluor, le chlore, un reste cyano ou hydroxy.

**3.** Composé suivant la revendication 1 ou 2, **caractérisé en ce que**
Ar représente un reste phényle, lequel reste phényle peut être substitué avec 1 ou 2 substituants, les substituants étant choisis indépendamment l'un de l'autre dans le groupe constitué des substituants méthyle, méthoxy, fluoro et chloro,
$R^1$ représente l'hydrogène, un reste méthyle, méthoxy, méthylthio, le fluor ou le chlore, $R^2$ représente l'hydrogène,
$R^3$ représente un reste méthyle, isopropyle, tertiobutyle, cyano, le fluor, le chlore ou un reste trifluorométhyle,
$R^4$ représente l'hydrogène,
$R^5$ représente l'hydrogène,
$R^6$ représente un reste aminocarbonyle, le fluor, le chlore, un reste cyano ou hydroxy, $R^7$ représente l'hydrogène
et
$R^8$ représente l'hydrogène, le fluor ou le chlore.

**4.** Composé suivant l'une des revendications 1 à 3, **caractérisé en ce que** $R^1$ représente l'hydrogène, un reste méthyle,

méthoxy ou le fluor.

**5.** Composé suivant l'une des revendications 1 à 4, **caractérisé en ce que** $R^1$ est un reste méthoxy.

**6.** Composé suivant l'une des revendications 1 à 5, **caractérisé en ce que** $R^1$ est lié au noyau phényle par l'intermédiaire de la position ortho par rapport au site de liaison du noyau phényle.

**7.** Composé suivant l'une des revendications 1, 2 et 4 à 6, **caractérisé en ce que** $R^2$ représente l'hydrogène.

**8.** Composé suivant l'une des revendications 1 à 7, **caractérisé en ce que** $R^3$ représente un reste trifluorométhyle, le chlore, un reste méthyle, isopropyle ou tertiobutyle.

**9.** Composé suivant l'une des revendications 1 à 8,
qui est
l'acide acétique de (8-fluoro-2-[4-(3-méthoxyphényle)pipérazine-1-yl]-3-[2-méthoxy-5-(trifluorométhyle) phényle]-3,4-dihydroquinazoline-4-yl}

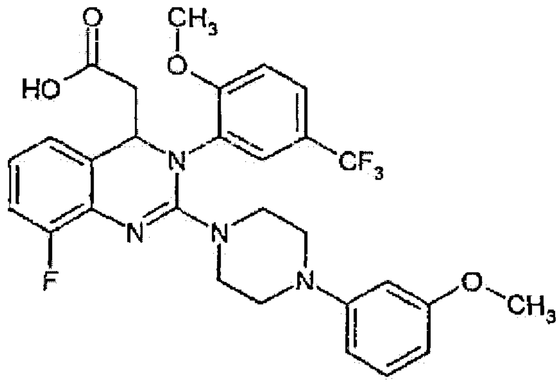

**10.** Composé suivant l'une des revendications 1 à 8, **caractérisé en ce que** $R^1$ est lié au noyau phényle par l'intermédiaire de la position ortho par rapport au site de liaison du noyau phényle et $R^3$ est lié au noyau phényle par l'intermédiaire de la position méta opposée à R1 par rapport au site de liaison du groupe phényle.

**11.** Composé suivant l'une des revendications 1, 2 et 4 à 8 et 10, **caractérisé en ce que** $R^4$ et $R^5$ représentent l'hydrogène.

**12.** Composé suivant l'une des revendications 1 à 8 et 10 à 11, **caractérisé en ce que** $R^6$ représente le fluor.

**13.** Composé suivant l'une des revendications 1, 2 et 4 à 8 et 10 à 12, **caractérisé en ce que** $R^7$ représente l'hydrogène.

**14.** Composé suivant l'une des revendications 1, 2 et 4 à 8 et 10 à 13, **caractérisé en ce que** $R^8$ représente l'hydrogène, un reste méthyle ou le fluor.

**15.** 1Composé suivant l'une des revendications 1, 2 et 4 à 8 et 10 à 14, **caractérisé en ce que** Ar représente un reste phényle, lequel reste phényle peut être substitué par 1 ou 2 substituants, les substituants étant choisis indépendamment l'un de l'autre dans le groupe constitué des restes méthyle, méthoxy, fluoro et chloro.

**16.** Procédé de production d'un composé de formule (I) suivant la revendication 1, **caractérisé en ce qu'**un composé de formule

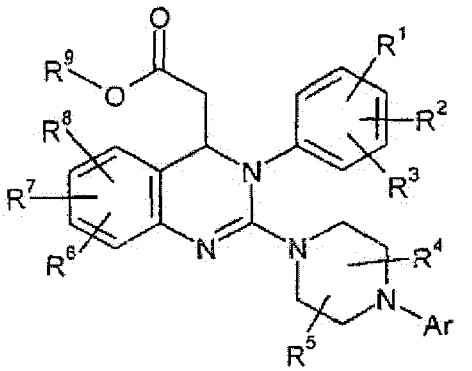

(II),

dans laquelle

Ar, R1, R2, R3, R4, R5, R6 R7 et R8 ont la définition indiquée dans la revendication 1
et
$R^9$ représente un reste alkyle, avantageusement méthyle ou éthyle ou un reste tertiobutyle,

avec une base ou un acide.

**17.** Composé suivant l'une des revendications 1 à 15, destiné au traitement et/ou à la prophylaxie de maladies.

**18.** Médicament contenant un composé suivant l'une des revendications 1 à 15 en combinaison avec une substance auxiliaire inerte non toxique pharmaceutiquement convenable.

**19.** Utilisation d'un composé suivant l'une des revendications 1 à 15 pour la préparation d'un médicament destiné au traitement et/ou à la prophylaxie d'infections virales.

**20.** Utilisation suivant la revendication 19, **caractérisée en ce que** l'infection virale est une infection impliquant le cytomégalovirus humain (HCMV) ou un autre représentant du groupe des Herpesviridae.

**21.** Médicament suivant la revendication 18, destiné au traitement et/ou à la prophylaxie d'infections virales.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **SAITO T. et al.** *Tetrahedron Lett.,* 1996, vol. 37, 209-212
- **WANG F. et al.** *Tetrahedron Lett.,* 1997, vol. 38, 8651-8654
- **C.G. FROST ; P. MENDONCA.** *J. Chem. Soc., Perkin Trans I,* 1998, 2615-2623
- **J. CINATL et al.** *FEMS Microbiology Reviews,* 2004, vol. 28, 59-77
- **K.T. CHONG et al.** Abstracts of 39th Interscience Conference on Antimicrobial Agents and Chemotherapy. 1999, 439
- Drug-Drug Interaction. **A. MADAN et al.** Drugs and the Pharmaceutical Science. Marcel Dekker Inc, 2002